# EUROPEAN PATENT SPECIFICATION

(11) **EP 0 787 148 B1**
(45) Date of publication and mention of the grant of the patent: **07.04.2004**
(21) Application number: 95942838.4
(22) Date of filing: 26.10.1995
(51) Int. Cl.: C07K 14/47, C12N 15/12, C07K 16/18, C07K 19/00, C12N 5/10, C12Q 1/68, A61K 38/12

(54) **AL-1 NEUROTROPHIC FACTOR, A LIGAND FOR AN EPH-RELATED TYROSINE KINASE RECEPTOR**
NEUROTROPHISCHER AL-1 FAKTOR, EINER LIGAND VERWANT MIT DEM EPH TYROSINE KINASE RECEPTOR
FACTEUR NEUROTROPHIQUE AL-1, UN LIGAND POUR LE RECEPTEUR TYROSINE KINASE APPARENTE A EPH

(30) Priority: 27.10.1994 US 330128; 07.06.1995 US 486449
(43) Date of publication of application: 06.08.1997
(73) Proprietor: GENENTECH, INC., South San Francisco, CA 94080-4990 (US)
(72) Inventor: CARAS, Ingrid, W., San Francisco, CA 94114 (US); WINSLOW, John, W., El Granada, CA 94018 (US)
(74) Representative: Kiddle, Simon John
(86) International application number: PCT/US1995/014016
(87) International publication number: WO 1996/013518

(56) References cited:
- EP-A- 0 597 503
- WO-A-94/11384
- WO-A-95/27060
- CELL, 1994, 79 157-168, CHENG HJ ET AL 'IDENTIFICATION AND CLONING OF ELF-1, A DEVELOPMENTALLY EXPRESSED LIGAND FOR THE MEK4 AND SEK RECEPTOR TYROSINE KINASES'
- ONCOGENE, vol. 8, 1993 pages 3277-3288, XP 000566895 MAISONPIERRE P.C. ET AL. 'Ehk-1 and Ehk-2: two novel members of the Eph receptor-liketyrosine kinase family with distinctive structures and neuronal expression.'
- J. NEUROSCI. RES. 37:129-143(01-1994)., ZHOU R. ET AL. '"Isolation and characterization of Bsk, a growth factor receptor-like tyrosine kinase associated with the limbic system'
- NEURON, vol. 14, May 1995 pages 973-981, WINSLOW J.W. ET AL. 'Cloning of AL-1, a ligand for an Eph-related tyrosine kinase receptor involved in axon bundle formation'
- SCIENCE, vol. 266, November 1994 pages 816-819, DAVIS S. ET AL. 'LIGANDS FOR EPH RELATED RECEPTOR TYROSINE KINASES THAT REQUIRE MEMBRANE ATTACHMENT OR CLUSTERING FOR ACTIVITY'

### SEQUENCE LISTING

(1) GENERAL INFORMATION:
   (i) APPLICANT: Genentech, Inc.
      Caras, Ingrid W.
      Winslow, John W.
   (ii) TITLE OF INVENTION: AL-1 Neurotrophic Factor
   (iii) NUMBER OF SEQUENCES: 18
   (iv) CORRESPONDENCE ADDRESS:
      (A) ADDRESSEE: Genentech, Inc.
      (B) STREET: 460 Point San Bruno Blvd
      (C) CITY: South San Francisco
      (D) STATE: California
      (E) COUNTRY: USA
      (F) ZIP: 94080
   (v) COMPUTER READABLE FORM:
      (A) MEDIUM TYPE: 3.5 inch, 1.44 Mb floppy disk
      (B) COMPUTER: IBM PC compatible
      (C) OPERATING SYSTEM: PC-DOS/MS-DOS
      (D) SOFTWARE: WinPatin (Genentech)
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: 08/330128
      (B) FILING DATE: 27-OCT-1994
   (vii) PRIOR APPLICATION DATA:
      (A) APPLICATION NUMBER: 08/486449
      (B) FILING DATE: 06-JUN-1995
   (viii) ATTORNEY/AGENT INFORMATION:
      (A) NAME: Torchia, Timothy E.
      (B) REGISTRATION NUMBER: 36,700
      (C) REFERENCE/DOCKET NUMBER: P0920P2PCT
   (ix) TELECOMMUNICATION INFORMATION:
      (A) TELEPHONE: 415/225-8674
      (B) TELEFAX: 415/952-9881
      (C) TELEX: 910/371-7168
(2) INFORMATION FOR SEQ ID NO:1:
   (i) SEQUENCE CHARACTERISTICS:
      (A) LENGTH: 4165 base pairs
      (B) TYPE: Nucleic Acid
      (C) STRANDEDNESS: Single
      (D) TOPOLOGY: Linear
   (xi) SEQUENCE DESCRIPTION: SEQ ID NO:1:
   AGCCCCCAGT CCTCTAGACA CTCTGCTTGC TTTTTCCCCG CTTCCCGCCG 50
   CCTTTTTTTT TTTACGGAAA ACATCATCTA GATTTAAGAT GACTCGTCTT 100
   CTTTAATCTC GTCCGTATCA GCACTGAAGA CTGAAAGGGA ACCTTCACCA 150
   CCACTCCAAC CCTGGTGGGG GCTTAAAAAA AAAAAATAAC AGTTCTAAAA 200
   AAGAAAGGGG GACCAAAAAA CAGAAAAAGG AAAGTCTTAA GAGGCAAAGG 250
   AGCGGGACTC GGGACCCTCT GCAGACCCTT GACTCAGCCC ACCCAGGACC 300
   GTACTAGCCA TCCGAACTTC TAATTCATCT TATCCAACTG AAAGGGAGGG 350
   CGGCACAAGC CAGAAGCAAA CTTCGGCGGT CTCTGCGGAT CTGTGATTCC 400
   CACATTGAGA GGGGTCGAGA GCCAGAAGGC ACAGGACCCC ACCAGGAGGA 450
   GGAGTTCCGC GCCTCTCTCG CCCCTTCCAC CAAGCCTGAA CCTTAGACTG 500
   AACCACGCGG GACCTAAGAG GCAGAAGAGG GTAGTAGAAA ATGCGGGGCT 550
   CCGGGCCCCG CGGTGCGGGA CGCCGACGGA CCCAGGGCAG AGGTGGCGGC 600
   GGCGACACCC CCCGCGTCCC TGCCTCTCTG GCAGGCTGCT ATTCCGCACC 650
   TCTAAAGGGG CCCCTCTGGA CGTGCCTTCT CCTGTGTGCG GCGCTCCGGA 700
   CCCTTTTGGC CAGCCCCAGT AACGAAGTGA ATTTATTGGA TTCGCGCACT 750
   GTCCTGGGAG ACCTTGGATG GATTGCTTTT CCAAAGAATG GGTGGGAAGA 800
   GATTGGTGAA GTTGATGAAA ACTATCCCCC CATCCACACC TATCAAGTGT 850
   GCAAAGTTAT GGAACAGAAT CAGAATAATT GGCTGTTGAC CAGTTGGATC 900
   TCTAACGAAG GTGCTTCCAG AATTTTTATT GAACTCAAGT TTACTCTGAG 950
   GGATTGCAAC AGCCTTCCTG GAGGACTGGG GACTTGCAAG GAGACCTTTA 1000
   ACATGTATTA TTTTGAGTCG GATGATGAGA ATGGGAGAAA TATCAAAGAG 1050
   AACCAGTACA TCAAGATCGA TACCATTGCT GCTGATGAGA GCTTCACCGA 1100
   ACTTGACCTT GGAGACCGGG TCATGAAGCT GAATACGGAG GTCAGAGATG 1150
   TAGGACCTCT GAGCAAAAAG GGATTTTATC TTGCTTTCCA AGATGTCGGT 1200
   GCTTGCATCG CTCTGGTTTC TGTCCGTGTC TACTATAAAA AATGTCCTTC 1250
   TGTAGTTAGA CATTTGGCTG TTTTCCCTGA CACGATCACT GGAGCAGATT 1300
   CTTCCCAGTT GCTAGAGGTG TCAGGCTCCT GCGTCAACCA TTCTGTGACA 1350
   GACGATCCTC CCAAAATGCA TTGCAGTGCT GAAGGGGAGT GGCTGGTTCC 1400
   CATCGGGAAA TGCATGTGCA AGGCCGGATA TGAAGAGAAA AATGGTACCT 1450
   GTCAAGTGTG CAGACCTGGG TTCTTCAAAG CCTCTCCTCA CAGCCAGACC 1500
   TGCAGCAAAT GTCCACCTCA CAGTTACACC CATGAGGAAG CTTCCACCTC 1550
   TTGTGTCTGT GAAAAGGATT ATTTCAGGAG GGAATCTGAT CCGCCCACAA 1600
   TGGCATGCAC AACTCCTTCT CCAGTCACCA ACGTGAAGAA GGGGAAGATC 1650
   GCAAAGAACA GCATTTCTTT GTCTTGGCAA GAGCCAGATC GCCCCAATGG 1700
   GATCATCCTG GAGTACGAAA TCAAGTACTT TGAAAAGGAC CAAGAGACCA 1750
   GCTACACAAT TATCAAGTCT AAAGAGACCA CTATTACGGC AGAGGGCCTG 1800
   AAACCTGCGT CTGTGTATGT CTTCCAAATT CGAGCACGTA CAGCAGCAGG 1850
   CTACGGCGTC TTCAGTCGAC GGTTTGAGTT TGAAACCACA CCAGTGTCAG 1900
   TTGCAGCATC TAATGATCAA AGCCAGATTC CCATCATTGC GGTGTCAGTG 1950
   ACGGTGGGAG TCATCTTGTT GGCAGTGATG ATCGGCTTCC TCCTCAGTGG 2000
   CAGTTGCTGC GAATGTGGCT GTGGGAGGGC TTCTTCCCTG TGCGCTGTTG 2050
   CCCATCCAAG CCTAATATGG CGGTGTGGCT ACAGCAAAGC AAAGCAGGAT 2100
   CCAGAAGAGG AAAAGATGCA CTTTCATAAC GGGCACATTA AACTGCCAGG 2150
   AGTAAGAACG TACATTGATC CACACACCTA CGAAGATCCC ACTCAAGCCG 2200
   TTCATGAATT TGCCAAGGAG ATCGAGGCCT CATGCATCAC CATTGAGAGA 2250
   GTTATTGGAG CAGGTGAATT TGGTGAAGTT TGTAGTGGAC GTTTGAAACT 2300
   ACCCGGAAAA AGAGAATTGC CTGTGGCTAT CAAAACTCTT AAAGTAGGCT 2350
   ACACTGAAAA GCAGCGCAGA GATTTTCTGA GTGAAGCGAG TATCATGGGG 2400
   CAGTTTGATC ATCCAAACAT CATCCATCTA GAAGGCGTTG TGACTAAAAG 2450
   TAAACCTGTG ATGATAGTGA CAGAGTACAT GGAGAATGGC TCCTTAGACA 2500
   CATTTTTAAA GAAAAACGAT GGCCAGTTCA CTGTGATTCA GCTTGTTGGC 2550
   ATGCTGAGAG GCATCGCTGC AGGAATGAAG TACCTTTCTG ACATGGGCTA 2600
   CGTGCACAGA GACCTTGCTG CTAGAAACAT CTTAATCAAC AGTAACCTTG 2650
   TGTGCAAAGT GTCTGACTTT GGACTTTCCA GGGTGCTGGA AGATGATCCT 2700
   GAGGCAGCCT ATACCACAAG GGGAGGCAAA ATTCCAATCA GATGGACTGC 2750
   TCCAGAAGCA ATAGCTTTTC GAAAGTTTAC CTCTGCCAGT GATGTCTGGA 2800
   GCTATGGAAT CGTAATGTGG GAAGTTGTGT CCTATGGAGA GAGACCGTAC 2850
   TGGGAGATGA CCAATCAGGA TGTGATCAAG GCAGTGGAAG AAGGCTACCG 2900
   CCTGCCAAGC CCCATGGATT GTCCTGCTGC CCTCTATCAA TTAATGCTGG 2950
   ATTGCTGGCA GAAAGATCGC AACAGCAGGC CCAAGTTTGA TGACATAGTC 3000
   AACATGCTGG ATAAGCTGAT ACGAAACCCA AGTAGTTTGA AGACACTGGT 3050
   GAACGCGTCA AGCAGGGTAT CTACTTTGTT GGCAGAACAT GGATCTTTGG 3100
   GGTCTGGGGC CTACAGATCA GTAGGTGAAT GGCTAGAAGC AATCAAAATG 3150
   GGCCGGTACA CAGAGATTTT CATGGAAAAT GGATACAGTT CAATGGACGC 3200
   TGTGGCTCAG GTGACCTTGG AGGATTTGAG ACGCCTGGGA GTGACTCTGG 3250
   TCGGTCACCA GAAGAAGATC ATGAACAGCC TTCAAGAGAT GAAGGTGCAG 3300
   CTGGTAAACG GGATGGTGCC AGTGTGACCC GCACACGGGT CACTTCTCCA 3350
   AGTGAACAAC TCTGCACTTT GTAAACAGCC CTAAGATTTA TTTTAACAGA 3400
   GAAAGGGAAA TGGGTGGTTC CTAAACCTTT GAAGGCACTT GTCTCATCCT 3450
   TTGACTTATA ATCAACATTT TACTAAAATC TCCAGATCTT CTTCTTAATG 3500
   TCTTCGTTTT TTTCACTATG TAAATGTAAC CTGCGAAGAG AGCTAACATG 3550
   AGAAACAACA TCCTATAAAA ACATAGTAAC TAAATCTTTC TCCACTTGCA 3600
   GCCCCTTTCA AAACTACCAG GGATCGACTT GAAAGGAAAA GTTTTAAAGC 3650
   CATGTGTGGG CAAAGAAACG CTGCATTTTA CTGACGTTTA CTTCGAGTTT 3700
   TATTTGTCTG CATAAGTGTA TTGGAGAGCA ATATGATTAG ATTATTTCTT 3750
   AAATACAGTT TGTAATTTAA AATGGAATTA CATGTTATAA GTTATAGAAA 3800
   ATAGTTTACA GACATGTTGC CCGGTCAAGG AAAAGTTCAG CACAGGGTGT 3850
   ATATTTATTT TTCTGTGTTA TATAATTTAC TTTTAGTTGC ACTTCTAGAG 3900
   AGTATTAGGC AATGAATGTG TATAGACTGT ATAGTTTGCA ATATACCGAG 3950
   GAATGGACTT AAATTGGAAA TGTATGTATA TGTGTGTGTA TGTGCGTATG 4000
   TGTGTGTGTG TTTGAATATG TAGATGGTAT TGTTCTGCTT GCCTTTTGTA 4050
   TAGGGTTTTA ATTTTGGCCT CATACAGCAA AGGGTGTTCT AGACTATTTT 4100
   ATGGGTAAGA GGAATAGGAA GCCTTAGACC AAATTTCCCT CAAGTAGGTG 4150
   TCCTTTCTCT CATTT 4165
(2) INFORMATION FOR SEQ ID NO:2:
   (i) SEQUENCE CHARACTERISTICS:
      (A) LENGTH: 928 amino acids
      (B) TYPE: Amino Acid
      (D) TOPOLOGY: Linear
   (xi) SEQUENCE DESCRIPTION: SEQ ID NO:2:
   Met Arg Gly Ser Gly Pro Arg Gly Ala Gly Arg Arg Arg Thr Gln
   1               5                   10                  15
   Gly Arg Gly Gly Gly Gly Asp Thr Pro Arg Val Pro Ala Ser Leu
                   20                  25                  30
   Ala Gly Cys Tyr Ser Ala Pro Leu Lys Gly Pro Leu Trp Thr Cys
                   35                  40                  45
   Leu Leu Leu Cys Ala Ala Leu Arg Thr Leu Leu Ala Ser Pro Ser
                   50                  55                  60
   Asn Glu Val Asn Leu Leu Asp Ser Arg Thr Val Leu Gly Asp Leu
                   65                  70                  75
   Gly Trp Ile Ala Phe Pro Lys Asn Gly Trp Glu Glu Ile Gly Glu
                   80                  85                  90
   Val Asp Glu Asn Tyr Pro Pro Ile His Thr Tyr Gln Val Cys Lys
                   95                  100                 105
   Val Met Glu Gln Asn Gln Asn Asn Trp Leu Leu Thr Ser Trp Ile
                   110                 115                 120
   Ser Asn Glu Gly Ala Ser Arg Ile Phe Ile Glu Leu Lys Phe Thr
                   125                 130                 135
   Leu Arg Asp Cys Asn Ser Leu Pro Gly Gly Leu Gly Thr Cys Lys
                   140                 145                 150
   Glu Thr Phe Asn Met Tyr Tyr Phe Glu Ser Asp Asp Glu Asn Gly
                   155                 160                 165
   Arg Asn Ile Lys Glu Asn Gln Tyr Ile Lys Ile Asp Thr Ile Ala
                   170                 175                 180
   Ala Asp Glu Ser Phe Thr Glu Leu Asp Leu Gly Asp Arg Val Met
                   185                 190                 195
   Lys Leu Asn Thr Glu Val Arg Asp Val Gly Pro Leu Ser Lys Lys
                   200                 205                 210
   Gly Phe Tyr Leu Ala Phe Gln Asp Val Gly Ala Cys Ile Ala Leu
                   215                 220                 225
   Val Ser Val Arg Val Tyr Tyr Lys Lys Cys Pro Ser Val Val Arg
                   230                 235                 240
   His Leu Ala Val Phe Pro Asp Thr Ile Thr Gly Ala Asp Ser Ser
                   245                 250                 255
   Gln Leu Leu Glu Val Ser Gly Ser Cys Val Asn His Ser Val Thr
                   260                 265                 270
   Asp Asp Pro Pro Lys Met His Cys Ser Ala Glu Gly Glu Trp Leu
                   275                 280                 285
   Val Pro Ile Gly Lys Cys Met Cys Lys Ala Gly Tyr Glu Glu Lys
                   290                 295                 300
   Asn Gly Thr Cys Gln Val Cys Arg Pro Gly Phe Phe Lys Ala Ser
                   305                 310                 315
   Pro His Ser Gln Thr Cys Ser Lys Cys Pro Pro His Ser Tyr Thr
                   320                 325                 330
   His Glu Glu Ala Ser Thr Ser Cys Val Cys Glu Lys Asp Tyr Phe
                   335                 340                 345
   Arg Arg Glu Ser Asp Pro Pro Thr Met Ala Cys Thr Thr Pro Ser
                   350                 355                 360
   Pro Val Thr Asn Val Lys Lys Gly Lys Ile Ala Lys Asn Ser Ile
                   365                 370                 375
   Ser Leu Ser Trp Gln Glu Pro Asp Arg Pro Asn Gly Ile Ile Leu
                   380                 385                 390
   Glu Tyr Glu Ile Lys Tyr Phe Glu Lys Asp Gln Glu Thr Ser Tyr
                   395                 400                 405
   Thr Ile Ile Lys Ser Lys Glu Thr Thr Ile Thr Ala Glu Gly Leu
                   410                 415                 420
   Lys Pro Ala Ser Val Tyr Val Phe Gln Ile Arg Ala Arg Thr Ala
                   425                 430                 435
   Ala Gly Tyr Gly Val Phe Ser Arg Arg Phe Glu Phe Glu Thr Thr
                   440                 445                 450
   Pro Val Ser Val Ala Ala Ser Asn Asp Gln Ser Gln Ile Pro Ile
                   455                 460                 465
   Ile Ala Val Ser Val Thr Val Gly Val Ile Leu Leu Ala Val Met
                   470                 475                 480
   Ile Gly Phe Leu Leu Ser Gly Ser Cys Cys Glu Cys Gly Cys Gly
                   485                 490                 495
   Arg Ala Ser Ser Leu Cys Ala Val Ala His Pro Ser Leu Ile Trp
                   500                 505                 510
   Arg Cys Gly Tyr Ser Lys Ala Lys Gln Asp Pro Glu Glu Glu Lys
                   515                 520                 525
   Met His Phe His Asn Gly His Ile Lys Leu Pro Gly Val Arg Thr
                   530                 535                 540
   Tyr Ile Asp Pro His Thr Tyr Glu Asp Pro Thr Gln Ala Val His
   545 550 555
   Glu Phe Ala Lys Glu Ile Glu Ala Ser Cys Ile Thr Ile Glu Arg
   560 565 570
   Val Ile Gly Ala Gly Glu Phe Gly Glu Val Cys Ser Gly Arg Leu
   575 580 585
   Lys Leu Pro Gly Lys Arg Glu Leu Pro Val Ala Ile Lys Thr Leu
   590 595 600
   Lys Val Gly Tyr Thr Glu Lys Gln Arg Arg Asp Phe Leu Ser Glu
   605 610 615
   Ala Ser Ile Met Gly Gln Phe Asp His Pro Asn Ile Ile His Leu
   620 625 630
   Glu Gly Val Val Thr Lys Ser Lys Pro Val Met Ile Val Thr Glu
   635 640 645
   Tyr Met Glu Asn Gly Ser Leu Asp Thr Phe Leu Lys Lys Asn Asp
   650 655 660
   Gly Gln Phe Thr Val Ile Gln Leu Val Gly Met Leu Arg Gly Ile
   665 670 675
   Ala Ala Gly Met Lys Tyr Leu Ser Asp Met Gly Tyr Val His Arg
   680 685 690
   Asp Leu Ala Ala Arg Asn Ile Leu Ile Asn Ser Asn Leu Val Cys
   695 700 705
   Lys Val Ser Asp Phe Gly Leu Ser Arg Val Leu Glu Asp Asp Pro
   710 715 720
   Glu Ala Ala Tyr Thr Thr Arg Gly Gly Lys Ile Pro Ile Arg Trp
   725 730 735
   Thr Ala Pro Glu Ala Ile Ala Phe Arg Lys Phe Thr Ser Ala Ser
   740 745 750
   Asp Val Trp Ser Tyr Gly Ile Val Met Trp Glu Val Val Ser Tyr
   755 760 765
   Gly Glu Arg Pro Tyr Trp Glu Met Thr Asn Gln Asp Val Ile Lys
   770 775 780
   Ala Val Glu Glu Gly Tyr Arg Leu Pro Ser Pro Met Asp Cys Pro
   785 790 795
   Ala Ala Leu Tyr Gln Leu Met Leu Asp Cys Trp Gln Lys Asp Arg
   800 805 810
   Asn Ser Arg Pro Lys Phe Asp Asp Ile Val Asn Met Leu Asp Lys
   815 820 825
   Leu Ile Arg Asn Pro Ser Ser Leu Lys Thr Leu Val Asn Ala Ser
                   830                 835                 840
   Ser Arg Val Ser Thr Leu Leu Ala Glu His Gly Ser Leu Gly Ser
                   845                 850                 855
   Gly Ala Tyr Arg Ser Val Gly Glu Trp Leu Glu Ala Ile Lys Met
                   860                 865                 870
   Gly Arg Tyr Thr Glu Ile Phe Met Glu Asn Gly Tyr Ser Ser Met
                   875                 880                 885
   Asp Ala Val Ala Gln Val Thr Leu Glu Asp Leu Arg Arg Leu Gly
                   890                 895                 900
   Val Thr Leu Val Gly His Gln Lys Lys Ile Met Asn Ser Leu Gln
                   905                 910                 915
   Glu Met Lys Val Gln Leu Val Asn Gly Met Val Pro Val
                   920                 925             928
(2) INFORMATION FOR SEQ ID NO:3:
   (i) SEQUENCE CHARACTERISTICS:
      (A) LENGTH: 1839 base pairs
      (B) TYPE: Nucleic Acid
      (C) STRANDEDNESS: Single
      (D) TOPOLOGY: Linear
   (xi) SEQUENCE DESCRIPTION: SEQ ID NO:3:
   ATTCCCCCCC GCCCTCGCCG CCGCCACCAC ACACACGCAC GCTTCTCTCC 50
   ATCTTGTGAT TCCTTTTTCC TCCTGAACCC TCCAGTGGGG GTGCGAGTTT 100
   GTCTTTATCA CCCCCCATCC CACCGCCTTC TTTTCTTCTC GCTCTCCTAC 150
   CCCTCCCCAG CTTGGTGGGC GCCTCTTTCC TTTCTCGCCC CCTTTCATTT 200
   TTATTTATTC ATATTTATTT GGCGCCCGCT CTCTCTCTGT CCCTTTGCCT 250
   GCCTCCCTCC CTCCGGATCC CCGCTCTCTC CCCGGAGTGG CGCGTCGGGG 300
   GCTCCGCCGC TGGCCAGGCG TGATGTTGCA CGTGGAGATG TTGACGCTGG 350
   TGTTTCTGGT GCTCTGGATG TGTGTGTTCA GCCAGGACCC GGGCTCCAAG 400
   GCCGTCGCCG ACCGCTACGC TGTCTACTGG AACAGCAGCA ACCCCAGATT 450
   CCAGAGGGGT GACTACCATA TTGATGTCTG TATCAATGAC TACCTGGATG 500
   TTTTCTGCCC TCACTATGAG GACTCCGTCC CAGAAGATAA GACTGAGCGC 550
   TATGTCCTCT ACATGGTGAA CTTTGATGGC TACAGTGCCT GCGACCACAC 600
   TTCCAAAGGG TTCAAGAGAT GGGAATGTAA CCGGCCTCAC TCTCCAAATG 650
   GACCGCTGAA GTTCTCTGAA AAATTCCAGC TCTTCACTCC CTTTTCTCTA 700
   GGATTTGAAT TCAGGCCAGG CCGAGAATAT TTCTACATCT CCTCTGCAAT 750
   CCCAGATAAT GGAAGAAGGT CCTGTCTAAA GCTCAAAGTC TTTGTGAGAC 800
   CAACAAATAG CTGTATGAAA ACTATAGGTG TTCATGATCG TGTTTTCGAT 850
   GTTAACGACA AAGTAGAAAA TTCATTAGAA CCAGCAGATG ACACCGTACA 900
   TGAGTCAGCC GAGCCATCCC GCGGCGAGAA CGCGGCACAA ACACCAAGGA 950
   TACCCAGCCG CCTTTTGGCA ATCCTACTGT TCCTCCTGGC GATGCTTTTG 1000
   ACATTATAGC ACAGTCTCCT CCCATCACTT GTCACAGAAA ACATCAGGGT 1050
   CTTGGAACAC CAGAGATCCA CCTAACTGCT CATCCTAAGA AGGGACTTGT 1100
   TATTGGGTTT TGGCAGATGT CAGATTTTGG TTTTCTTTCT TTCAGCCTGA 1150
   ATTCTAAGCA ACAACTTCAG GTTGGGGGCC TAAACTTGTT CCTGCCTCCC 1200
   TCACCCCACC CCGCCCCACC CCCAGCCCTG GCCCTTGGCT TCTCTCACCC 1250
   CTCCCAAATT AAATGGACTC CAGATGAAAA TGCCAAATTG TCATAGTGAC 1300
   ACCAGTGGTT CGTCAGCTCC TGTGCATTCT CCTCTAAGAA CTCACCTCCG 1350
   TTAGCGCACT GTGTCAGCGG GCTATGGACA AGGAAGAATA GTGGCAGATG 1400
   CAGCCAGCGC TGGCTAGGGC TGGGAGGGTT TTGCTCTCCT ATGCAATATT 1450
   TATGCCTTCT CATTCAGAAC TGTAAGATGA TCGCGCAGGG CATCATGTCA 1500
   CCATGTCAGG TCCGGAGGGG AGGTATTAAG AATAGATACG ATATTACACC 1550
   ATTTCCTATA GGAGTATGTA AATGAACAGG CTTCTAAAAG GTTGAGACAC 1600
   TGGNTTTTTT TTTTAATATG ACTGTCTTAA AGCATTCTTG ACASCCCAAC 1650
   TTGTGCTCTC TAAAAGAAGC CTTTTTTTTT TTTCTAGGAG ACAGAGTGGG 1700
   TGTGGAATGC TAATACAGAG CAGGTGTGWA AACAGAGAAA ACTACAGGTT 1750
   TGCTGGGGGT GTGTATGTGT GAGTGCCTCT AATTTTTTTG GTGACTGGGC 1800
   AGTGCACACC AGATATTTTT TCTTTGAATA CAGATCACG 1839
(2) INFORMATION FOR SEQ ID NO:4:
   (i) SEQUENCE CHARACTERISTICS:
      (A) LENGTH: 228 amino acids
      (B) TYPE: Amino Acid
      (D) TOPOLOGY: Linear
   (xi) SEQUENCE DESCRIPTION: SEQ ID NO:4:
   Met Leu His Val Glu Met Leu Thr Leu Val Phe Leu Val Leu Trp
    1               5                  10                  15
   Met Cys Val Phe Ser Gln Asp Pro Gly Ser Lys Ala Val Ala Asp
                   20                  25                  30
   Arg Tyr Ala Val Tyr Trp Asn Ser Ser Asn Pro Arg Phe Gln Arg
                   35                  40                  45
   Gly Asp Tyr His Ile Asp Val Cys Ile Asn Asp Tyr Leu Asp Val
                   50                  55                  60
   Phe Cys Pro His Tyr Glu Asp Ser Val Pro Glu Asp Lys Thr Glu
                   65                  70                  75
   Arg Tyr Val Leu Tyr Met Val Asn Phe Asp Gly Tyr Ser Ala Cys
                   80                  85                  90
   Asp His Thr Ser Lys Gly Phe Lys Arg Trp Glu Cys Asn Arg Pro
                   95                 100                 105
   His Ser Pro Asn Gly Pro Leu Lys Phe Ser Glu Lys Phe Gln Leu
                  110                 115                 120
   Phe Thr Pro Phe Ser Leu Gly Phe Glu Phe Arg Pro Gly Arg Glu
                  125                 130                 135
   Tyr Phe Tyr Ile Ser Ser Ala Ile Pro Asp Asn Gly Arg Arg Ser
                  140                 145                 150
   Cys Leu Lys Leu Lys Val Phe Val Arg Pro Thr Asn Ser Cys Met
                  155                 160                 165
   Lys Thr Ile Gly Val His Asp Arg Val Phe Asp Val Asn Asp Lys
                  170                 175                 180
   Val Glu Asn Ser Leu Glu Pro Ala Asp Asp Thr Val His Glu Ser
                  185                 190                 195
   Ala Glu Pro Ser Arg Gly Glu Asn Ala Ala Gln Thr Pro Arg Ile
                  200                 205                 210
   Pro Ser Arg Leu Leu Ala Ile Leu Leu Phe Leu Leu Ala Met Leu
                  215                 220                 225
   Leu Thr Leu
          228
(2) INFORMATION FOR SEQ ID NO:5:
   (i) SEQUENCE CHARACTERISTICS:
      (A) LENGTH: 37 base pairs
      (B) TYPE: Nucleic Acid
      (C) STRANDEDNESS: Single
      (D) TOPOLOGY: Linear
   (xi) SEQUENCE DESCRIPTION: SEQ ID NO:5:
   CCCTCGAGGT CGACCAYMGN GAYYTNGCNR CNMGNAA 37
(2) INFORMATION FOR SEQ ID NO:6:
   (i) SEQUENCE CHARACTERISTICS:
      (A) LENGTH: 37 base pairs
      (B) TYPE: Nucleic Acid
      (C) STRANDEDNESS: Single
      (D) TOPOLOGY: Linear
   (xi) SEQUENCE DESCRIPTION: SEQ ID NO:6:
   CCCTCGAGGT CGACCAYMGN GAYYTNGCNT GYMGNAA 37
(2) INFORMATION FOR SEQ ID NO:7:
   (i) SEQUENCE CHARACTERISTICS:
      (A) LENGTH: 31 base pairs
      (B) TYPE: Nucleic Acid
      (c) STRANDEDNESS: Single
      (D) TOPOLOGY: Linear
   (xi) SEQUENCE DESCRIPTION: SEQ ID NO:7:
   CCGCGGTGCG GCCGCCRWAN SHCCANACRT C 31
(2) INFORMATION FOR SEQ ID NO:8:
   (i) SEQUENCE CHARACTERISTICS:
      (A) LENGTH: 21 base pairs
      (B) TYPE: Nucleic Acid
      (C) STRANDEDNESS: Single
      (D) TOPOLOGY: Linear
   (xi) SEQUENCE DESCRIPTION: SEQ ID NO:8:
   TCTGTGACAG ACGATCCTCC C 21
(2) INFORMATION FOR SEQ ID NO:9:
   (i) SEQUENCE CHARACTERISTICS:
      (A) LENGTH: 54 base pairs
      (B) TYPE: Nucleic Acid
      (C) STRANDEDNESS: Single
      (D) TOPOLOGY: Linear
   (xi) SEQUENCE DESCRIPTION: SEQ ID NO:9:
   GCACGGTGGA CATGTGTGAG TTTTGTCCTG GCTTTGATCA TTAGATGCTG 50
   CAAC 54
(2) INFORMATION FOR SEQ ID NO:10:
   (i) SEQUENCE CHARACTERISTICS:
      (A) LENGTH: 54 base pairs
      (B) TYPE: Nucleic Acid
      (C) STRANDEDNESS: Single
      (D) TOPOLOGY: Linear
   (xi) SEQUENCE DESCRIPTION: SEQ ID NO:10:
   GTTGCAGCAT CTAATGATCA AAGCCAGGAC AAAACTCACA CATGTCCACC 50
   GTGC 54
(2) INFORMATION FOR SEQ ID NO:11:
   (i) SEQUENCE CHARACTERISTICS:
      (A) LENGTH: 18 base pairs
      (B) TYPE: Nucleic Acid
      (C) STRANDEDNESS: Single
      (D) TOPOLOGY: Linear
   (xi) SEQUENCE DESCRIPTION: SEQ ID NO:11:
   GCACTTGTAC TCCTTGCC 18
(2) INFORMATION FOR SEQ ID NO:12:
   (i) SEQUENCE CHARACTERISTICS:
      (A) LENGTH: 28 amino acids
      (B) TYPE: Amino Acid
      (D) TOPOLOGY: Linear
   (xi) SEQUENCE DESCRIPTION: SEQ ID NO:12:
   Asp Arg Tyr Ala Val Tyr Trp Asn Ser Ser Asn Pro Arg Phe Gln
   1 5 10 15
   Arg Gly Asp Tyr His Asn Asp Val Xaa Asn Asn Asp Tyr
   20 25 28
(2) INFORMATION FOR SEQ ID NO:13:
   (i) SEQUENCE CHARACTERISTICS:
      (A) LENGTH: 24 amino acids
      (B) TYPE: Amino Acid
      (D) TOPOLOGY: Linear
   (xi) SEQUENCE DESCRIPTION: SEQ ID NO:13:
   Met Lys Thr Asn Gly Val His Asp Arg Val Phe Asp Val Asn Asp
   1 5 10 15
   Lys Val Glu Asn Xaa Leu Glu Pro Ala
   20 24
(2) INFORMATION FOR SEQ ID NO:14:
   (i) SEQUENCE CHARACTERISTICS:
      (A) LENGTH: 23 amino acids
      (B) TYPE: Amino Acid
      (D) TOPOLOGY: Linear
   (xi) SEQUENCE DESCRIPTION: SEQ ID NO:14:
   Val Asn Phe Asp Gly Tyr Ser Ala Xaa Asp His Thr Ser Lys Gly
   1 5 10 15
   Phe Lys Arg Xaa Glu Xaa Asn Arg
   20 23
(2) INFORMATION FOR SEQ ID NO:15:
   (i) SEQUENCE CHARACTERISTICS:
      (A) LENGTH: 27 amino acids
      (B) TYPE: Amino Acid
      (D) TOPOLOGY: Linear
   (xi) SEQUENCE DESCRIPTION: SEQ ID NO:15:
   Phe Gln Leu Phe Thr Pro Phe Ser Leu Gly Xaa Glu Xaa Arg Xaa
   1 5 10 15
   Gly Arg Glu Xaa Phe Tyr Asn Ser Xaa Ala Asn Pro
   20 25 27
(2) INFORMATION FOR SEQ ID NO:16:
   (i) SEQUENCE CHARACTERISTICS:
      (A) LENGTH: 8 amino acids
      (B) TYPE: Amino Acid
      (D) TOPOLOGY: Linear
   (xi) SEQUENCE DESCRIPTION: SEQ ID NO:16:
   Lys Arg Trp Glu Cys Asn Arg Pro
   1 5 8
(2) INFORMATION FOR SEQ ID NO:17:
   (i) SEQUENCE CHARACTERISTICS:
      (A) LENGTH: 37 base pairs
      (B) TYPE: Nucleic Acid
      (C) STRANDEDNESS: Single
      (D) TOPOLOGY: Linear
   (xi) SEQUENCE DESCRIPTION: SEQ ID NO:17:
   CCCTCGAGGT CGACGAYMGN TAYGCNGTNT AYTGGAA 37
(2) INFORMATION FOR SEQ ID NO:18:
   (i) SEQUENCE CHARACTERISTICS:
      (A) LENGTH: 42 base pairs
      (B) TYPE: Nucleic Acid
      (C) STRANDEDNESS: Single
      (D) TOPOLOGY: Linear
   (xi) SEQUENCE DESCRIPTION: SEQ ID NO:18:
   CCGCGGTGCG GCCGCTCTAG ARTANCCRTC RAARTTNACC AT 42

## Description

### Field of the Invention

This application relates to the production of polypeptides involved in neuronal survival and/or growth and in angiogenesis, in particular the production of purified forms thereof by means of recombinant DNA technology.

### Background of the Invention

A number of protein neurotrophic factors, or neurotrophins, have been identified which influence growth and development of the vertebrate nervous system. It is believed that these factors play an important role in promoting the differentiation, survival, and function of diverse groups of neurons in the brain and periphery.

The belief that neurotrophic factors have important signalling functions in neural tissues is based upon the precedent established by work with nerve growth factor (NGF). NGF has been shown, both *in vitro* and *in vivo*, to support the survival of sympathetic, sensory, and basal forebrain neurons. Administration of exogenous NGF rescues neurons from cell death during development, Conversely, removal or sequestration of endogenous NGF by administration of anti-NGF antibodies promotes such cell death. Heumann. *J. Exp. Biol*. 132:133-150 (1987); Hefti, *J. Neurosci*. 6:2155-2162 (1986); Thoenen and Barde, *Annu. Rev. Physiol.* 60:284-335 (1980).

Additional neurotrophic factors related to NGF have since been identified. These include brain-derived neurotrophic factor (BDNF) (Leibrock, et al., *Nature* 341:149-152 (1989)), neurotrophin-3 (NT-3) (Kaisho, et al., *FEBS Lett*. 266:187 (1990); Maisonpierre, et al., *Science* 247:1446 (1990); Rosenthal, et al., *Neuron* 4:767 (1990)), and neurotrophin 4/5 (NT-4/5) (Berkmeier, et al., *Neuron* 7:857-866 (1991)).

Neurotrophins, similar to other polypeptide growth factors, affect their target cells through interactions with cell surface receptors. According to our current understanding, two kinds of transmembrane glycoproteins act as receptors for the known neurotrophins. Equilibrium binding studies have shown that neurotrophin-responsive neuronal cells possess a common low molecular weight (65,000 - 80,000 Daltons), low affinity receptor, typically referred to as p75^{LNGFR} or p75, and high molecular weight (130,000 - 150,000 Daltons), high and low affinity receptors that are members of the trk family of receptor tyrosine kinases.

Receptor tyrosine kinases are known to serve as receptors for a variety of protein factors that promote cellular proliferation. differentiation, and survival. In addition to the trk receptors, examples of other receptor tyrosine kinases include the receptors for epidermal growth factor (EGF), fibroblast growth factor (FGF), and platelet-derived growth factor (PDGF). Typically, these receptors span the cell membrane, with one portion of the receptor being intracellular and in contact with the cytoplasm, and another portion of the receptor being extracellular. Binding of a ligand to the extracellular portion of the receptor is believed to induce tyrosine kinase activity in the intracellular portion of the receptor, with ensuing phosphorylation of various intracellular proteins involved in cellular signalling pathways.

In addition to receptor tyrosine kinases that serve as receptors for known protein factors, many receptor-like tyrosine kinases have been identified for which no ligand is known. Examples of such "orphan" receptors include recently discovered members of the eph family, which includes eph, elk, cek5, cek7, mek4/cek4/hek, sek, hek2, and bsk (Tuzi, et al., Br. J. Cancer 69:417-421 (1994); Zhou, et al., J. Neurosci. Res. 37:129-143 (1994)). Recently, other eph family receptor ligands have been reported including B61, an Eck receptor ligand (Bartley, *et al., Nature* 368:558-560 (1994) and Pandey *et al. Science* (1995) 268:567-569), ELF-1, a Mek4 and Sek receptor ligand (Cheng *et al*. Cell (1995) 82:371-381), Ehk-1-2, LERK2, HTK-L and CEK5-L. After the discovery reported in the present application was made, a chicken AL-1 homolog termed RAGS was reported (Drescher *et al. Cell* (1995) 82:359-370).

Although eph family members are expressed in many different tissues, several family members are expressed in the nervous system or specifically in neurons (Maisonpierre, et al., Oncogene 8:3277-3288 (1993); Lai, et al., Neuron 6:691-704 (1991). In order to better understand the role of these and other orphan receptor tyrosine kinases in the nervous system, it would be useful to identify new ligands that bind to such receptors.

The present invention is based on successful research resulting in the identification, cloning, and sequencing of an eph-related tyrosine kinase receptor, referred to as REK7, and its ligand, referred to as AL-1.

### Summary of the Invention

The present invention provides isolated DNA comprising the nucleotide coding sequence for AL-1, an expression vector comprising the nucleotide coding sequence for AL-1, host cells transformed with the vector, including mammalian and bacterial host cells, and a method of using a nucleic acid molecule encoding AL-1 to effect the production of AL-1, comprising culturing a host cell transfected to express such nucleic acid molecule and recovering AL-1 from the host cell culture, as defined in the claims. In this method, preferably the host cell is transfected with an expression vector comprising the nucleotide coding sequence for AL-1.

By providing the full nucleotide coding sequence for AL-1, the invention enables the production of AL-1 by means of recombinant DNA technology, thereby making available for the first time sufficient quantities of substantially pure AL-1 protein or AL-1 antagonists for diagnostic and therapeutic uses with a variety of neurological disorders and with diseases and conditions that are angiogenesis-dependent such as solid tumors, diabetic retinopathy, rheumatoid arthritis, and wound healing.
In a preferred embodiment, the invention provides AL-1 that is free of other human proteins.

Modified and variant forms of AL-1 are produced *in vitro* by means of chemical or enzymatic treatment or *in vivo* by means of recombinant DNA technology. Such polypeptides differ from native AL-1, for example, by virtue of one or more amino acid substitutions, deletions or insertions, or in the extent or pattern of glycosylation, but substantially retain a biological activity of native AL-1.

Antibodies to AL-1 are produced by immunizing an animal with AL-1 or a fragment thereof, optionally in conjunction with an immunogenic polypeptide, and thereafter recovering antibodies from the serum of the immunized animals. Alternatively, monoclonal antibodies are prepared from cells of the immunized animal in conventional fashion. Antibodies obtained by routine screening will bind to AL-1 but will not substantially bind to (i.e., cross react with) NGF, BDNF, NT-3, NT-4/5, or other neurotrophic factors. Immobilized anti-AL-1 antibodies are particularly useful in the detection of AL-1 in clinical samples for diagnostic purposes, and in the purification of AL-1.

AL-1, its derivatives, or its antibodies are formulated with physiologically acceptable carriers, especially for therapeutic use. Such carriers are used, for example, to provide sustained-release formulations of AL-1.

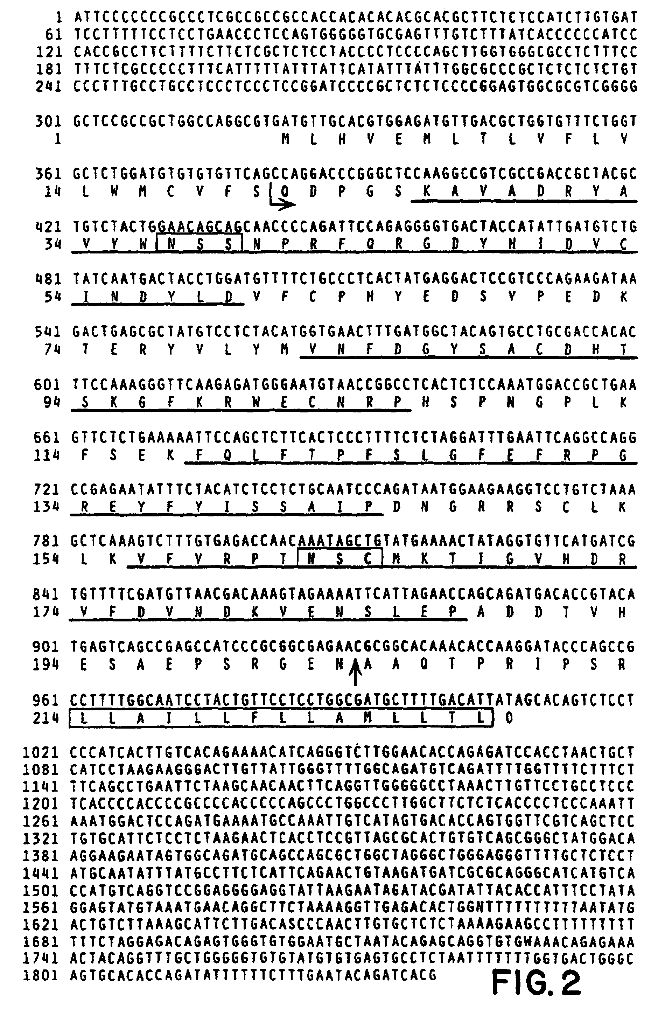

In further aspects, the invention provides a method comprising:
(a) contacting nucleic acid from a cell or a tissue with a labelled DNA which comprises a sequence of at least 15 nucleotides selected from the nucleotide sequence shown in Figure 2 and which is unique to AL-1, under conditions which will allow hybridization of complementary nucleotide sequences specific to AL-1 or its alleles; and
(b) detecting any hybridization which occurs.

DNA comprising all or a portion of the nucleotide coding sequence for AL-1 is also used in hybridisation assays to identify and to isolate nucleic acids sharing substantial sequence identity to the coding sequence for AL-1, such as nucleic acids that encode allelic variants of AL-1.

Also provided is a method comprising:
(a) contacting nucleic acid from a cell or a tissue with a DNA which comprises a sequence of at least 15 nucleotides selected from the nucleotide sequence shown in Figure 2 and which is unique to AL-1, under conditions which will allow hybridization of complementary nucleotide sequences specific to AL-1 or its alleles;
(b) amplifying in a polymerase chain reaction the nucleic acid to which the DNA of step (a) hybridizes; and
(c) detecting any nucleic acid produced by the polymerase chain reaction of step (b).

### Brief Description of the Drawings

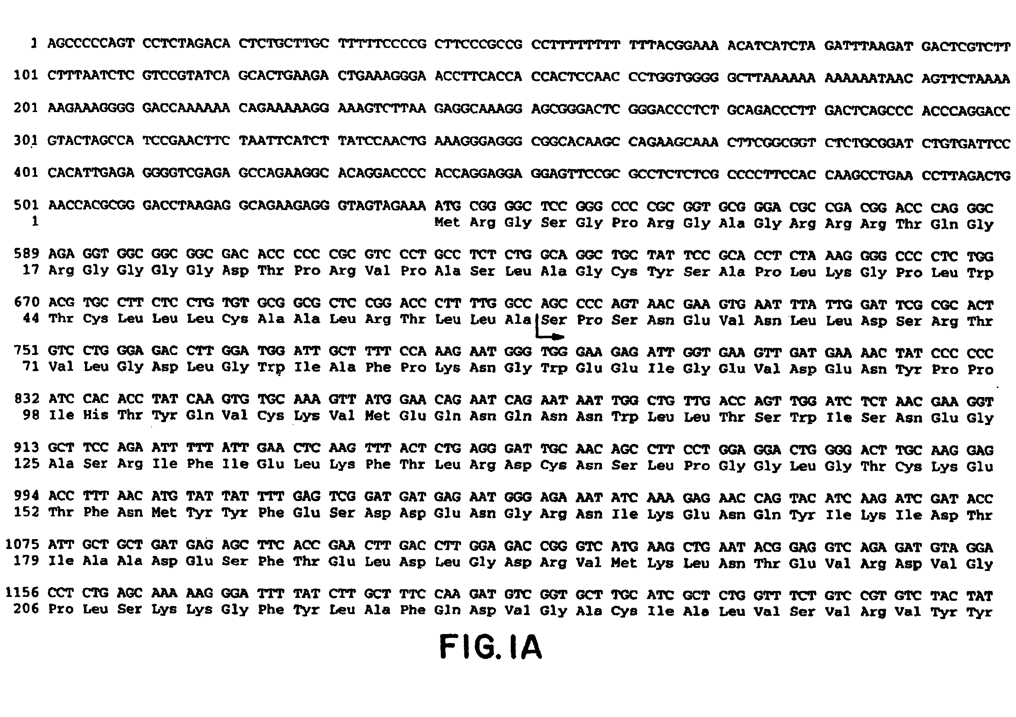

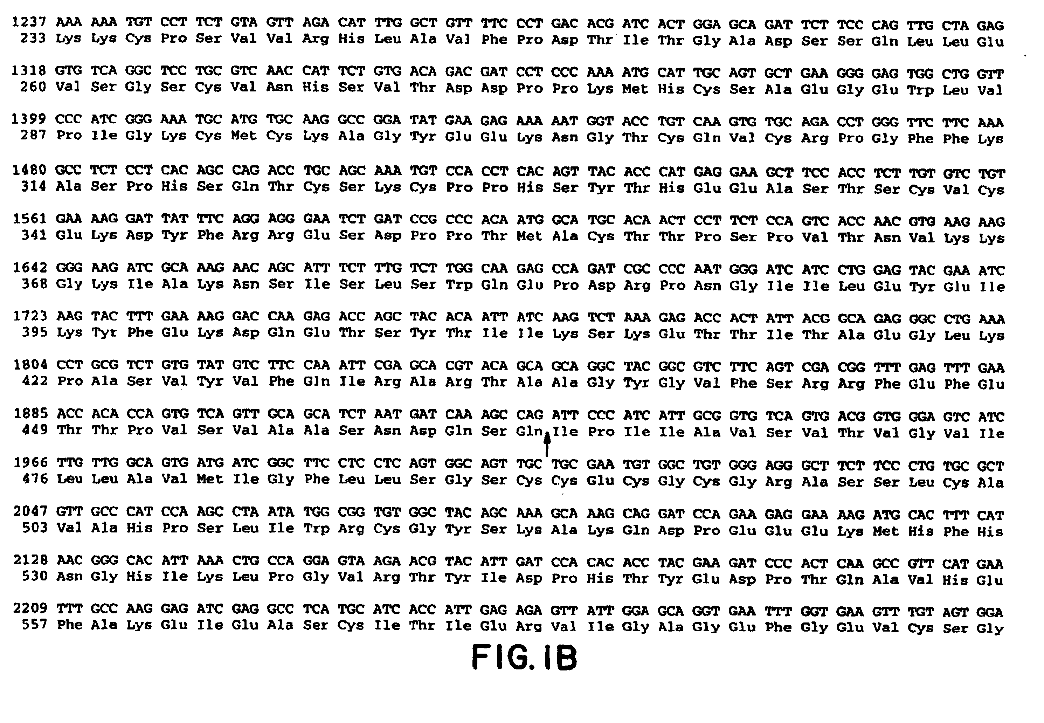

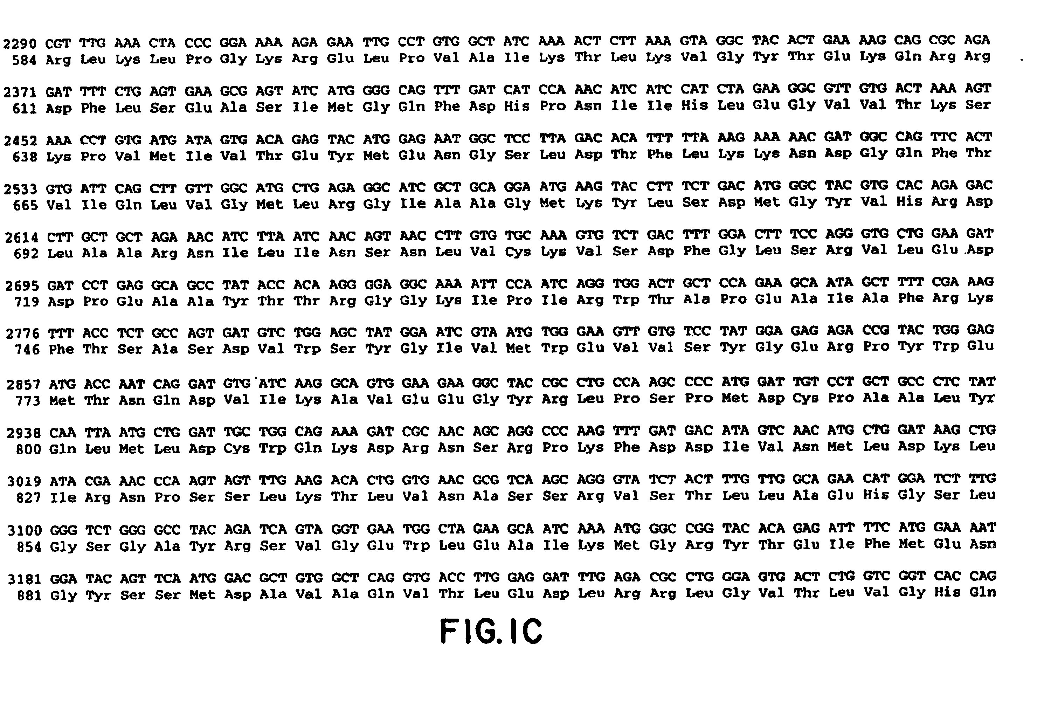

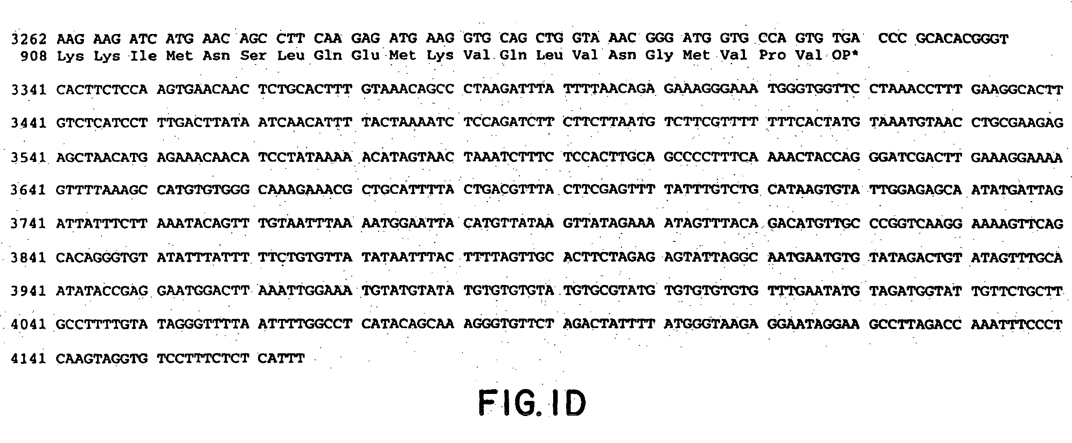

Figure 1 shows the nucleotide coding sequence (SEQ. ID. NO: 1), and the deduced amino acid sequence (SEQ. ID. NO: 2) encloded by the isolated REK7 cDNA. The N-terminus of the mature REK7 protein is indicated by a rightward arrow and the C-terminus of he REK7 extracellular domain is indicated by a vertical arrow.

Figure 2 shows the nucleotide coding sequence (SEQ. ID. NO: 3), and the deduced amino acid sequence (SEQ. ID. NO: 4) encoded by the isolated AL-1 cDNA. The N-terminus of the mature AL-1 protein is indicated bya rightward arrow (the mature protein begins with amino acid residue number 21). The underlined sequences corresponds to the sequences obtained by sequencing of purified BT20 cell-derived AL-1. The shaded boxes indicate potential N-glycosylation sites. The unshaded box shows the C-terminal hydrophobic domain and the upward arrow indicates a potential attachment site for glycophosphatidyl-inositol (GPI). The anchor is likely to be at Asn-103. As taught herein AL-1 expressed on the surface of BT20 cells is GPI anchored.

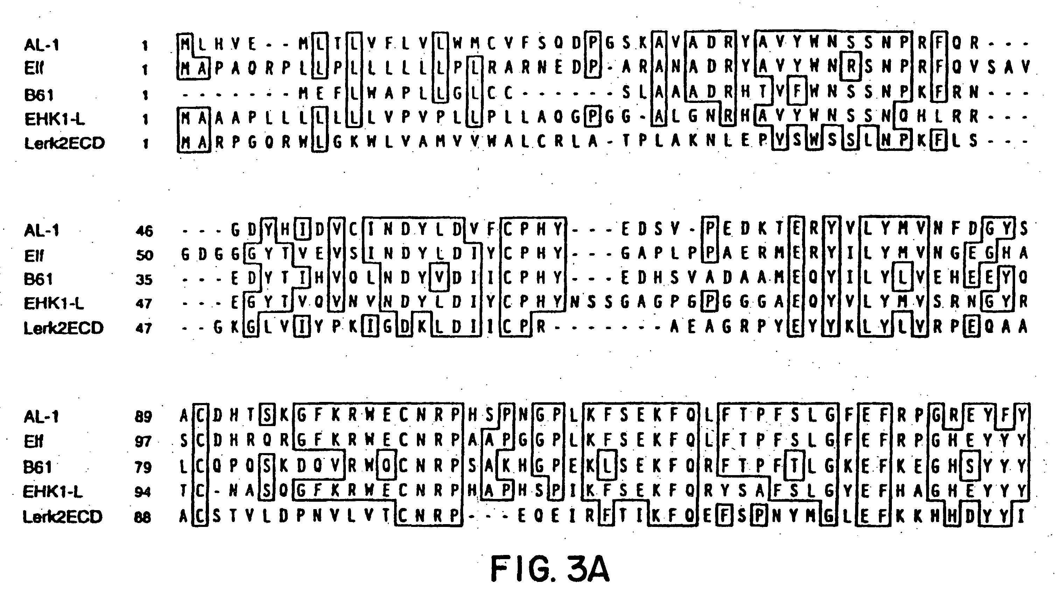

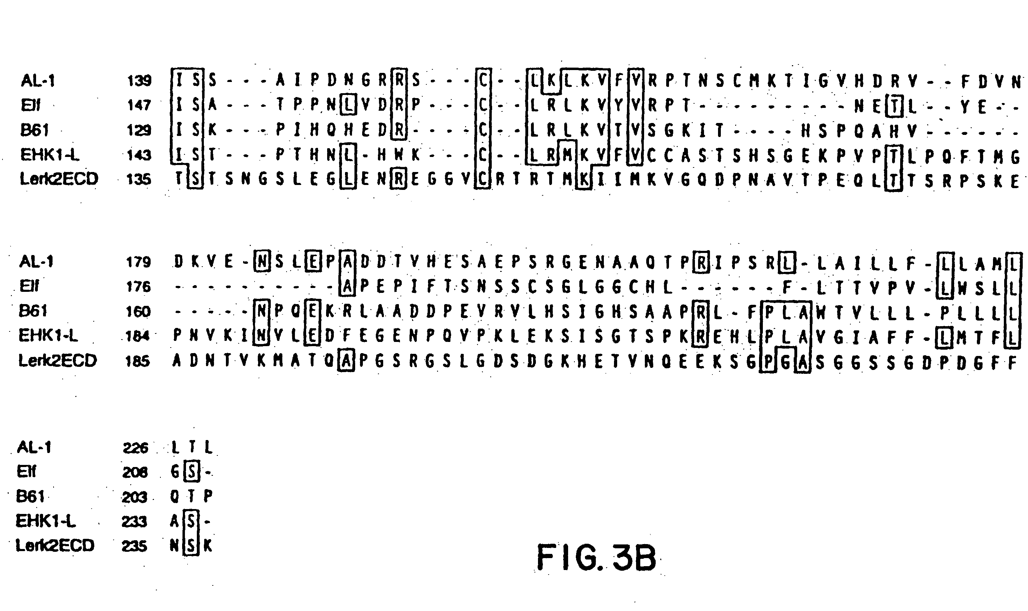

Figure 3 shows a comparison of the AL-1 sequence with that of ELF-1 (Cheng and Flanagan, *Cell* 79:157-168 (1994)), B61 (Bartley et al., *Nature* 368:558-560 (1994)). EHK-1-L (Davis et al. *Science* 266:816-819 (1994)) and the extracellular domain of LERK2 (Beckmann et al., *EMBO J*. 13:3757-3762 (1994)). Identical amino acids are boxed, and gaps introduced for optimal alignment are indicated by dashes. Shaded areas highlight conserved cysteine residues.

### Detailed Description of the Preferred Embodiments

"AL-1" or "AL-1 protein" refers to a polypeptide or protein encoded by the AL-1 nucleotide sequence set forth in Figure 2; a polypeptide that is the translated amino acid sequence set forth in Figure 2; fragments thereof having greater than about 5 amino acid residues and comprising an immune epitope or other biologically active site of AL-1; amino acid sequence variants of the amino acid sequence set forth in Figure 2 wherein one or more amino acid residues are added at the N- or C-terminus of, or within, said Figure 2 sequence or its fragments as defined above; amino acid sequence variants of said Figure 2 sequence or its fragments as defined above wherein one or more amino acid residues of said Figure 2 sequence or fragment thereof are deleted, and optionally substituted by one or more amino acid residues; and derivatives of the above proteins, polypeptides, or fragments thereof, wherein an amino acid residue has been covalently modified so that the resulting product is a non-naturally occurring amino acid. AL-1 amino acid sequence variants may be made synthetically, for example, by site-directed or PCR mutagenesis, or may exist naturally, as in the case of allelic forms and other naturally occurring variants of the translated amino acid sequence set forth in Figure 2 that may occur in human and other animal species. In any event, such fragments, variants, and derivatives exclude any polypeptide heretofore identified, including any known neurotrophic factor, such as nerve growth factor (NGF), brain derived neurotrophic factor (BDNF), neurotrophin-3 (NT-3), and neurotrophin-4/5 (NT-4/5), as well as statutorily obvious variants thereof.

An AL-1 amino acid sequence variant is included within the scope of the invention provided that it is functionally active. As used herein, "functionally active" and "functional activity" in reference to AL-1 means that the AL-1 is able to promote or enhance the growth, survival, function, and/or differentiation of neurons and glia, especially axon fasciculation and process outgrowth, whether the neurons be central, peripheral, motomeurons, or sensory neurons, e.g. photoreceptors, vestibular ganglia, spinal ganglia, auditory hair cells, and/or that the AL-1 is immunologically cross-reactive with an antibody directed against an epitope of naturally occurring AL-1. Therefore, AL-1 amino acid sequence variants generally will share at least about 75% (preferably greater than 80% and more preferably greater than 90%) sequence identity with the translated amino acid sequence set forth in Figure 2, after aligning the sequences to provide for maximum homology, as determined, for example, by the Fitch, et al., *Proc. Nat, Acad Sci. USA* 80:1382-1386 (1983), version of the algorithm described by Needleman, et al., *J. Mol. Biol*. 48:443-453 (1970).

Amino acid sequence variants of AL-1 are prepared by introducing appropriate nucleotide changes into AL-1 DNA and thereafter expressing the resulting modified DNA in a host cell, or by *in vitro* synthesis. Such variants include, for example, deletions from, or insertions or substitutions of, amino acid residues within the AL-1 amino acid sequence set forth in Figure 2. Any combination of deletion, insertion, and substitution may be made to arrive at an amino acid sequence variant of AL-1, provided that such variant possesses the desired characteristics described herein. Changes that are made in the amino acid sequence set forth in Figure 2 to arrive at an amino acid sequence variant of AL-1 also may result in further modifications of AL-1 upon its expression in host cells, for example, by virtue of such changes introducing or moving sites of glycosylation, or introducing membrane anchor sequences as described, for example, in PCT Pat. Pub. No. WO 89/01041 (published February 9, 1989).

There are two principal variables in the construction of amino acid sequence variants of AL-1: the location of the mutation site and the nature of the mutation. These are variants from the amino acid sequence set forth in Figure 2, and may represent naturally occurring allelic forms of AL-1, or predetermined mutant forms of AL-1 made by mutating AL-1 DNA, either to arrive at an allele or a variant not found in nature. In general, the location and nature of the mutation chosen will depend upon the AL-1 characteristic to be modified.

For example, due to the degeneracy of nucleotide coding sequences, mutations can be made in the AL-1 nucleotide sequence set forth in Figure 2 without affecting the amino acid sequence of the AL-1 encoded thereby. Other mutations can be made that will result in a AL-1 that has an amino acid sequence different from that set forth in Figure 2, but which is functionally active. Such functionally active amino acid sequence variants of AL-1 are selected, for example, by substituting one or more amino acid residues in the amino acid sequence set forth in Figure 2 with other amino acid residues of a similar or different polarity or charge.

One useful approach is called "alanine scanning mutagenesis." Here, a an amino acid residue or group of target residues are identified (e.g., charged residues such as arg, asp, his, lys, and glu) and, by means of recombinant DNA technology, replaced by a neutral or negatively charged amino acid (most preferably alanine or polyalanine) to affect the interaction of the amino acids with the surrounding aqueous environment in or outside the cell. Cunningham, et al., Science 244: 1081-1085(1989). Those domains demonstrating functional sensitivity to the substitutions then are refined by introducing further or other variants at or for the sites of substitution. Obviously, such variations that, for example, convert the amino acid sequence set forth in Figure 2 to the amino acid sequence of a known neurotrophic factor, such as NGF, BDNF, NT-3, NT-4/5, or another known polypeptide or protein (see Figure 3) are not included within the scope of this invention, nor are any other fragments, variants, and derivatives of the amino acid AL-1 that are not novel and unobvious over the prior art. Thus, while the site for introducing an amino acid sequence variation is predetermined, the nature of the mutation per se need not be predetermined. For example, to optimize the performance of a mutation at a given site, ala scanning or random mutagenesis is conducted at the target codon or region and the expressed AL-1 variants are screened for functional activity.

Amino acid sequence deletions generally range from about 1 to 30 residues, more preferably about 1 to 10 residues, and typically are contiguous. Deletions from regions of substantial homology with other tyrosine kinase receptor ligands, for example, are more likely to affect the functional activity of AL-1. Generally, the number of consecutive deletions will be selected so as to preserve the tertiary structure of AL-1 in the affected domain, e.g., beta-pleated sheet or alpha helix.

Amino acid sequence insertions include amino- and/or carboxyl-terminal fusions ranging in length from one amino acid residue to polypeptides containing a hundred or more residues, as well as intrasequence insertions of single or multiple amino acid residues. Intrasequence insertions (i.e.. insertions made within the amino acid sequence set forth in Figure 2) may range generally from about 1 to 10 residues, more preferably 1 to 5, most preferably 1 to 3. Examples of terminal insertions include AL-1 with an N-terminal methionyl residue (such as may result from the direct expression of AL-1 in recombinant cell culture), and AL-1 with a heterologous N-terminal signal sequence to improve the secretion of AL-1 from recombinant host cells. Such signal sequences generally will be homologous to the host cell used for expression of AL-1, and include STII or Ipp for *E*. *coli,* alpha factor for yeast, and viral signals such as herpes gD for mammalian cells. Other insertions include the fusion to the N- or C-terminus of AL-1 of immunogenic polypeptides (for example, bacterial polypeptides such as beta-lactamase or an enzyme encoded by the *E. coli* trp locus, or yeast protein), and C-terminal fusions with proteins having a long half-life such as immunoglobulin constant regions, albumin, or ferritin, as described in PCT Pat. Pub. No. WO 89/02922 (published April 6, 1989).

The third group of variants are those in which at least one amino acid residue in the amino acid sequence set forth in Figure 2, preferably one to four, more preferably one to three, even more preferably one to two, and most preferably only one, has been removed and a different residue inserted in its place. The sites of greatest interest for making such substitutions are in the regions of the amino acid sequence set forth in Figure 2 that have the greatest homology with other tyrosine kinase receptor ligands (for non-limiting examples, see Figure 3). Those sites are likely to be important to the functional activity of the AL-1. Accordingly, to retain functional activity, those sites, especially those falling within a sequence of at least three other identically conserved sites, are substituted in a relatively conservative manner. Such conservative substitutions are shown in Table 1 under the heading of preferred substitutions. If such substitutions do not result in a change in functional activity, then more substantial changes, denominated exemplary substitutions in Table 1, or as further described below in reference to amino acid classes, may be introduced and the resulting variant AL-1 analyzed for functional activity.

**Table 1**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro | pro |
| His (H) | asn; gln; lys; arg | arg |
| Ile (I) | leu; val; met; ala; phe; | |
| | norleucine | leu |
| Leu (L) | norleucine; ile; val; | |
| | met; ala; phe | ile |
| Lys (K) | arg; gln; asn | arg |
| Met (M) | leu; phe; ile | leu |
| Phe (F) | leu; val; ile; ala | leu |
| Pro (P) | gly | gly |
| Ser (S) | thr | thr |
| Thr (T) | ser | ser |
| Trp (W) | tyr | tyr |
| Tyr (Y) | trp; phe; thr; ser | phe |
| Val (V) | ile; leu; met; phe; | |
| | ala; norleucine | leu |

Insertional, deletional, and substitutional changes in the amino acid sequence set forth in Figure 2 may be made to improve the stability of AL-1. For example, trypsin or other protease cleavage sites are identified by inspection of the encoded amino acid sequence for an arginyl or lysinyl residue. These are rendered inactive to protease by substituting the residue with another residue, preferably a basic residue such as glutamine or a hydrophobic residue such as serine; by deleting the residue; or by inserting a prolyl residue immediately after the residue. Also, any cysteine residues not involved in maintaining the proper conformation of AL-1 for functional activity may be substituted, generally with serine, to improve the oxidative stability of the molecule and prevent aberrant crosslinking.

Figure 3 shows a comparison of AL-1 sequence with that of other sequences and indicates AL-1 is a new member of an emerging ligand family. ELF-1 is a ligand for the Eph-related receptors HEK and SEK, and is the most closely related to AL-1 (50.5% identity). B61 shows 42.9% similarity and EHKL-1, a reported ligand for EHK-1/REK7, shows 41.4% identity. Accordingly, additional sites for mutation in AL-1 (or alternatively in REK7) are those sites that are conserved in AL-1 (or alternatively REK7) amongst species variants of AL-1 (or REK7) but are not conserved between AL-1 and another ligand in the eph family, preferably between AL-1 and at least two ligands, and more preferably at least three ligands. Such sites are candidates sites for modulating receptor specificity and selectivity.

Covalent modifications of AL-1 molecules also are included within the scope of this invention. For example, covalent modifications are introduced into AL-1 by reacting targeted amino acid residues of the AL-1 with an organic derivatizing agent that is capable of reacting with selected amino acid side chains or the N- or C-terminal residues.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing α-amino-containing residues include imidoesters such as methyl picolinimidate; pyridoxal phosphate; pyridoxal; chloroborohydride; trinitrobenzenesulfonic acid; O-methylisourea; 2,4-pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acerylimidizole and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay, the chloramine T method described above being suitable.

Carboxyl side groups (aspartyl or glutamyl) are selectively modified by reaction with carbodiimides (R'-N=C=N-R'), where R and R' are different alkyl groups, such as 1-cyclohexyl-3-(2-morpholinyl-4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Derivatization with bifunctional agents is useful for crosslinking AL-1 to a water-insoluble support matrix or surface for use in the method for purifying anti-AL-1 antibodies, or for therapeutic use. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-[(p-azidophenyl)dithio]propioimidate yield photoactivatable intermediates that are capable of forming crosslinks in the presence of light. Alternatively, reactive water-insoluble matrices such as cyanogen bromide-activated carbohydrates and the reactive substrates described in U.S. Pat. Nos. 3,969,287; 3,691,016; 4,195,128; 4,247,642; 4,229,537; and 4,330,440 are employed for protein immobilization.

Glutaminyl and asparaginyl residues are frequency deamidated to the corresponding glutamyl and aspartyl residues, respectively. Alternatively, these residues are deamidated under mildly acidic conditions. Either form of these residues falls within the scope of this invention.

Other modifications include hydroxylation of proline and lysine, phosphorylation of hydroxyl groups of seryl or threonyl residues, methylation of the α-amino groups of lysine, arginine, and histidine side chains, acetylation of the N-terminal amine, and amidation of any C-terminal carboxyl group. Creighton, Proteins: Structure and Molecular Properties, pp.79-86 (W.H. Freeman & Co., 1983). AL-1 also is covalently linked to nonproteinaceous polymers, e.g. polyethylene glycol, polypropylene glycol or polyoxyalkylenes, in the manner set forth in U.S. Pat. Nos. 4,179,337; 4,301,144; 4,496,689; 4,640,835; 4,670,417; or 4,791,192.

"AL-1 antagonist" or "antagonist" refers to a substance that opposes or interferes with a functional activity of AL-1.

"Cell," "host cell," "cell line," and "cell culture" are used interchangeably and all such terms should be understood to include progeny. Thus, the words "transformants" and "transformed cells" include the primary subject cell and cultures derived therefrom without regard for the number of times the cultures have been passaged. It should also be understood that all progeny may not be precisely identical in DNA content, due to deliberate or inadvertent mutations.

"Plasmids" are DNA molecules that are capable of replicating within a host cell, either extrachromosomally or as part of the host cell chromosome(s), and are designated by a lower case "p" preceded and/or followed by capital letters and/or numbers. The starting plasmids herein are commercially available, are publicly available on an unrestricted basis, or can be constructed from such available plasmids as disclosed herein and/or in accordance with published procedures. In certain instances, as will be apparent to the ordinarily skilled artisan, other plasmids known in the art may be used interchangeably with plasmids described herein.

"Control sequences" refers to DNA sequences necessary for the expression of an operably linked nucleotide coding sequence in a particular host cell. The control sequences that are suitable for expression in prokaryotes, for example, include origins of replication, promoters, ribosome binding sites, and transcription termination sites. The control sequences that are suitable for expression in eukaryotes, for example, include origins of replication, promoters, ribosome binding sites, polyadenylation signals, and enhancers.

An "exogenous" element is one that is foreign to the host cell, or homologous to the host cell but in a position within the host cell in which the element is ordinarily not found.

"Digestion" of DNA refers to the catalytic cleavage of DNA with an enzyme that acts only at certain locations in the DNA. Such enzymes are called restriction enzymes or restriction endonucleases, and the sites within DNA where such enzymes cleave are called restriction sites. If there are multiple restriction sites within the DNA, digestion will produce two or more linearized DNA fragments (restriction fragments). The various restriction enzymes used herein are commercially available and their reaction conditions, cofactors, and other requirements as established by the enzyme manufacturers are used. Restriction enzymes commonly are designated by abbreviations composed of a capital letter followed by other letters representing the microorganism from which each restriction enzyme originally was obtained and then a number designating the particular enzyme. In general, about 1 µg of DNA is digested with about 1-2 units of enzyme in about 20 µl of buffer solution. Appropriate buffers and substrate amounts for particular restriction enzymes are specified by the manufacturer, and/or are well known in the art.

"Recovery" or "isolation" of a given fragment of DNA from a restriction digest typically is accomplished by separating the digestion products, which are referred to as "restriction fragments," on a polyacrylamide or agarose gel by electrophoresis, identifying the fragment of interest on the basis of its mobility relative to that of marker DNA fragments of known molecular weight, excising the portion of the gel that contains the desired fragment, and separating the DNA from the gel, for example by electroelution.

"Ligation" refers to the process of forming phosphodiester bonds between two double-stranded DNA fragments. Unless otherwise specified, ligation is accomplished using known buffers and conditions with 10 units of T4 DNA ligase per 0.5 µg of approximately equimolar amounts of the DNA fragments to be ligated.

"Oligonucleotides" are short-length, single- or double-stranded polydeoxynucleotides that are chemically synthesized by known methods (involving, for example, triester, phosphoramidite, or phosphonate chemistry), such as described by Engels, et al., *Agnew. Chem. Int. Ed. Engl*. 28:716-734 (1989). They are then purified, for example, by polyacrytamide gel electrophoresis.

"Polymerase chain reaction," or "PCR," as used herein generally refers to a method for amplification of a desired nucleotide sequence *in vitro*, as described in U.S. Pat. No. 4,683,195. In general, the PCR method involves repeated cycles of primer extension synthesis, using two oligonucleotide primers capable,of hybridizing preferentially to a template nucleic acid. Typically, the primers used in the PCR method will be complementary to nucleotide sequences within the template at both ends of or flanking the nucleotide sequence to be amplified, although primers complementary to the nucleotide sequence to be amplified also may be used. Wang, et al., in PCR Protocols, pp.70-75 (Academic Press, 1990); Ochman, et al., in PCR Protocols, pp. 219-227; Triglia, et al., *Nuc. Acids Res.* 16:8186 (1988).

"PCR cloning" refers to the use of the PCR method to amplify a specific desired nucleotide sequence that is present amongst the nucleic acids from a suitable cell or tissue source, including total genomic DNA and cDNA transcribed from total cellular RNA. Frohman, et al., *Proc. Nat. Acad. Sci. USA* 85:8998-9002 (1988); Saiki, et al., *Science* 239:487-492 (1988); Mullis, et al., *Meth. Enzymol*. 155:335-350(1987).

"Stringent conditions" for hybridization or annealing of nucleic acid molecules are those that (1) employ low ionic strength and high temperature for washing, for example, 0.015 M NaCl/0.0015 M sodium citrate/0.1% sodium dodecyl sulfate (SDS) at 50°C, or (2) employ during hybridization a denaturing agent such as formamide, for example, 50% (vol/vol) formamide with 0.1% bovine serum albumin/0.1% Ficoll/0.1% polyvinylpyrrolidone/50 mM sodium phosphate buffer at pH 6.5 with 750 mM NaCl, 75 mM sodium citrate at 42°C. Another example is use of 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/mL), 0.1% SDS, and 10% dextran sulfate at 42°C, with washes at 42°C in 0.2 x SSC and 0.1% SDS.

"AL-1 nucleic acid" is RNA or DNA that encodes AL-1. "AL-1 DNA" is DNA that encodes AL-1. AL-1 DNA is obtained from cDNA or genomic DNA libraries, or by *in vitro* synthesis. Identification of AL-1 DNA within a cDNA or a genomic DNA library, or in some other mixture of various DNAs, is conveniently accomplished by the use of an oligonucleotide hybridization probe that is labeled with a detectable moiety, such as a radioisotope. Keller, et al., *DNA Probes*, pp.149-213 (Stockton Press, 1989). To identify DNA encoding AL-1, the nucleotide sequence of the hybridization probe preferably is selected so that the hybridization probe is capable of hybridizing preferentially to DNA encoding the AL-1 amino acid sequence set forth in Figure 2, or a variant or derivative thereof as described herein, under the hybridization conditions chosen. Another method for obtaining AL-1 nucleic acid is to chemically synthesize it using one of the methods described, for example, by Engels, et al., *Agnew. Chem. Int. Ed Engl.* 28:716-734 (1989).

If the entire nucleotide coding sequence for AL-1 is not obtained in a single cDNA, genomic DNA. or other DNA, as determined, for example, by DNA sequencing or restriction endonuclease analysis, then appropriate DNA fragments (e.g., restriction fragments or PCR amplification products) may be recovered from several DNAs and covalently joined to one another to construct the entire coding sequence. The preferred means of covalently joining DNA fragments is by ligation using a DNA ligase enzyme, such as T4 DNA ligase.

"Isolated" AL-1 nucleic acid is AL-1 nucleic acid that is identified and separated from (or otherwise substantially free from), contaminant nucleic acid encoding other polypeptides. The isolated AL-1 nucleic acid can be incorporated into a plasmid or expression vector, or can be labeled for diagnostic and probe purposes, using a label as described further herein in the discussion of diagnostic assays and nucleic acid hybridization methods.

For example, isolated AL-1 DNA, or a fragment thereof comprising at least about 15 nucleotides, is used as a hybridization probe to detect, diagnose, or monitor disorders or diseases that involve changes in AL-1 expression, such as may result from neuron damage. In one embodiment of the invention, total RNA in a tissue sample from a patient (that is, a human or other mammal) can be assayed for the presence of AL-1 messenger RNA, wherein the decrease in the amount of AL-1 messenger RNA is indicative of neuronal degeneration.

Isolated AL-1 nucleic acid also is used to produce AL-1 by recombinant DNA and recombinant cell culture methods. In various embodiments of the invention, host cells are transformed or transfected with recombinant DNA molecules comprising an isolated AL-1 DNA, to obtain expression of the AL-1 DNA and thus the production of AL-1 in large quantities. DNA encoding amino acid sequence variants of AL-1 is prepared by a variety of methods known in the art. These methods include, but are not limited to, isolation from a natural source (in the case of naturally occurring amino acid sequence variants of AL-1) or preparation by site-directed (or oligonucleotide-mediated) mutagenesis, PCR mutagenesis, and cassette mutagenesis of an earlier prepared DNA encoding a variant or a non-variant form of AL-1.

Site-directed mutagenesis is a preferred method for preparing substitution, deletion, and insertion variants of AL-1 DNA. This technique is well known in the art, Zoller, et al., *Meth Enz.* 100:4668-500 (1983); Zoller, et al., *Meth Enz.* 154:329-350 (1987); Carter, *Meth. Enz*. 154:382-403 (1987); Horwitz, et al., *Meth Enz*. 185:599-611 (1990), and has been used, for example, to produce amino acid sequence variants of trypsin and T4 lysozyme. which variants have certain desired functional properties. Perry, et al., *Science* 226:555-557 (1984); Craik, et al., *Science* 228:291-297 (1985).

Briefly, in carrying out site-directed mutagenesis of AL-1 DNA. the AL-1 DNA is altered by first hybridizing an oligonucleotide encoding the desired mutation to a single strand of such AL-1 DNA. After hybridization, a DNA polymerase is used to synthesize an entire second strand, using the hybridized oligonucleotide as a primer, and using the single strand of AL-1 DNA as a template. Thus, the oligonucleotide encoding the desired mutation is incorporated in the resulting double-stranded DNA.

Oligonucleotides for use as hybridization probes or primers may be prepared by any suitable method, such as by purification of a naturally occurring DNA or by *in vitro* synthesis. For example, oligonucleotides are readily synthesized using various techniques in organic chemistry, such as described by Narang, et al., *Meth. Enzymol.* 68:90-98 (1979); Brown, et al., *Meth. Enzymol.* 68:109-151 (1979); Caruther, et al., *Meth. Enzymol*. 154:287-313 (1985). The general approach to selecting a suitable hybridization probe or primer is well known. Keller, et al., DNA Probes, pp.11-18 (Stockton Press, 1989). Typically, the hybridization probe or primer will contain 10-25 or more nucleotides, and will include at least 5 nucleotides on either side of the sequence encoding the desired mutation so as to ensure that the oligonucleotide will hybridize preferentially to the single-stranded DNA template molecule.

Multiple mutations are introduced into AL-1 DNA to produce amino acid sequence variants of AL-1 comprising several or a combination of insertions, deletions, or substitutions of amino acid residues as compared to the amino acid sequence set forth in Figure 2. If the sites to be mutated are located close together, the mutations may be introduced simultaneously using a single oligonucleotide that encodes all of the desired mutations. If, however, the sites to be mutated are located some distance from each other (separated by more than about ten nucleotides), it is more difficult to generate a single oligonucleotide that encodes all of the desired changes. Instead, one of two alternative methods may be employed.

In the first method, a separate oligonucleotide is generated for each desired mutation. The oligonucleotides are then annealed to the single-stranded template DNA simultaneously, and the second strand of DNA that is synthesized from the template will encode all of the desired amino acid substitutions.

The alternative method involves two or more rounds of mutagenesis to produce the desired mutant. The first round is as described for introducing a single mutation: a single strand of a previously prepared AL-1 DNA is used as a template, an oligonucleotide encoding the first desired mutation is annealed to this template, and a heteroduplex DNA molecule is then generated. The second round of mutagenesis utilizes the mutated DNA produced in the first round of mutagenesis as the template. Thus, this template already contains one or more mutations. The oligonucleotide encoding the additional desired amino acid substitution(s) is then annealed to this template. and the resulting strand of DNA now encodes mutations from both the first and second rounds of mutagenesis. This resultant DNA can be used as a template in a third round of mutagenesis, and so on.

PCR mutagenesis is also suitable for making amino acid sequence variants of AL-1. Higuchi, in PCR Protocols, pp.177-183 (Academic Press, 1990); Vallette, et al., *Nuc. Acids Res.* 17:723-733 (1989). Briefly, when small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template. For introduction of a mutation into a plasmid DNA, for example, one of the primers is designed to overlap the position of the mutation and to contain the mutation; the sequence of the other primer must be identical to a nucleotide sequence within the opposite strand of the plasmid DNA, but this sequence can be located anywhere along the plasmid DNA. It is preferred, however, that the sequence of the second primer is located within 200 nucleotides from that of the first, such that in the end the entire amplified region of DNA bounded by the primers can be easily sequenced. PCR amplification using a primer pair like the one just described results in a population of DNA fragments that differ at the position of the mutation specified by the primer, and possibly at other positions, as template copying is somewhat error-prone. Wagner, et al., in PCR Topics, pp.69-71 (Springer-Verlag, 1991).

If the ratio of template to product amplified DNA is extremely low, the majority of product DNA fragments incorporate the desired mutation(s). This product DNA is used to replace the corresponding region in the plasmid that served as PCR template using standard recombinant DNA methods. Mutations at separate positions can be introduced simultaneously by either using a mutant second primer, or performing a second PCR with different mutant primers and ligating the two resulting PCR fragments simultaneously to the plasmid fragment in a three (or more)-part ligation.

Another method for preparing variants, cassette mutagenesis, is based on the technique described by Wells et al., *Gene*, 34:315-323 (1985). The starting material is the plasmid (or other vector) comprising the AL-1 DNA to be mutated. The codon(s) in the AL-1 DNA to be mutated are identified. There must be a unique restriction endonuclease site on each side of the identified mutation site(s). If no such restriction sites exist, they may be generated using the above-described oligonucleotide-mediated mutagenesis method to introduce them at appropriate locations in the AL-1 DNA. The plasmid DNA is cut at these sites to linearize it. A double-stranded oligonucleotide encoding the sequence of the DNA between the restriction sites but containing the desired mutation(s) is synthesized using standard procedures, wherein the two strands of the oligonucleotide are synthesized separately and then hybridized together using standard techniques. This double-stranded oligonucleotide is referred to as the cassette. This cassette is designed to have 5' and 3' ends that are compatible with the ends of the linearized plasmid, such that it can be directly ligated to the plasmid. This plasmid now contains the mutated AL-1 DNA sequence.

AL-1 DNA, whether cDNA or genomic DNA or a product of *in vitro* synthesis, is ligated into a replicable vector for further cloning or for expression. "Vectors" are plasmids and other DNAs that are capable of replicating autonomously within a host cell, and as such, are useful for performing two functions in conjunction with compatible host cells (a vector-host system). One function is to facilitate the cloning of the nucleic acid that encodes the AL-1, i.e., to produce usable quantities of the nucleic acid. The other function is to direct the expression of AL-1. One or both of these functions are performed by the vector-host system. The vectors will contain different components depending upon the function they are to perform as well as the host cell with which they are to be used for cloning or expression.

To produce AL-1, an expression vector will contain nucleic acid that encodes AL-1 as described above. The AL-1 of this invention is expressed directly in recombinant cell culture, or as a fusion with a heterologous polypeptide, preferably a signal sequence or other polypeptide having a specific cleavage site at the junction between the heterologous polypeptide and the AL-1.

In one example of recombinant host cell expression, mammalian cells are transfected with an expression vector comprising AL-1 DNA and the AL-1 encoded thereby is recovered from the culture medium in which the recombinant host cells are grown. But the expression vectors and methods disclosed herein are suitable for use over a wide range of prokaryotic and eukaryotic organisms.

Prokaryotes may be used for the initial cloning of DNAs and the construction of the vectors useful in the invention. However, prokaryotes may also be used for expression of DNA encoding AL-1. Polypeptides that are produced in prokaryotic host cells typically will be non-glycosylated.

Plasmid or viral vectors containing replication origins and other control sequences that are derived from species compatible with the host cell are used in connection with prokaryotic host cells, for cloning or expression of an isolated DNA. For example, *E. coli* typically is transformed using pBR322, a plasmid derived from an *E. coli* species. Bolivar, et al., *Gene* 2:95-113 (1987). PBR322 contains genes for ampicillin and tetracycline resistance so that cells transformed by the plasmid can easily be identified or selected. For it to serve as an expression vector, the pBR322 plasmid, or other plasmid or viral vector, must also contain, or be modified to contain, a promoter that functions in the host cell to provide messenger RNA (mRNA) transcripts of a DNA inserted downstream of the promoter. Rangagwala, et al., *Bio*/*Technology* 9:477-479 (1991).

In addition to prokaryotes, eukaryotic microbes, such as yeast, may also be used as hosts for the cloning or expression of DNAs useful in the invention. Saccharomyces cerevisiae, or common baker's yeast, is the most commonly used eukaryotic microorganism. Plasmids useful for cloning or expression in yeast cells of a desired DNA are well known, as are various promoters that function in yeast cells to produce mRNA transcripts.

Furthermore, cells derived from multicellular organisms also may be used as hosts for the cloning or expression of DNAs useful in the invention. Mammalian cells are most commonly used, and the procedures for maintaining or propagating such cells *in vitro*, which procedures are commonly referred to as tissue culture, are well known. Kruse & Patterson, eds., Tissue Culture (Academic Press, 1977). Examples of useful mammalian cells are human cell lines such as 293, HeLa, and WI-38, monkey cell lines such as COS-7 and VERO, and hamster cell lines such as BHK-21 and CHO, all of which are publicly available from the American Type Culture Collection (ATCC), Rockville, Maryland 20852 USA.

Expression vectors, unlike cloning vectors, should contain a promoter that is recognized by the host organism and is operably linked to the AL-1 nucleic acid. Promoters are untranslated sequences that are located upstream from the start codon of a gene and that control transcription of the gene (that is, the synthesis of mRNA). Promoters typically fall into two classes, inducible and constitutive. Inducible promoters are promoters that initiate high level transcription of the DNA under their control in response to some change in culture conditions, for example, the presence or absence of a nutrient or a change in temperature.

A large number of promoters are known, that may be operably linked to AL-1 DNA to achieve expression of AL-1 in a host cell. This is not to say that the promoter associated with naturally occurring AL-1 DNA is not usable. However, heteralogous promoters generally will result in greater transcription and higher yields of expressed AL-1.

Promoters suitable for use with prokaryotic hosts include the β-lactamase and lactose promoters, Goeddel, et al., *Nature* 281:544-548 (1979), tryptophan (trp) promoter, Goeddel, et al., *Nuc. Acids Res.* 8:4057-4074 (1980), and hybrid promoters such as the tac promoter, deBoer, et al., *Proc. Natl. Acad. Sci. USA* 80:21-25 (1983). However, other known bacterial promoters are suitable. Their nucleotide sequences have been published, Siebenlist, et al., *Cell* 20:269-281 (1980), thereby enabling a skilled worker operably to ligate them to DNA encoding AL-1 using linkers or adaptors to supply any required restriction sites. Wu, et al., *Meth. Enz*. 152:343-349 (1987).

Suitable promoters for use with yeast hosts include the promoters for 3-phosphoglycerate kinase, Hitzeman, et al., *J. Biol. Chem*. 255:12073-12080 (1980); Kingsman, et al., *Meth. Enz*. 185:329-341 (1990), or other glycolytic enzymes such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Dodson, et al., *Nuc. Acids* res. 10:2625-2637 (1982); Emr, *Meth. Enz*. 185:231-279 (1990).

Expression vectors useful in mammalian cells typically include a promoter derived from a virus. For example, promoters derived from polyoma virus, adenovirus, cytomegalovirus (CMV), and simian virus 40 (SV40) are commonly used. Further, it is also possible, and often desirable, to utilize promoter or other control sequences associated with a naturally occurring DNA that encodes AL-1, provided that such control sequences are functional in the particular host cell used for recombinant DNA expression.

Other control sequences that are desirable in an expression vector in addition to a promoter are a ribosome binding site, and in the case of an expression vector used with eukaryotic host cells, an enhancer. Enhancers are cis-acting elements of DNA, usually about from 10-300 bp, that act on a promoter to increase the level of transcription. Many enhancer sequences are now known from mammalian genes (for example, the genes for globin, elastase, albumin, α-fetoprotein and insulin). Typically, however, the enhancer used will be one from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. Kriegler, Meth. Enz. 185:512-527 (1990).

Expression vectors may also contain sequences necessary for the termination of transcription and for stabilizing the messenger RNA (mRNA). Balbas, et al., *Meth. Enz.* 185:14-37 (1990); Levinson, *Meth. Enz.* 185:485-511 (1990). In the case of expression vectors used with eukaryotic host cells, such transcription termination sequences may be obtained from the untranslated regions of eukaryotic or viral DNAs or cDNAs. These regions contain polyadenylation sites as well as transcription termination sites. Birnsteil, et al., *Cell* 41:349-359 (1985).

In general, control sequences are DNA sequences necessary for the expression of an operably liked coding sequence in a particular host cell. "Expression" refers to transcription and/or translation. "Operably linked" refers to the covalent joining of two or more DNA sequences, by means of enzymatic ligation or otherwise, in a configuration relative to one another such that the normal function of the sequences can be performed. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous and, in the case of a secretory leader, contiguous and in reading phase. Linking is accomplished by ligation at convenient restriction sites. If such sites do not exist, then synthetic oligonucleotide adaptors or linkers are used, in conjunction with standard recombinant DNA methods.

Expression and cloning vectors also will contain a sequence that enables the vector to replicate in one or more selected host cells. Generally, in cloning vectors this sequence is one that enables the vector to replicate independently of the host chromosome(s), and includes origins of replication or autonomously replicating sequences. Such sequences are well known for a variety of bacteria, yeast, and viruses. The origin of replication from the plasmid pBR322 is suitable for most gram-negative bacteria, the 2µ plasmid origin is suitable for yeast, and various viral origins (for example, from SV40, polyoma, or adenovirus) are useful for cloning vectors in mammalian cells. Most expression vectors are "shuttle" vectors, i.e. they are capable of replication in at least one class of organisms but can be transfected into another organism for expression. For example, a vector may be cloned in *E. coli* and then the same vector is transfected into yeast or mammalian cells for expression even though it is not capable of replicating independently of the host cell chromosome.

The expression vector may also include an amplifiable gene, such as that comprising the coding sequence for dihydrofolate reductase (DHFR). Cells containing an expression vector that includes a DHFR gene may be cultured in the presence of methotrexate, a competitive antagonist of DHFR. This leads to the synthesis of multiple copies of the DHFR gene and, concomitantly, multiple copies of other DNA sequences comprising the expression vector, Ringold, et al., *J. Mol. Apl. Genet*. 1: 165-175 (1981), such as a DNA sequence encoding AL-1. In that manner, the level of AL-1 produced by the cells may be increased.

DHFR protein encoded by the expression vector also may be used as a selectable marker of successful transfection. For example, if the host cell prior to transformation is lacking in DHFR activity, successful transformation by an expression vector comprising DNA sequences encoding AL-1 and DHFR protein can be determined by cell growth in medium containing methotrexate. Also, mammalian cells transformed by an expression vector comprising DNA sequences encoding AL-1, DHFR protein, and aminoglycoside 3' phosphotransferase (APH) can be determined by cell growth in medium containing an aminoglycoside antibiotic such as kanamycin or neomycin. Because eukaryotic cells do not normally express an endogenous APH activity, genes encoding APH protein, commonly referred to as neo^{r} genes, may be used as dominant selectable markers in a wide range of eukaryotic host cells, by which cells transfected by the vector can easily be identified or selected. Jiminez, et al., *Nature*, 287:869-871 (1980); Colbere-Garapin, et al., *J. Mol. Biol*. 150:1-14 (1981); Okayama & Berg. *Mol. Cell. Biol*., 3:280-289 (1983).

Many other selectable markers are known that may be used for identifying and isolating recombinant host cells that express AL-1. For example, a suitable selection marker for use in yeast is the trp1 gene present in the yeast plasmid YRp7. Stinchcomb, et al., *Nature* 282:39-43 (1979); Kingsman, et al., *Gene* 7:141-152 (1979); Tschemper, et al., *Gene* 10:157-166 (1980). The trp1 gene provides a selection marker for a mutant strain of yeast lacking the ability to grow in tryptophan, for example, ATCC No. 44076 or PEP4-1 (available from the American Type Culture Collection, Rockville, Maryland 20852 USA). Jones, *Genetics* 85:12 (1977). The presence of the trp1 lesion in the yeast host cell genome then provides an effective environment for detecting transformation by growth in the absence of tryptophan. Similarly, Leu2-deficient yeast strains (ATCC Nos. 20622 or 38626) are complemented by known plasmids bearing the Leu2 gene.

Particularly useful in the invention are expression vectors that provide for the transient expression in mammalian cells of DNA encoding AL-1. In general, transient expression involves the use of an expression vector that is able to efficiently replicate in a host cell, such that the host cell accumulates many copies of the expression vector and, in turn, synthesizes high levels of a desired polypeptide encoded by the expression vector. Transient expression systems, comprising a suitable expression vector and a host cell, allow for the convenient positive identification of polypeptides encoded by cloned DNAs, as well as for the rapid screening of such polypeptides for desired biological or physiological properties. Yang, et al., *Cell* 47:3-10 (1986); Wong; et al., *Science* 228:810-815 (1985); Lee, et al., *Proc. Nat Acad. Sci. USA* 82:4360-4364 (1985). Thus, transient expression systems are particularly useful in the invention for expressing DNAs encoding amino acid sequence variants of AL-1, to identify those variants which are functionally active.

Since it is often difficult to predict in advance the characteristics of an amino acid sequence variant of AL-1, it will be appreciated that some screening of such variants will be needed to identify those that are functionally active. Such screening may be performed *in vitro*, using routine assays for receptor binding, or assays for axonal growth or development, or using immunoassays with monoclonal antibodies that selectively bind to AL-1 that is functionally active AL-1, such as a monoclonal antibody that selectively binds to the active site or receptor binding site of AL-1.

As used herein, the terms "transformation" and "transfection" refer to the process of introducing a desired nucleic acid, such a plasmid or an expression vector, into a host cell. Various methods of transformation and transfection are available, depending on the nature of the host cell. In the case of *E. coli* cells, the most common methods involve treating the cells with aqueous solutions of calcium chloride and other salts. In the case of mammalian cells, the most common methods are transfection mediated by either calcium phosphate or DEAE-dextran, or electroporation. Sambrook, et al., eds., Molecular Cloning, pp. 1.74-1.94 and 16.30-16.55 (Cold Spring Harbor Laboratory Press, 1989). Following transformation or transfection, the desired nucleic acid may integrate into the host cell genome, or may exist as an extrachromosomal element.

Host cells that are transformed or transfected with the above-described plasmids and expression vectors are cultured in conventional nutrient media modified as is appropriate for inducing promoters or selecting for drug resistance or some other selectable marker or phenotype. The culture conditions, such as temperature, pH, and the like, suitably are those previously used for culturing the host cell used for cloning or expression, as the case may be, and will be apparent those skilled in the art.

Suitable host cells for cloning or expressing the vectors herein are prokaryotes, yeasts. and higher eukaryotes, including insect, vertebrate, and mammalian host cells. Suitable prokaryotes include eubacteria, such as Gram-negative or Gram-positive organisms, for example, E. coli, Bacillus species such as B. subtilis, Pseudomonas species such as P. aeruginosa, Salmonella typhimurium. or Serratia marcescans.

In addition to prokaryotes, eukaryotic microbes such as filamentous fungi or yeast are suitable hosts for AL-1-encoding vectors. Saccharomyces cerevisiae, or common baker's yeast, is the most commonly used among lower eukaryotic host microorganisms. However, a number of other genera, species, and strains are commonly available and useful herein, such as Schizosaccharomyces pombe, Beach and Nurse, *Nature* 290:140-142 (1981), Pichia pastoris, Cregg, et al., *Bio*/*Technology* 5:479-485 (1987); Sreekrishna, et al., *Biochemistry* 28:4117-4125 (1989), Neurospora crassa, Case, et al., *Proc. Natl. Acad. Sci. USA* 76:5259-5263 (1979), and Aspergillus hosts such as A. nidulans, Ballance, et al., *Biochem. Biophys. Res. Commun*. 112:284-289 (1983); Tilburn, et al., *Gene* 26:205-221 (1983); Yelton, et al., *Proc. Natl. Acad. Sci. USA* 81:1470-1474 (1984), and A. niger, Kelly, et al., *EMBO J*. 4:475-479 (1985).

Suitable host cells for the expression of AL-1 also are derived from multicellular organisms. Such host cells are capable of complex processing and glycosylation activities. In principle, any higher eukaryotic cell culture is useable, whether from vertebrate or invertebrate culture. It will be appreciated, however, that because of the species-, tissue-, and cell-specificity of glycosylation. Rademacher, et al., *Ann*. *Rev*. *Biochem.* 57:785-838 (1988), the extent or pattern of glycosylation of AL-1 in a foreign host cell typically will differ from that of AL-1 obtained from a cell in which it is naturally expressed.

Examples of invertebrate cells include insect and plant cells. Numerous baculoviral strains and variants and corresponding permissive insect host cells from hosts such as Spodoptera frugiperda (caterpillar), Aedes aegypti (mosquito), Aedes abopictus (mosquito), Drosophila melanogaster (fruitfly), and Bombyx mori host cells have been identified. Luckow, et al., *Bio*/*Technology* 6:47-55 (1988); Miller, et al., in Genetic Engineering, vol. 8, pp.277-279 (Plenum Publishing, 1986); Maeda. et al., *Nature* 315:592-594 (1985):

Plant cell cultures of cotton, corn, potato, soybean, petunia, tomato, and tobacco can be utilized as hosts. Typically, plant cells are transfected by incubation with certain strains of the bacterium Agrobacterium tumefaciens, which has been previously altered to contain AL-1 DNA. During incubation of the plant cells with A. tumefaciens, the DNA encoding the AL-1 is transferred into cells, such that they become transfected, and will, under appropriate conditions, express the AL-1 DNA. In addition, regulatory and signal sequences compatible with plant cells are available, such as the nopaline synthase promoter and polyadenylation signal sequences, and the ribulose biphosphate carboxylase promoter. Depicker, et at., J. Mol. Appl. Gen.1:561-573 (1982). Herrera-Estrella, et al., *Nature* 310:115-120 (1984). In addition, DNA segments isolated from the upstream region of the T-DNA 780 gene are capable of activating or increasing transcription levels of plant-expressible genes in recombinant DNA-containing plant tissue. European Pat. Pub. No. EP 321,196 (published June 21,1989).

However, interest has been greatest in vertebrate cells, and propagation of vertebrate cells in culture (tissue culture) has become a routine procedure in recent years. Kruse & Patterson, eds., Tissue Culture (Academic Press, 1973). Examples of useful mammalian host cells are the monkey kidney CV1 line transformed by SV40 (COS-7, ATCC CRL 1651); human embryonic kidney line 293 (or 293 cells subcloned for growth in suspension culture), Graham, et al., *J. Gen Virol*. 36:59-72 (1977); baby hamster kidney cells (BHK, ATCC CCL 10); Chinese hamster ovary cells (including DHFR-deficient CHO cells, Urlaub, et al., *Proc. Natl. Acad. Sci. USA* 77:4216-4220 (1980); mouse sertoli cells (TM4, Mather, Biol. Reprod. 23:243-251 (1980); monkey kidney cells (CV1, ATCC CCL 70); African green monkey kidney cells (VERO-76. ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather, et al., *Annals N.Y. Acad. Sci.* 383:44-68 (1982)); MRC 5 cells; FS4 cells; and a human hepatoma line (Hep G2).

Construction of suitable vectors containing the nucleotide sequence encoding AL-1 and appropriate control sequences employs standard recombinant DNA methods. DNA is cleaved into fragments, tailored, and ligated together in the form desired to generate the vectors required.

For analysis to confirm correct sequences in the vectors constructed, the vectors are analyzed by restriction digestion (to confirm the presence in the vector of predicted restriction endonuclease) and/or by sequencing by the dideoxy chain termination method of Sanger, et al., *Proc. Nat*. *Acad. Sci. USA* 72:3918-3921 (1979).

The mammalian host cells used to produce the AL-1 of this invention may be cultured in a variety of media. Commercially available media such as Ham's F10 (Sigma), Minimal Essential Medium (MEM, Sigma), RPMI-1640 (Sigma), and Dulbecco's Modified Eagle's Medium (DMEM, Sigma) are suitable for culturing the host cells. In addition, any of the media described in Harn, et al., Meth. Enz. 58:44-93 (1979); Barnes, et al., *Anal. Biochem.* 102:255-270 (1980); Bottenstein, et al., Meth. Enz. 58:94-109 (1979); U.S. Pat. Nos. 4,560,655; 4,657,866; 4,767,704; or 4,927,762; or in PCT Pat. Pub. Nos. WO 90/03430 (published April 5, 1990), may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleosides (such as adenosine and thymidine), antibiotics, trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

The host cells referred to in this disclosure encompass cells in culture *in vitro* as well as cells that are within a host animal, for example, as a result of transplantation or implantation.

It is further contemplated that the AL-1 of this invention may be produced by homologous recombination, for example, as described in PCT Pat. Pub. No. WO 91/06667 (published May 16, 1991). Briefly, this method involves transforming cells containing an endogenous gene encoding AL-1 with a homologous DNA, which homologous DNA comprises (1) an amplifiable gene, such as DHFR, and (2) at least one flanking sequence, having a length of at least about 150 base pairs, which is homologous with a nucleotide sequence in the cell genome that is within or in proximity to the gene encoding AL-1. The transformation is carried out under conditions such that the homologous DNA integrates into the cell genome by recombination. Cells having integrated the homologous DNA then are subjected to conditions which select for amplification of the amplifiable gene, whereby the AL-1 gene amplified concomitantly. The resulting cells then are screened for production of desired amounts of AL-1. Flanking sequences that are in proximity to a gene encoding AL-1 are readily identified, for example, by the method of genomic walking, using as a starting point the AL-1 nucleotide sequence set forth in Figure 2. Spoerel, et al., Meth. Enz. 152:598-603 (1987).

Gene amplification and/or gene expression may be measured in a sample directly. for example, by conventional Southern blotting to quantitate DNA, or Northern blotting to quantitate mRNA, using an appropriately labeled oligonucleotide hybridization probe, based on the sequences provided herein. Various labels may be employed, most commonly radioisotopes, particularly ³²P. However, other techniques may also be employed, such as using biotin-modified nucleotides for introduction into a polynucleotide. The biotin then serves as the site for binding to avidin or antibodies, which may be labeled with a wide variety of labels, such as radioisotopes, fluorophores, chromophores, or the like. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of tissue sections and assay of cell culture or body fluids, to quantitate directly the expression of the gene product, AL-1. With immunohistochemical staining techniques, a cell sample is prepared, typically by dehydration and fixation, followed by reaction with labeled antibodies specific for the gene product coupled, where the labels are usually visually detectable, such as enzymatic labels, fluorescent labels, luminescent labels, and the like. A particularly sensitive staining technique suitable for use in the present invention is described by Hsu, et al., *Am. J.. Clin. Path*., 75:734-738 (1980). Antibodies useful for immunohistochemical staining and/or assay of sample fluids may be either monoclonal or polyclonal. Conveniently, the antibodies may be prepared against a synthetic peptide based on the DNA sequences provided herein.

AL-1 preferably is recovered from the culture medium as a secreted polypeptide, although it also may be recovered from host cell lysates. To obtain AL-1 that is substantially free of contaminating proteins or polypeptides of the host cell in which it is produced it is necessary to purify the AL-1, based on the differential physical properties of AL-1 as compared to the contaminants with which it may be associated. For example, as a first step, the culture medium or lysate is centrifuged to remove particulate cell debris. AL-1 thereafter is purified from contaminant soluble proteins and polypeptides, for example, by ammonium sulfate or ethanol precipitation, gel filtration (molecular exclusion chromatography), ion-exchange chromatography, immunoaffinity chromatography, reverse phase HPLC, and/or gel electrophoresis. For example. AL-1 can be purified by immunoaffinity chromatography using a REK7-IgG resin (comprising REK7-IgG coupled to the resin material), as described in Example 5. Secreted, soluble AL-1 resulting from removal of the GPI membrane anchor acts is an example of an antagonist since it binds but does not activate its receptor as demonstrated in the Examples. In contrast, secreted, soluble Al-1-IgG in which AL-1 extracellular domain is fused to an IgG Fc region acts as an AL-1 agonist. Its dimeric form may be responsible for its activity.

Amino acid sequence variants and derivatives of AL-1 are recovered in the same fashion, taking account of any distinguishing features or physical properties of the particular AL-1. For example, in the case of a fusion protein comprising AL-1 and another protein or polypeptide, such as a bacterial or viral antigen, a significant degree of purification may be obtained by using an immunoaffinity column containing antibody to the antigen. In any event, the ordinarily skilled artisan will appreciate that purification methods suitable for naturally occurring AL-1 may require modification to account for changes in the character of AL-1 or its variants or derivatives produced in recombinant host cells.

The purity of AL-1 produced according to the present invention is determined according to methods well known in the art, such as by analytical sodium dodecyl sulfate (SDS) gel electrophoresis, immunoassay, or amino acid composition or sequence analysis electrophoresis. Preferably, the AL-1 is purified to such an extent that it is substantially free of other proteins. For therapeutic uses, the purified AL-1 will be greater than 99% AL-1 and, accordingly, non-AL-1 proteins will comprise less than 1% of the total protein in the purified AL-1 composition.

AL-1 may be used as an immunogen to generate anti-AL-1 antibodies. Such antibodies, which specifically bind to AL-1, are useful as standards in assays for AL-1, such as by labeling purified AL-1 for use as a standard in a radioimmunoassay, enzyme-linked immunoassay, or competitive-type receptor binding assays radioreceptor assay, as well as in affinity purification techniques. Ordinarily, the anti-AL-1 antibody will bind AL-1 with an affinity of at least about 10⁶ L/mole, and preferably at least about 10⁷ L/mole.

Polyclonal antibodies directed toward AL-1 generally are raised in animals by multiple subcutaneous or intraperitoneal injections of AL-1 and an adjuvant. It may be useful to conjugate AL-1 or a peptide fragment thereof to a carrier protein that is immunogenic in the species to be immunized, such as keyhole limpet hemocyanin, serum albumin, bovine thyroglobulin, or soybean trypsin inhibitor, using a bifunctional or derivatizing agent, for example, maleimidobenzoyl sulfosuccinimide ester (conjugation through cysteine residues), N-hydroxysuccinimide (conjugation through lysine residues), glutaraldehyde, succinic anhydride, SOCl₂, or R¹N = C =NR, where R and R¹ are different alkyl groups.

Animals are immunized with such AL-1-carrier protein conjugates combining 1 mg or 1 µg of conjugate (for rabbits or mice, respectively) with 3 volumes of Freund's complete adjuvant and injecting the solution intradermally at multiple sites. One month later the animals are boosted with 1/5th to 1/10th the original amount of conjugate in Freund's complete adjuvant by subcutaneous injection at multiple sites. 7 to 14 days later animals are bled and the serum is assayed for anti-AL-1 antibody titer. Animals are boosted until the antibody titer plateaus. Preferably, the animal is boosted by injection with a conjugate of the same AL-1 with a different carrier protein and/or through a different cross-linking agent. Conjugates of AL-1 and a suitable carrier protein also can be made in recombinant cell culture as fusion proteins. Also, aggregating agents such as alum are used to enhance the immune response.

Monoclonal antibodies directed toward AL-1 are produced using any method which provides for the production of antibody molecules by continuous cell lines in culture. The modifier "monoclonal" indicates the character of the antibody as being obtained from a substantially homogeneous population of antibodies, and is not to be construed as requiring production of the antibody by any particular method. Examples of suitable methods for preparing monoclonal antibodies include the original hybridoma method of Kohler, et al.. *Nature* 256:495-497 (1975), and the human B-cell hybridoma method, Kozbor, *J. Immunol*. 133:3001 (1984); Brodeur, et al., Monoclonal Antibody Production Techniques and Applications, pp.51-63 (Marcel Dekker, Inc., New York, 1987).

The monoclonal antibodies of the invention specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (Cabilly, et al., U.S. Patent No. 4,816,567; Morrison, et al., *Proc. Natl. Acad. Sci.* 81:6851-6855 (1984)).

In a preferred embodiment, the chimeric anti-AL-1 antibody is a "humanized" antibody. Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain.

Humanization can be performed following methods known in the art (Jones, et al., *Nature* 321:522-525 (1986); Riechmann, et al., *Nature,* 332:323-327 (1988); Verhoeyen, et al., *Science* 239:1534-1536 (1988)), by substituting rodent complementarity-determining regions (CDRs) for the corresponding regions of a human antibody. Alternatively, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, for example, Jakobovits, et al., *Proc. Natl. Acad. Sci.* 90: 2551-2555 (1993); Jakobovits, et al., *Nature* 362:255-258 (1993); Bruggermann, et al., Year in Immuno. 7:33 (1993). Human antibodies can also be produced in phage-display libraries (Hoogenboom, et al., *J. Mol. Biol*. 227:381 (1991); Marks, et al., *J. Mol. Biol*. 222:581 (1991).

For diagnostic applications, anti-AL-1 antibodies typically will be labeled with a detectable moiety. The detectable moiety can be any one which is capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin; radioactive isotopic labels, such as, e.g., ¹²⁵I, ³²P, ¹⁴C, or ³H, or an enzyme, such as alkaline phosphatase, beta-galactosidase or horseradish peroxidase.

Any method known in the art for separately conjugating the antibody to the detectable moiety may be employed, including those methods described by David, et al., *Biochemistry* 13:1014-1021 (1974); Pain, et al., *J. Immunol. Meth.* 40:219-231 (1981); and Bayer, et al., Meth. Enz. 184:138-163 (1990).

The anti-AL-1 antibodies may be employed in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard (e.g., AL-1 or an immunologically reactive portion thereof) to compete with the test sample analyte (AL-1) for binding with a limited amount of antibody. The amount of AL-1 in the test sample is inversely proportional to the amount of standard that becomes bound to the antibodies. To facilitate determining the amount of standard that becomes bound, the antibodies generally are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three part complex. David, ei al., U.S. Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

The anti-AL-1 antibodies of the invention also are useful for *in vivo* imaging, wherein an antibody labeled with a detectable moiety is administered to a host, preferably into the bloodstream, and the presence and location of the labeled antibody in the host is assayed. This imaging technique is useful in the staging and treatment of various neurological disorders. The antibody may be labeled with any moiety that is detectable in a host, whether by nuclear magnetic resonance, radiology, or other detection means known in the art.

Neutralizing anti-AL-1 antibodies are useful as antagonists of AL-1. The term "neutralizing anti-AL-1 antibody" as used herein refers to an antibody that is capable of specifically binding to AL-1, and which is capable of substantially inhibiting or eliminating the functional activity of AL-1 *in vivo* or *in vitro*. Typically a neutralizing antibody will inhibit the functional activity of AL-1 at least about 50%, and preferably greater than 80%, as determined, for example, by an *in vitro* receptor binding assay, or *in vitro* axon bundling assay, such as described in Example 8.

Other AL-1 antagonists are prepared using AL-1 receptor proteins, such as REK7. One example of an AL-1 antagonist is the REK7-IgG chimeric protein described herein. Another example of an AL-1 antagonist is a soluble form of an AL-1 receptor, which comprises the extracellular domain or the receptor substantially free of the transmembrane domain. The soluble form of the receptor can be used directly as an antagonist, or the receptor can be used to screen for small molecules that would antagonize AL-1 activity.

AL-1 is believed to be useful in promoting the development, maintenance, or regeneration of neurons *in vivo,* including central (brain and spinal chord), peripheral (sympathetic, parasympathetic, sensory, and enteric neurons), and motomeurons. Accordingly, AL-1 may be utilized in methods for the diagnosis and/or treatment of a variety of neurologic diseases and disorders.

In various embodiments of the invention, purified AL-1 can be administered to patients in whom the nervous system has been damaged by trauma, surgery, stroke, ischemia, infection, metabolic disease, nutritional deficiency, malignancy, or toxic agents, to promote the survival or growth of neurons. For example, AL-1 can be used to promote the survival or growth of motomeurons that are damaged by trauma or surgery. Also, AL-1 can be used to treat motomeuron disorders, such as amyotrophic lateral sclerosis (Lou Gehrig's disease), Bell's palsy, and various conditions involving spinal muscular atrophy, or paralysis. AL-1 can be used to treat human neurodegenerative disorders, such as Alzheimer's disease, Parkinson's disease, epilepsy, multiple sclerosis, Huntington's chorea, Down's Syndrome, nerve deafness, and Meniere's disease. AL-1 can be used as cognitive enhancer, to enhance leaming particularly in dementias or trauma, since they can promote axonal outgrowth and synaptic plasticity, particularly of hippocampal neurons that express REK7 and cortical neurons that express AL-1. Alzheimer's disease, which has been identified by the National Institutes of Aging as accounting for more than 50% of dementia in the elderly, is also the fourth or fifth leading cause of death in Americans over 65 years of age. Four million Americans, 40% of Americans over age 85 (the fastest growing segment of the U.S. population), have Alzheimer's disease. Twenty-five percent of all patients with Parkinson's disease also suffer from Alzheimer's disease-like dementia. And in about 15% of patients with dementia, Alzheimer's disease and multi-infarct dementia coexist. The third most common cause of dementia, after Alzheimer's disease and vascular dementia, is cognitive impairment due to organic brain disease related directly to alcoholism, which occurs in about 10% of alcoholics. However, the most consistent abnormality for Alzheimer's disease, as well as for vascular dementia and cognitive impairment due to organic brain disease related to alcoholism, is the degeneration of the cholinergic system arising from the basal forebrain (BF) to both the codex and hippocampus (Bigl *et al*. in Brain Cholinergic Systems, M. Steriade and D. Biesold, eds., Oxford University Press, Oxford, pp.364-386 (1990)). And there are a number of other neurotransmitter systems affected by Alzheimer's disease (Davies *Med. Res. Rev*.3:221 (1983)). However, cognitive impairment, related for example to degeneration of the cholinergic neurotransmitter system, is not limited to individuals suffering from dementia. It has also been seen in otherwise healthy aged adults and rats. Studies that compare the degree of learning impairment with the degree of reduced cortical cerebral blood flow in aged rats show a good correlation (Berman *et.al. Neurobiol. Aging* 9:691 (1988)). In chronic alcoholism the resultant organic brain disease, like Alzheimer's disease and normal aging, is also characterized by diffuse reductions in cortical cerebral blood flow in those brain regions where cholinergic neurons arise (basal forebrain) and to which they project (cerebral cortex) (Lofti *et al*., *Cerebrovasc. and Brain Metab. Rev* 1:2 (1989)).

Further, AL-1 can be used to treat neuropathy, and especially peripheral neuropathy. "Peripheral neuropathy" refers to a disorder affecting the peripheral nervous system, most often manifested as one or a combination of motor, sensory, sensorimotor, or autonomic neural dysfunction. The wide variety of morphologies exhibited by peripheral neuropathies can each'be attributed uniquely to an equally wide number of causes. For example, peripheral neuropathies can be genetically acquired, can result from a systemic disease, or can be induced by a toxic agent. Examples include but are not limited to distal sensorimotor neuropathy, or autonomic neuropathies such as reduced motility of the gastrointestinal tract or atony of the urinary bladder. Examples of neuropathies associated with systemic disease include post-polio syndrome; examples of hereditary neuropathies include Charcot-Marie-Tooth disease, Refsum's disease, Abetalipoproteinemia, Tangier disease, Krabbe's disease. Metachromatic leukodystrophy, Fabry's disease, and Dejerine-Sottas syndrome; and examples of neuropathies caused by a toxic agent include those caused by treatment with a chemotherapeutic agent such as vincristine, cisplatin, methotrexate, or 3'-azido-3'-deoxythymidine.

In still further embodiments of the invention, AL-1 antagonists, and especially anti-AL-1 antibodies, can be administered to patients suffering from neurologic diseases and disorders characterized by excessive production or activity of AL-1. AL-1 antagonists can be used in the prevention of aberrant regeneration of sensory neurons such as may occur post-operatively, or in the selective ablation of sensory neurons, for example, in the treatment of chronic pain syndromes.

The development of a vascular supply, angiogenesis, is essential for the growth, maturation, and maintenance of normal tissues, including neuronal tissues. It is also required for wound healing and the rapid growth of solid tumors and is involved in a variety of other pathological conditions. Current concepts of angiogenesis, based in large part on studies on the vascularization of tumors, suggest that cells secrete angiogenic factors which induce endothelial cell migration, proliferation, and capillary formation. Numerous factors have been identified which induce vessel formation *in vitro* or *in vivo* in animal models. These include FGFα, FGFβ, TGF-α, TNT-α, VPF or VEGF, monobutyrin, angiotropin, angiogenin, hyaluronic acid degradation products, and more recently, B61 for TNF-α-induced angiogenesis (Pandey et al. (1995) *Science* 268:567-569). Inhibitors of angiogenesis include a cartilage-derived inhibitor identified as TIMP, PF-4, thrombospondin, laminin peptides, heparin/cortisone, minocycline, fumagillin, difluoromethyl omithine, sulfated chitin derivatives, and B61 antibody. The major development of the vascular supply occurs during embryonic development, at ovulation during formation of the corpus luteum, and during wound and fracture healing. Many pathological disease states are characterized by augmented angiogenesis including tumor growth, diabetic retinopathy, neovascular glaucoma, psoriasis, and rheumatoid arthritis. During these processes normally quiescent endothelial cells which line the blood vessels sprout from sites along the vessel, degrade extracellular matrix barriers, proliferate, and migrate to form new vessels. Angiogenic factors, secreted from surrounding tissue, direct the endothelial cells to degrade stromal collagens, undergo directed migration (chemotaxis), proliferate, and reorganize into capillaries.

AL-1 can find further use in promoting or enhancing angiogenesis by receptor activation on endothelial or stromal cells. The induction of vascularization is a critical component of the wound healing process. Neovascularization, also known as angiogenesis, is a complex process involving several sequential steps including basement membrane degradation, endothelial cell mobilization and proliferation, vessel canalization, and new basement membrane formation (Mantovani, *Int. J. Cancer* 25:617 (1980)). Vascularization ensures that proliferating and differentiating fibroblasts are supplied with nutrients and oxygen, and that elements of humoral and cellular immunity are delivered to sites of potential bacterial infection. It is desirable to induce neovascularization as early as possible in the course of wound healing, particularly in the case of patients having conditions that tend to retard wound healing, e.g. burns, decubitus ulcers, diabetes, obesity and malignancies. Even normal post-surgical patients will be benefited if they can be released from hospital care at any earlier date because of accelerated wound healing. This invention provides novel compositions and methods for modulating angiogenesis. A patient bearing a wound can be treated by applying an angiogenically active dose of an AL-1 compound to the wound. This facilitates the neovascularization of surgical incisions, burns, traumatized tissue, skin grafts, ulcers and other wounds or injuries where accelerated healing is desired. In individuals who have substantially impaired wound healing capacity, thereby lack the ability to provide to the wound site endogenous factors necessary for the process of wound healing, the addition of exogenous AL-1 and compositions of the invention enable wound healing to proceed in a normal manner. The proteins of the present invention are expected to accelerate the healing process in a broad spectrum of wound conditions. Novel topical compositions containing an Al-1 compound are provided for use in the inventive method, as are novel articles such as sutures, grafts and dressings containing an AL-1 compound. The term "wound" is defined herein as any opening in the skin, mucosa or epithelial linings, most such openings generally being associated with exposed, raw or abraded tissue. There are no limitations as to the type of wound or other traumata that can be treated in accordance with this invention, such wounds including (but are not limited to): first, second and third degree burns (especially second and third degree); surgical incisions, including those of cosmetic surgery; wounds, including lacerations, incisions, and penetrations; and ulcers, e.g., chronic non-healing dermal ulcers, including decubital ulcers (bedsores) and ulcers or wounds associated with diabetic, dental, hemophilic, malignant and obese patients: Furthermore, normal wound-healing may be retarded by a number of factors, including advanced age, diabetes, cancer, and treatment with anti-inflammatory drugs or anticoagulants, and the proteins described herein may be used to offset the delayed wound-healing effects of such treatments.

Although the primary concern is the healing of major wounds by neovascularization, it is contemplated that an AL-1 compound may also be useful for minor wounds, and for cosmetic regeneration of epithelial cells. Preferably, the wounds to be treated are bums and surgical incisions, whether or not associated with viral infections or tumors. In most cases wounds are not the result of a tumor or a viral infection and ordinarily they do not include tumor cells.

AL-1 is preferably delivered to wounds by topical application, ''topical'' in this context meaning topical to the wound, and does not necessarily refer to epidermal application. When applied topically, the AL-1 compound is usually combined with other ingredients, such as carriers and/or adjuvants. There are no limitations on the nature of such other ingredients, except that they must be pharmaceutically acceptable, efficacious for their intended administration, and cannot degrade or inactivate AL-1. AL-1 is applied to burns in the form of an irrigant or salve, and if so then in an isotonic solution such as physiological saline solution or D5W. AL-1 is particularly useful in accelerating the growth and survival of skin grafts applied to burns. Ordinarily, an AL-1-containing composition is impregnated into the grafts or adherently coated onto the face of the graft, either on the side of the graft to be applied to the burn or on the exterior side of the graft. AL-1 also is included in burn debridement salves which contain proteases so long as the debridement enzyme does not proteolytically inactivate the AL-1.

AL-1 is impregnated into surgical articles in accordance with this invention, such articles being defined as items to be contacted with wounds which articles are typically water adsorbent or hydratable and which have a therapeutic utility in treating wounds. Examples of surgical articles are dressings, sutures, pledgets, skin grafting films (including living skin grafts as well as collagen-containing membranes or synthetic skin substitutes) and the like as will be known to the clinician. Dressings for use herein generally comprise water adsorbent laminates containing AL-1 to be adherently placed into contact with wounds. Improved dressings for use with AL-1 as described herein preferably will have a membrane such as a dialysis membrane interposed between the wound surface and the adsorbent substance in the dressing, the membrane containing pores sufficiently small for AL-1 to diffuse into the wound but not sufficiently large for epithelial cells to penetrate into the adsorbent. The degree of adsorbency will vary considerably and in fact dressings are included herein which are nonadsorbent, i.e., the AL-1 is deposited or stored in an aqueous reservoir which is used to irrigate the wound on a continuous or intermittent basis.

AL-1 also is formulated into ointments or suspensions, preferably in combination with purified collagen, in order to produce semisolid or suspension vehicles. Conventional oleaginous formulations containing AL-1 are useful as salves. Such AL-1 carriers and formulations release AL-1 on a sustained basis at the wound, thereby serving to create a chemotactic gradient that directionally orients neovascularization, e.g. into a skin graft. Sustained release formulations for AL-1 include semipermeable polymer matrices in the form of shaped articles, e.g. films, or microcapsules. Implantable sustained release matrices include copolymers of L-glutamic acid and gamma ethyl-L-glutamate (Sidman et al., *Biopolymers* 22(1):547-556 (1983)), poly (2-hydroxyethyl-methacrylate) (Langer et al., *J. Biomed. Mater*. *Res.* 15:167-277 (1981) and Langer *Chem. Tech*. 12:98-105 (1982)), ethylene vinyl acetate (Langer et al., Id.), or poly-D-(-)-3-Hydroxybutyric acid (EP 133,988A). These formulations may function as bioerodible matrices or as stable sources for the passive diffusion of AL-1.

Sustained release AL-1 compositions for contact with wounds also include liposomally entrapped AL-1. Liposomes containing AL-1 are prepared by methods known per se: DE 3,218,121A; Epstein et al., *Proc. Natl. Acad. Sci. USA* 82:3688-3692 (1985); Hwang et al. *Proc. Natl. Acad. Sci. USA* 77:4030-4034 (1980); EP 52322A; EP 36676A; EP 88046A; EP 143949A; EP 142641A; Japanese patent application 83-118008; U.S. patents 4,485,045 and 4,544,545; and EP 102,324A. Ordinarily the liposomes are of the small (about 200-800 Angstroms) unilamelar type in which the lipid content is greater than about 30 mol. % cholesterol, the selected proportion being adjusted for the optimal rate of AL-1 leakage.

AL-1 is formulated with other ingredients such as carriers and/or adjuvants, e.g. albumin, nonionic surfactants and other emulsifiers. There are no limitations on the nature of such other ingredients, except that they must be pharmaceutically acceptable, efficacious for their intended administration, and cannot degrade the activity of the active ingredients of the compositions. Suitable adjuvants include collagen or hyaluronic acid preparations, fibronectin, factor XIII, or other proteins or substances designed to stabilize or otherwise enhance the active therapeutic ingredient(s).

AL-1 optionally is supplied with other known angiogenic agents such as heparin, which has been shown to accelerate the healing of thermal burns, TNF, TGF-α, TGF-β, fibroblast growth factor, epidermal growth factor, B61, angiogenin, platelet factor 4, insulin, PDGF, and angiogenesis factor and the angiogenic activity of the combinations observed for synergistic effects. AL-1 optionally also is combined with an with an IFN, e.g. IFN-γ, and other cytokines, or may be free of interferons such as IFN-γ. Where such cytokines or known angiogenic agents are species-specific, the appropriate cytokine or agent will be selected for the species to be treated.

Animals or humans are treated in accordance with this invention. It is possible but not preferred to treat an animal of one species with AL-1 of another species. A preferred AL-1 for use herein is soluble AL-1-IgG.

The amount of AL-1 to be contacted with the wound depends upon a great number of variables that will be taken into account by the clinician, including the presence of other angiogenic agents in the AL-1 formulations, the nature of the wound to be treated, the condition of the patient, the AL-1 formulation selected, the neovascularizing activity of the molecular species of AL-1 chosen and the route of administration. Lesser amounts of AL-1 typically are administered when the AL-1 is formulated into a sustained release vehicle, e.g. dressing or ointment, and when the AL-1 is administered by direct topical contact rather than impregnated into a bandage or dressing. Concentrations in the range of 0.10 ng/ml - 100 ug/ml may be used. The typical topical formulation will be capable of delivering a concentration of AL-1-IgG, or equivalent, at the neovascularization target site (for example, a skin graft) in a range of about from 0.10 ng/ml to 10000 ng/ml, more preferably 0.20 ng/ml to 1000 ng/ml, and even more preferably 0.25 ng/ml to 350 ng/ml, although this therapeutic dose range is subject to considerable variation as noted above. Delivery of concentrations outside of this range may offer certain of the benefits of AL-1 neovascularization, but the clinician will be expected to monitor dosages in order to optimize performance of AL-1 in wound healing. It also should be noted that the weight amount will vary for other AL-1 variants and forms if their molecular weight and/or angiogenic potency differ from that of AL-1-IgG. Potency differences are easily determined by comparing the degree of neovascularization achieved with the candidate AL-1 and AL-1-IgG in any of the assays set forth in the Examples herein.

Accordingly, a method for accelerating the neovascularization of a wound is provided that includes the step of applying to the wound an angiogenically effective dose of a composition comprising tumor necrosis factor. The composition can be applied topically by direct contact with the wound, particularly when the wound is a fresh surgical incision. In a preferred embodiment the composition further comprises collagen or a synthetic skin substitute. The caregiver may further administer to the wound a growth factor, an antibiotic, a debridement agent, and/or angiogenin. A preferred composition for the debridement of bums contains an AL-1 compound and a proteolytic enzyme which does not inactivate the neovascularizing activity of AL-1. The therapeutic compositions can be reapplied at one-to-several-day-intervals until healing is complete.

Therapeutic options for patients with vascular disease, particularly vascular obstructive disease, are sometimes limited. Such patients are often refractory to conservative measures and typically unresponsive to drug therapy (Takeshita et al., *J. Clin. Invest*. 93:662-670 (1994)). When vascular obstruction is lengthy and/or widespread, nonsurgical revascularization may not be feasible. Surgical therapy, consisting of arterial bypass and/or amputation, may be complicated by a variable morbidity and mortality, and is often dependent for its efficacy upon short- and long-term patency of the conduit used. Therapeutic angiogenesis constitutes an alternative treatment strategy for such patients. The present invention provides methods for enhancing angiogenesis in a mammal comprising administering to the mammal an effective amount of AL-1. The AL-1 alone may be administered to the mammal, or alternatively, may be administered to the mammal in combination with other therapies and/or pharmacologic agents. In particular AL-1 finds use in patients suffering from vascular insufficiency or limb ischemia secondary to arterial occlusive disease. The effects of AL-1 proteins of the invention on angiogenesis can be tested, for example, in a rabbit model of hindlimb ischemia. This rabbit model was designed to simulate ischemia characteristics of patients with severe lower extremity arterial occlusive disease. (Takeshita et al., *supra*) and is performed essentially as described in Takeshita et al., *supra*. Measurements of calf blood pressure (BP) index; angiographic score of collateral formation; intravascular Doppler-wire analysis of blood flow; and microsphere-based analysis of muscle perfusion at rest and during stress are performed.

AL-1 antagonists can find use in inhibiting, preventing or treating pathological angiogenesis, such as during tumor vascularization. Tumor neovascularization is a vital stage in the growth of solid tumors (Polverini, P.J. et al. *Lab. Invest*. 51:635-642 (1985)). The progressive growth of solid tumors is strictly dependent on their ability to attract new blood vessels that will supply them with oxygen and essential nutrients (Bouck, *Cancer Cells* 2(6):179-185 (1990)). Angiogenesis has been shown to precede or accompany malignancy. In the absence of neovascularization the size of tumor grafts becomes limited. When angiogenesis is absent, tumors tend to remain dormant. Therefore, angiogenic activity has been directly correlated with tumor growth. AL-1 antagonist compositions and methods of the invention can modulate (e.g., prevent or reduce) new capillary growth into tumors.

A variety of non-neoplastic diseases, previously thought to be unrelated, can be considered "angiogenic diseases" because they are dominated by the pathological growth of capillary blood vessels. These diseases include diabetic retinopathy, arthritis, hemangiomas, psoriasis, and ocular neovascularization. AL-1 antagonist compositions and methods of the invention can be used to treat these conditions.

Vascularization also plays a critical role in chronic inflammatory conditions such as rheumatoid arthritis (Koch, A.E. et al. *Arthr. Rheum*. 29:471-479 (1986)). Rheumatoid arthritis ("RA") is a chronic heterogeneous disorder in which a variety of etiological agents may be responsible for initiating a series of events leading to inflammation in multiple joints. The cause of the disease remains unknown, although by analogy with other forms of arthritis such as that accompanying Lyme disease, it has been postulated that infection with as yet unidentified bacteria or viruses in a genetically susceptible host is an initiating event. Persistence could result from the presence of viral or bacterial antigens that generate an immune response or cross-react with host tissues together with amplification effects of cellular products of the host. While many patients have systemic manifestations in RA, many of the most serious consequences of RA stem from its effects on articular connective tissues, which are characterized by alterations of the synovial membrane with proliferation of lining cells and infiltration by chronic inflammatory cells. Erosions of bone occur in areas contiguous with the inflammatory cell mass as well as in regions adjacent to bone marrow distant from the inflammation. The bone erosions are probably produced through induction of differentiation and activation of osteoclast progenitors. The erosion of soft connective tissues, e.g., cartilage, joint capsules, tendons, and ligaments, results from direct release of proteolytic enzymes from cells of the inflammatory cell mass or from polymorphonuclear leukocytes that are typically abundant in rheumatoid synovial fluids, although rare in the synovial membrane. See, for example, Harris, in W. N. Kelley et al., eds., Textbook of Rheumatology, W.B. Saunders, Philadelphia, pp. 886-915 (1985); Dayer and Krane, Clin. Rheum. Dis., 4: 517-537 (1978); Krane, in Arthritis and Allied Conditions. A textbook of Rheumatology, ed. by D.J. McCarty, pp. 593-604, Lea and Febiger, Philadelphia (1985); and Krane et al., *Lymphokines* 7:75-136 (1982). Therapy for RA depends on the stage of the disease. Stage 1, where a postulated antigen is presented to T-cells with no obvious arthritic symptoms, is not treated. Stage 2 involves T-cell and B-cell proliferation and angiogenesis in synovial membrane, resulting in malaise, mild joint stiffness, and swelling. During Stage 3, neutrophils accumulate in synovial fluid and synovial cells proliferate without polarization or invasion of cartilage, resulting in joint pain and swelling, morning stiffness, malaise, and weakness. Current therapy for Stages 2 and 3 includes bed rest, application of heat, supplemental icosapentaenoic and docosahexanoic acid, and drugs. Nonsteroidal anti-inflammatory drugs, including aspirin, continue to be the foundation of drug therapy in treating Stages 2 and 3 of the disease. Those anti-inflammatory drugs other than aspirin include indomethacin, phenylbutazone, phenylacetic acid derivatives such as ibuprofen and fenoprofen, naphthalene acetic acids (naproxen), pyrrolealkanoic acid (tometin), indoleacetic acids (sulindac), halogenated anthranilic acid (meclofenamate sodium), piroxicam, zomepirac, and diflunisal. Second-line drugs for RA Stages 2 and 3 include anti-malarial drugs such as hydroxychloroquine, sulfasalazine, gold salts, and penicillamine, and low-dose methotrexate. These alternatives frequently produce severe side effects, including retinal lesions and kidney and bone marrow toxicity. The irreversible destruction of cartilage occurs in Stage 4 of the disease. Currently available drugs and treatments include total lymphoid irradiation, high-dose intravenous methylprednisolone, and cyclosporine. Cyclosporine is nephrotoxic and the other treatments exert substantial toxicity as well. As a result, such immunosuppressive agents heretofore have been used only in the treatment of severe and unremitting RA. Other possible therapeutic drugs for Stage 4 of RA include cyclic oligosaccharides (cyclodextrins), which, when combined with a noninflammatory steroid (cortexolone), inhibit angiogenesis *in vivo.* Folkman et al., *Science* 243:1490-1493 (1989). Antibodies against crucial components of the early phase of the immune response include anti-Class II MHC antibodies (Gaston et al., *Arthritis Rheum.* 31:21-30 (1988): Sany et al., *Arthritis Rheum*. 25:17-24 (1982)), anti-interleukin-2 receptor antibodies (Kyle et al., *Ann. Rheum. Dis*., 48:428-429 (1989)), anti-CD4 antibodies (Herzog et al., *J. Autoimmun*., 2:627-642 (1989); Walker et al., *J. Autoimmun*., 2:643-649 (1989)), and antithymocyte globulin (Shmerling and Trentham, *Arthritis Rheum*., 32:1495-1496 (1989)). The last three of these drugs have been used in patients with RA. The present invention provides compositions and methods that down-regulate inflammatory and proliferative pathways in RA by modulating the associated angiogenesis. The compositions contain an angiogenesis-modulating effective amount of an AL-1 antagonist. The compositions can further comprise other angiogenesis-modulating agents, particularly agents for treating RA as discussed above or agents for treating tumors. The methods involve the step of administering an angiogenesis-modulating effective amount of an AL-1 antagonist to a mammal in need of such treatment. By "modulating" in the context of conditions in which angiogenesis is undesirable is meant blocking, inhibiting, preventing, reversing, or reducing angiogenesis, or preventing further progression of angiogenesis.

There seems to be little or no biochemical difference between angiogenic peptides expressed by tumors and those expressed by normal tissues. Nor are there any morphological differences between the new capillaries that respond to a malignancy and the capillary growth that occurs during physiological neovascularization (Folkman et al. *Science* 235:442-447 (1987)). As there is no qualitative difference between the angiogenic capabilities of nonmalignant and malignant diseases, results from normal and malignant vascularization assays can be easily compared, and progress can be made in either area independently of the system of investigation used (Paweletz et al., *Critical Reviews in Oncology*/*Hematology* 9(3):197-198 (1989)). Thus, the invention relates to a method comprising inhibiting angiogenesis by administering an effective amount of AL-1 antagonist. The invention comprises a method for the treatment of angiogenesis-dependent diseases by administering an effective amount of AL-1 antagonist to a mammal. Angiogenesis dependent diseases include, but are not limited to diabetic retinopathy, arthritis, tumor growth and metastasis, neovascular glaucoma, retinopathy of prematurity, senile macular degeneration, and hypergeneration of scars after wound healing. The pharmaceutical composition of the present invention exhibit therapeutic properties and preventative or inhibitive properties against diseases associated with angiogenesis, for example, inflammatory diseases (e.g., rheumatoid arthritis), diabetic retinopathy, tumors such as malignant tumors (e.g., cancer such as mastocarcinoma, hepatoma, colic carcinoma, Kaposi's sarcoma, lung carcinomas and other epithelial carcinomas).

Numerous methods, *in vitro* and *in vivo*, are available to screen candidate AL-1 or AL-1 antagonists compounds for angiogenic or angiogenesis-inhibiting properties. Several *in vitro* assays of endothelial cell growth, migration, and capillary tube formation are known and can be used with the compounds of the invention, particularly as initial screening methods for angiogenic or angiostatic substances. Further testing would typically use *in vivo* animal testing. U.S. Patent 5,382,514, which is incorporated herein, describes numerous models for angiogenesis *in vivo*. For example, the corneal pocket assay involves the surgical implantation of polymer pellets containing angiogenic factors in the cornea of larger animals such as rabbits. Since quantitation can be difficult the assay is usually used for preferred candidate compounds. The rabbit ear chamber assay requires the surgical insertion of a glass or plastic viewing device and measurement of capillary migration by microscopy. The rat dorsal air sac assay involves implants of stainless steel chambers containing angiogenic factors. An alginate assay which generates an angiogenic response has been described which involves the injection of tumor cells encased in alginate subcutaneously into mice. The accumulation of hemoglobin in the injected gel is used to quantitate the angiogenic response. A compound can be administered to the chorio-allantoic membranes of aged, typically three-day-aged, fertilized chicken eggs and the appearance of neovascularization after a lapse of time, typically two days is observed (CAM assay; Ausprunk et al. *Am. J. Pathol*. 97:597 (1975)). The neovascularization inhibitory rates are compared with an untreated control group. A more recent assay method involves providing a liquid matrix material which forms a matrix gel when injected into a host; adding an angiogenic agent to the liquid matrix material; injecting the liquid matrix material containing the angiogenic agent into a host to form a matrix gel; recovering the matrix gel from the host: and quantitating angiogenesis of the recovered matrix gel. A variation of this can be used to test for inhibitors of vascularization in a tissue by providing a liquid matrix material which forms a matrix gel when injected into a host; adding an angiogenic inhibiting agent to the liquid matrix material; and injecting the liquid matrix material containing the angiogenic inhibiting agent into a tissue situs of a host to form a matrix gel. This system can be used with compounds of the invention when inducing vascularization in a tissue is desired by providing a liquid matrix material which forms a matrix gel when injected into a host; adding an angiogenic inducing agent to the liquid matrix material; and injecting the liquid matrix material containing the angiogenic inducing agent into a tissue situs of a host to form a matrix gel. In a preferred embodiment, a solution of basement membrane proteins supplemented with fibroblast growth factor and heparin is injected subcutaneously in a host, e.g., a mouse, where it forms a gel. Sprouts from vessels in the adjacent tissue penetrate into the gel within days connecting it with the external vasculature. Angiogenesis is then quantitated by image analysis of vessels and by measuring the hemoglobin present in the vessels within the gel. This assay method facilitates the testing of both angiogenic and angiostatic agents *in vivo*. In addition, the endothelial cells responding to the angiogenic factors can be recovered *in vitro* for further studies. Preferred compounds have 50-70% inhibition rates, and more preferred compounds show 80-100% inhibition rates. As described herein the angiogenically active proteins of the invention provide use in *in vitro* and *in vivo* screens for compounds that inhibit angiogenesis by measuring inhibition of AL-1 stimulated angiogenesis in the presence and absence of the candidate inhibitor.

Therapeutic formulations of AL-1 and AL-1 antagonists for treating neurologic diseases and disorders and for modulating angiogenesis are prepared by mixing AL-1 or AL-1 antagonist, e.g., anti-AL-1 antibody or soluble REK7, having the desired degree ofpurity, with optional physiologically acceptable carriers, excipients, or stabilizers which are well known. Acceptable carriers, excipients or stabilizers are nontoxic at the dosages and concentrations employed, and include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as Tween, Pluronics or polyethylene glycol (PEG).

It may be desirable to adsorb AL-1 onto a membrane, such as a silastic membrane, which can be implanted in proximity to damaged neural tissue, or to incorporate AL-1 into liposomes. PCT Pat. Pub. No. WO 91/04014 (published April 4, 1991). In another embodiment, the AL-1 used for therapeutic effect is AL-1 covalently joined to another protein, such as an immunoglobulin domain (for example, to produce an AL1-IgG fusion protein). Immunoglobulin fusions, immunoadhesins, are chimeric antibody-like molecules that combine the functional domain(s) of a binding protein (in this case AL-1 or its receptor) with the an immunoglobulin sequence. The immunoglobulin sequence preferably (but not necessarily) is an immunoglobulin constant domain. Immunoglobulins (Ig) and certain variants thereof are known and many have been prepared in recombinant cell culture. For example, see U.S. Patent 4,745,055; EP 256,654; Faulkner et al., *Nature* 298:286 (1982); EP 120,694; EP 125,023; Morrison, *J. Immun*. 123:793 (1979); Köhler et al., *Proc. Nat'l. Acad. Sci*. *USA* 77:2197 (1980); Raso et al., *Cancer Res.* 41:2073 (1981); Morrison et al., *Ann. Rev. Immunol*. 2:239 (1984); Morrison, *Science 229:1202* (1985); Morrison et al., *Proc. Nat'l. Acad. Sci. USA* 81:6851 (1984); EP 255,694; EP 266,663: and WO 88/03559. Reasserted immunoglobulin chains also are known. See for example U.S. patent 4,444,878; WO 88/03565; and EP 68,763 and references cited therein. The immunoglobulin moiety in the chimeras of the present invention may be obtained from IgG-1, IgG-2, IgG-3 or IgG-4 subtypes, IgA, IgE, IgD or IgM, but preferably IgG-1 or IgG-3.

Chimeras constructed from a receptor sequence linked to an appropriate immunoglobulin constant domain sequence (immunoadhesins) are known in the art. Immunoadhesins reported in the literature include fusions of the T cell receptor* (Gascoigne et al., *Proc. Natl.Acad. Sci. USA* 84:2936-2940 (1987)); CD4* (Capon et al., *Nature* 337:525-531 (1989); Traunecker et al., *Nature* 339:68-70 (1989); Zettmeissl *et al., DNA Cell Biol USA* 9:347-353 (1990); Byrn et al., *Nature* 344667-670 (1990)); L-selectin (homing receptor) (Watson et al. *J. Cell. Biol.* 110:2221-2229 (1990); Watson et al., *Nature* 349:164-167 (1991)); CD44* (Aruffo et al., *Cell* 61:1303-1313 (1990)); CD28* and B7* (Linsley et al., *J. Exp. Med*. 173721-730 (1991)); CTLA-4* (Lisley et al., *J. Exp. Med.* 174:61-569 (1991)); CD22* (Stamenkovic et al., *Cell* 66:1133-1144 (1991)); where the asterisk (*) indicates that the receptor is member of the immunoglobulin superfamily.

The simplest and most straightforward immunoadhesin design combined the binding region(s) of the 'adhesin' protein (eg AL-1 or REK-7) with the hinge and Fc regions of an immunoglobulin heavy chain. Ordinarily, when preparing chimeras of the present invention, nucleic acid encoding the extracellular domain or a fragment thereof of AL-1 or REK-7 will be fused C-terminally to nucleic acid encoding the N-terminus of an immunoglobulin constant domain sequence, however N-terminal fusions are also possible. Typically, in such fusions the encoded chimeric polypeptide will retain at least functionally active hinge, CH2 and CH3 domains of the constant region of an immunoglobulin heavy chain. Fusions are also made to the C-terminus of the Fc portion of a constant domain, or immediately N-terminal to the CH1 of the heavy chain or the corresponding region of the light chain. The precise site at which the fusion is made is not critical; particular sites are well known and may be selected in order to optimize the biological activity, secretion or binding characteristics of the AL-1 or REK-7 receptor-immunoglobulin chimeras.

In some embodiments, chimeras are assembled as monomers, or hetero- or homo-multimers. and particularly as dimers or tetramers, essentially as illustrated in WO 91/08298. In a preferred embodiment, the AL-1 or REK-7 extracellular domain sequence is fused to the N-terminus of the C-terminal portion of an antibody (in particular the Fc domain), containing the effector functions of an immunoglobulin, e.g. immunoglobulin G₁ (IgG-1). It is possible to fuse the entire heavy chain constant region to the AL-1 or REK-7 extracellular domain sequence. Preferably a sequence beginning in the hinge region just upstream of the papain cleavage site (which defines IgG Fc chemically; residue 216, taking the first residue of heavy chain constant region to be 114, or analogous sites of other immunoglobulins) is used in the fusion. In some embodiments an AL-1 or REK-7 amino acid sequence is fused to the hinge region and CH2 and CH3 or CH1, hinge, CH2 and CH3 domains of an IgG-1, IgG-2, or IgG-3 heavy chain. The precise site at which the fusion is made is not critical, and the optimal site can be determined by routine experimentation. The immunoglobulin portion can genetically engineered or chemically modified to inactivate a biological activity of the immunoglobulin portion, such as T-cell binding, while retaining desirable properties such as its scaffolding property for presenting AL-1 or REK7 function to an axon or target cell. Chimeras can be assembled as multimers, particularly as homo-dimers or -tetramers. Generally, these assembled immunoglobulins will have known unit structures. A basic four chain structural unit is the form in which IgG, IgD, and IgE exist. A four unit is repeated in the higher molecular weight immunoglobulins; IgM generally exists as a pentamer of basic four units held together by disulfide bonds. IgA globulin, and occasionally IgG globulin, may also exist in multimeric form in serum. In the case of multimer, each four unit may be the same or different. Alternatively. the AL-1 or REK-7 extracellular domain sequences can be inserted between immunoglobulin heavy chain and light chain sequences such that an immunoglobulin comprising a chimeric heavy chain is obtained. In this embodiment, the sequences are fused to the 3' end of an immunoglobulin heavy chain in each arm of an immunoglobulin. either between the hinge and the CH2 domain, or between the CH2 and CH3 domains. See Hoogenboom, H. R. et al., *Mol. Immunol*. 28:1027-1037 (1991). The presence of an immunoglobulin light chain is not required in the immunoadhesins of the present invention; an immunoglobulin light chain might be present either covalently associated to a immunoglobulin heavy chain fusion polypeptide, or directly fused to the AL-1 or REK-7 extracellular domain. In the former case, DNA encoding an immunoglobulin light chain is typically coexpressed with the DNA encoding the AL-1- or REK-7-immunoglobulin heavy chain fusion protein. Upon secretion, the hybrid heavy chain and the light chain will be covalently associated to provide an immunoglobulin-like structure comprising two disulfide-linked immunoglobulin heavy chain-light chain pairs. Preparation of such structures are, for example, disclosed in U.S. Patent No. 4,816,567 issued 28 March 1989. The immunoglobulin sequences used in the construction of the immunoadhesins of the present invention can be from an IgG immunoglobulin heavy chain constant domain. For human immunoadhesins, the use of human IgG1 and IgG3 immunoglobulin sequences is preferred. A major advantage of using IgG1 is that IgG1 immunoadhesins can be purified efficiently on immobilized protein A. In contrast, purification of IgG3 requires protein G. a significantly less versatile medium. However, other structural and functional properties of immunoglobulins should be considered when choosing the Ig fusion partner for a particular immunoadhesin construction. For example, the IgG3 hinge is longer and more flexible, so it can accommodate larger 'adhesin' domains that may not fold or function properly when fused to IgG1. Another consideration may be valency; IgG immunoadhesins are bivalent homodimers, whereas Ig subtypes like IgA and IgM may give rise to dimeric or pentameric structures, respectively, of the basic Ig homodimer unit. For AL-1-Ig or REK-7-Ig immunoadhesins designed for *in vivo* application, the phannacokinetic properties and the effector functions specified by the Fc region are important as well. Although IgG1, IgG2 and IgG4 all have *in vivo* half-lives of 21 days, their relative potencies at activating the complement system are different. IgG4 does not activate complement, and IgG2 is significantly weaker at complement activation than IgG1. Moreover, unlike IgG1, IgG2 does not bind to Fc receptors on mononuclear cells or neutrophils. While IgG3 is optimal for complement activation, its *in vivo* half-life i approximately one third of the other IgG isotypes. Another important consideration for immunoadhesins designed to be used as human therapeutics is the number of allotypic variants of the particular isotype. In general, IgG isotypes with fewer serologically-defined allotypes are preferred. For example, IgG1 has only four serologically-defined allotypic sites, two of which (G1m and 2) are located in the Fc region; and one of these sites G1m1, is non-immunogenic. In contrast, there are 12 serologically-defined allotypes in IgG3, all of which are in the Fc region: only three of these sites (G3m5, 11 and 21) have one allotype which is nonimmunogenic. Thus, the potential irrimunogenicity of a γ3 immunoadhesin is greater than that of a γ1 immunoadhesin.

In designing the chimeras of the present invention domains that are not required for neurotrophin binding and/or biological activity may be deleted. In such structures, it is important to place the fusion junction at residues that are located between domains, to avoid misfolding. With respect to the parental immunoglobulin, a useful joining point is just upstream of the cysteines of the hinge that form the disulfide bonds between the two heavy chains. In a frequently used design, the codon for the C-terminal residue of the 'adhesin' part of the molecule is placed directly upstream of the codons for the sequence DKTHTCPPCP of the IgG1 hinge region.

The general methods suitable for the construction and expression of immunoadhesins are the same those disclosed hereinabove with regard to (native or variant) AL-1 or REK7. For example, AL-1-1g immunoadhesins are most conveniently constructed by fusing the cDNA sequence encoding the AL-1 portion in-frame to an 1g cDNA sequence. However, fusion to genomic Ig fragments can also be used (see, e.g. Gascoigne et al., *Proc. Natl. Acad. Sci. USA* 84:2936-2940 (1987); Aruffo et al., *Cell* 61:1303-1313 (1990); Stamenkovic et al., *Cell* 66:1133-1144 (1991)). The latter type of fusion requires the presence of Ig regulatory sequences for expression. cDNAs encoding IgG heavy-chain constant regions can be isolated based on published sequence from cDNA libraries derived from spleen or peripheral blood lymphocytes, by hybridization or by polymerase chain reaction (PCR) techniques. The cDNAs encoding the 'adhesin' and the Ig parts of the immunoadhesin are inserted in tandem into a plasmid vector that directs efficient expression in the chosen host cells. For expression in mammalian cells pRK5-based vectors (Schall et al., *Cell* 61:361-370 (1990)] and CDM8-based vectors (Seed, *Nature* 329:840 (1989)). The exact junction can be created by removing the extra sequences between the designed junction codons using oligonucleotide-directed deletional mutagenesis (Zoller and Smith, *Nucleic Acids Res.* 10:6487 (1982); Capon et al., *Nature* 337:525-531 (1989)). Synthetic oligonucleotides can be used, in which each half is complementary to the sequence on either side of the desired junction; ideally, these are 36 to 48-mers. Alternatively, PCR techniques can be used to join the two parts of the molecule in-frame with an appropriate vector.

The choice of host cell line for the expression of AL-1-1g immunoadhesins depends mainly on the expression vector. Another consideration is the amount of protein that is required. Milligram quantities often can be produced by transient transfections. For example, the adenovirus EIA-transformed 293 human embryonic kidney cell line can be transfected transiently with pRK5-based vectors by a modification of the calcium phosphate method to allow efficient immunoadhesin expression. CDM8-based vectors can be used to transfect COS cells by the DEAE-dextran method (Aruffo *et al*., *Cell* 61:1303-1313 (1990); Zettmeissl *et al*., *DNA Cell Biol*. (US) 9:347-353 (1990)). If larger amounts of protein are desired, the immunoadhesin can be expressed after stable transfection of a host cell line. For example, a pRK5-based vector can be introduced into Chinese hamster ovary (CHO) cells in the presence of an additional plasmid encoding dihydrofolate reductase (DHFR) and conferring resistance to G418. Clones resistant to G418 can be selected in culture; these clones are grown in the presence of increasing levels of DHFR inhibitor methotrexate; clones are selected, in which the number of gene copies encoding the DHFR and immunoadhesin sequences is co-amplified. If the immunoadhesin contains a hydrophobic leader sequence at its N-terminus, it is likely to be processed and secreted by the transfected cells. The expression of immunoadhesins with more complex structures may require uniquely suited host cells; for example, components such as light chain or J chain may be provided by certain myeloma or hybridoma cell hosts (Gascoigne et al., 1987, *supra*; Martin et al., *J. Virol.* 67, 3561-3568 (1993)).

Immunoadhesins can be conveniently purified by affinity chromatography. The suitability of protein A as an affinity ligand depends on the species and isotype of the immunoglobulin Fc domain that is used in the chimera. Protein A can be used to purify immunoadhesins that are based on human γ1, γ2, or γ4 heavy chains (Lindmark et al., *J. Immunol. Meth*. 62:1-13 (1983)). Protein G is recommended for all mouse isotypes and for human γ3 (Guss et al., *EMBO J*. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. The conditions for binding an immunoadhesin to the protein A or G affinity column are dictated entirely by the characteristics of the Fc domain; that is, its species and isotype. Generally, when the proper ligand is chosen, efficient binding occurs directly from unconditioned culture fluid. One distinguishing feature of immunoadhesins is that, for human γ1 molecules, the binding capacity for protein A is somewhat diminished relative to an antibody of the same Fc type. Bound immunoadhesin can be efficiently eluted either at acidic pH (at or above 3.0), or in a neutral pH buffer containing a mildly chaotropic salt. This affinity chromatography step can result in an immunoadhesin preparation that is >95% pure.

Other methods known in the art can be used in place of, or in addition to, affinity chromatography on protein A or G to purify immunoadhesins. Immunoadhesins behave similarly to antibodies in thiophilic gel chromatography (Hutchens and Porath, *Anal. Biochem*. 159:217-226 (1986)) and immobilized metal chelate chromatography (Al-Mashikhi and Makai, *J. Dairy Sci.* 71:1756-1763 (1988)). In contrast to antibodies, however, their behavior on ion exchange columns is dictated not only by their isoelectric points, but also by a charge dipole that may exist in the molecules due to their chimeric nature.

If desired, the immunoadhesins can be made bispecific, that is, directed against two distinct ligands. Thus, the immunoadhesins of the present invention may have binding specificities for AL-1 and REK-7. or may specifically bind to a AL-1 (or conversely REK7) and to an other determinant specifically expressed on the cells expressing the AL-1 (or the REK7) to which the REK7 (or AL-1) portion of the immunoadhesin structure binds. For bispecific molecules, trimeric molecules, composed of a chimeric antibody heavy chain in one arm and a chimeric antibody heavy chain-light chain pair in the other arm of their antibody-like structure are advantageous, due to ease of purification. In contrast to antibody-producing quadromas traditionally used for the production of bispecific immunoadhesins, which produce a mixture of ten tetramers, cells transfected with nucleic acid encoding the three chains of a trimeric immunoadhesin structure produce a mixture of only three molecules, and purification of the desired product from this mixture is correspondingly easier.

It is apparent that AL-1 antagonists can be prepared or used applying the above guidelines appropriately. For example, a REK7-IgG or anti-AL-1 antibody can be adsorbed onto a membrane, such as a silastic membrane, which can be implanted in proximity to tumors or RA tissue, or can be incorporated into liposomes. PCT Pat. Pub. No. WO 91/04014 (published April 4, 1991).

AL-1 optionally is combined with or administered in concert with other neurotrophic factors to achieve a desired therapeutic effect. For example, AL-1 may be used together with NGF, NT-3, BDNF, NT-4/5, an insulin-like growth factor (eg., IGF-1, IGF-2, or IGF-3) or another neurotrophic factor to achieve a synergistic stimulatory effect on the growth of sensory neurons, wherein the term "synergistic" means that the effect of the combination of AL-1 with a second substance is greater than that achieved with either substance used individually.

AL-1 and AL-1 antagonists to be used for in vivo administration must be sterile. This is readily accomplished by filtration of a salution of AL-1 or anti-AL-1 antibody through sterile filtration membranes. Thereafter, the filtered solution may be placed into a container having a sterile access port, for example, an intravenous solution bag or vial having a stopper pierceable by a hypodermic injection needle. The filtered solution also may be lyophilized to produce sterile AL-1 or anti-AL-1 antibody in a powder form.

Methods for administering AL-1 and AL-1 antagonists *in vivo* include injection or infusion by intravenous, intraperitoneal, intracerebral, intrathecal, intramuscular, intraocular, intraarterial, or intralesional routes, and by means of sustained-release formulations.

Sustained-release formulations generally consist of AL-1 or AL-1 antagonists and a matrix from which the AL-1 or AL-1 antagonists are released over some period of time. Suitable matrices include semipermeable polymer matrices in the form of shaped articles, for example, membranes, fibers, or microcapsules. Sustained release matrices may comprise polyesters, hydrogels, polylactides, U.S. Pat. No. 3,773,919, copolymers of L-glutamic acid and gamma ethyl-L-glutamate, Sidman, et al., *Biopolymers,* 22: 547-556 (1983), poly (2-hydroxyethyl-methacrylate), or ethylene vinyl acetate, Langer, et al., *J. Biomed. Mater. Res.* 15: 167-277 (1981); Langer, *Chem. Tech*. 12:98-105 (1982).

In one embodiment of the invention, the therapeutic formulation comprises AL-1 or AL-1 antagonist entrapped within or complexed with liposomes. For example, AL-1 covalently joined to a glycophosphatidyl-inositol moiety may be used to form a liposome comprising AL-1. In a further embodiment, the therapeutic formulation comprises cells actively producing AL-1 or AL-1 antagonist. Such cells may be directly introduced into the tissue of a patient, or may be encapsulated within porous membranes which are then implanted in a patient, in either case providing for the delivery of AL-1 or anti-AL-1 antagonist into areas within the body of the patient in need of increased or decreased concentrations of AL-1. Alternatively, an expression vector comprising AL-1 DNA may be used for in vivo transformation of a patient's cells to accomplish the same result.

An effective amount of AL-1 or AL-1 antagonist, e.g., anti-AL-1 antibody, to be employed therapeutically will depend, for example, upon the therapeutic objectives, the route of administration, and the condition of the patient. Accordingly, it will be necessary for the therapist to titer the dosage and modify the route of administration as required to obtain the optimal therapeutic effect. A typical daily dosage might range from about 1 µg/kg to up to 100 mg/kg or more, depending on the factors mentioned above. Where possible, it is desirable to determine appropriate dosage ranges first *in vitro,* for example, using assays for neuronal cell survival or growth which are known in the art, and then in suitable animal models, from which dosage ranges for human patients may be extrapolated. In a specific embodiment of the invention, a pharmaceutical composition effective in promoting the survival or growth of neurons will provide a local GPA concentration *in vivo* of between about 0.1 and 10 ng/ml.

In the treatment of tumors the compositions described herein can be administered subcutaneously or intramuscularly, for example, and the pharmacological activities of an AL-1 antagonist can be maintained over a long period of time by the sustained-release effect of a composition of the present invention. The number of administrations can therefore be reduced. The composition can also be by directly injecting the composition into a tumor-controlling artery. In the case of treatment of an adult patient having a tumor, the dose of the AL-1 antagonist can be appropriately selected depending upon the kind of rumor, site, size, and kind of AL-1 antagonist. For example, the dose of a protein AL-1 antagonists, particularly an antibody, can be about 0.1 mg to about 500 mg, typically about 1.0 mg to about 300 mg, more typically about 25 mg to about 100 mg. The administration frequency can be appropriately selected depending upon the kind of disease and dosage form. In the case of injection into the tumor-controlled artery or tumor itself, frequently repeated injections are not required and a single injection once every one to 4 weeks may be sufficient for the desired therapeutic effects.

In summary, by providing nucleic acid molecules encoding AL-1, the present invention enables for the first time the production of AL-1 by recombinant DNA methods, thus providing a reliable source of sufficient quantities of AL-1 for use in various diagnostic and therapeutic applications. In view of its distinct biological properties, purified recombinant AL-1 will be especially useful in a variety of circumstances where it is necessary or desirable to assure neuronal function, growth and survival, or angiogenesis, but where other neurotrophic factors or angiogenic agents either cannot be used or are ineffective.

The following examples are offered by way of illustration only and are not intended to limit the invention in any manner.

### EXAMPLE 1

### Identification and Isolation of REK7 cDNA

To'isolate novel growth factors that might act on central nervous system neurons, a search for new tyrosine kinase receptors was made using a polymerase chain reaction (PCR). Degenerate sense and antisense primers were prepared that corresponded to conserved amino acid sequences within the kinase domain of many receptor tyrosine kinases. The nucleotide sequences of the primers were as follows:

These degenerate primers were used to amplify the cDNAs of an adult mouse hippocampal cDNA library using standard PCR methods, and the resulting amplified cDNAs were subcloned and sequenced. One of these cDNAs, approximately 200 bp in size, encoded a deduced amino acid sequence that appeared to be related to an amino acid sequence within the kinase domains of the eph family of receptor tyrosine kinases. This 200 bp cDNA then was used as a probe to isolate a full-length cDNA from a rat hippocampal cDNA library. Figure 1 shows the nucleotide sequence (SEQ. ID NO. 1) of the full-length cDNA, and the deduced amino acid sequence (SEQ. ID NO. 2) of the encoded protein, termed REK7.

REK7 appears to be the rat homologue of cek7, and is closely related to ehk-I and bsk. (Maisonpierre, et al., *Oncogene* 8:3277-3288 (1993); Zhou, et al., *J. Neurosci. Res*. 37:129-143 (1994)). In particular, the REK7 cDNA corresponds to a splice variant of ehk-I lacking the first of two tandem fibronectin type-III domains.

### EXAMPLE 2

### Construction of REK7 Expression Plasmid

A cDNA encoding REK7 was cloned into the mammalian expression vector pRK7 (PCT Publication No. WO 90/02798, published September 22, 1990), between the XbaI and BamHI restriction endonuclease cleavage sites of pRK7, to produce the expression plasmid pRK-REK7.

### EXAMPLE 3

### Production of REK7-IgG Fusion Protein

DNA encoding a soluble REK7-IgG chimera was constructed by joining DNAs encoding the extracellular domain of REK7 and the F_{c} domain of the IgG, heavy chain. The DNA sequence encoding the IgG₁ portion of the REK7-IgG chimera was obtained from the -IgG expression plasmid pRKCD4₂F_{c1} (Capon, et al., Nature 337:525 (1989); Byrn, et al., *Nature* 344:667 (1990)). That plasmid encodes a hybrid polypeptide consisting of residues 1-180 of the mature human CD4 protein (two N-teiminal CD4 variable domains) fused to the portion of the human immunoglobulin IgG₁ protein extending from the aspartic acid residue at position 216 (which is the first residue of the IgG₁ hinge after the cysteine residue involved in heavy-light chain bonding). to amino acid residue 441 (the numbering of residues in IgG₁ is based on amino acid residue 114 being the first residue of the heavy chain constant region (Kabat, et al., Sequences of Proteins of Immunological Interest, 4th ed. (1987)).

PCR was used to generate a 600 bp fragment containing sequences encoding the 3'-end of the REK7 extracellular domain (terminating at Gln₄₆₂, the junction between the extracellular domain and the transmembrane domain), joined to sequences encoding the 5'-end of a human IgG₁ Fc domain. The 600 bp fragment was constructed in three steps. First, a DNA fragment containing the 3'-end of the REK7 extracellular domain was amplified using pRK/EK7 as template and 5'-TCTGTGACAGACGATCCTCCC (primer 1. SEQ. ID. NO. 8) and 5'-GCACGGTGGACATGTGTGAGTTTTGTCCTGGCTTTGATCATTAGATGCTGCAAC (SEQ. ID. NO. 9) as primers. Second, a DNA fragment containing the 5'-end of a human IgG₁ Fc domain was amplified using pRKCD4-IgG as template and 5'-GTTGCAGCATCTAATGATCAAAGCCAGGACAAAACTCACACATGTCCACCGTGC (SEQ.ID. NO.10) and 5'-GCACTTGTACTCCTTGCC (primer 2, SEQ. ID. NO. 11) as primers. Finally, the two resulting amplified DNA fragments were mixed together and covalently joined in a third PCR using primers 1 and 2, to produce a 600 bp DNA fragment encoding a REK7-IgG fusion protein.

Since the REK7 cDNA appears to contain sequences that promote rearrangements and deletions during DNA manipulation, assembly of an expression plasmid encoding the full-length REK7-IgG protein was carried out in three steps. First, the 600 bp PCR product (see above) was cut by digestion with KpnI and SacII restriction endonucleases and the resulting KPNI-SacII fragment was gel purified. pRKCD4-IgG₁ was cleaved with HindIII and SacII restriction endonucleases and a 502 bp fragment was isolated. These KpnI-SacII fragment and the 502 bp fragment were ligated into a Bluescript vector (Stratagene, La Jolla, california USA) cleaved with KpnI and HindIII, to produce REK7-IgG Intermediate 1.

Second, REK-IgG Intermediate 1 was cut with KpnI and EcoRI restriction endonucleases and the resulting 1000 bp fragment was isolated. pRK-REK7 was cut with KpnI and ApaI restriction endonucleases and a 786 bp fragment was isolated. The 1000 bp fragment and the 786 bp fragment were ligated into a Bluescript vector cut with ApaI and EcoRI, to produce REK7-IgG Intermediate 2.

Finally, the complete pRKREK-IgG expression plasmid (7004 bp) was assembled by ligation of the three fragments as follows. REK7-IgG Intermediate 2 was cut with PflMI and EcoRI restriction endonucleases and a 1860 bp fragment was isolated. pRK-REK7 was cut with PflMI and PstI restriction endonucleases and a 512 bp fragment was isolated. These two fragments were ligated to the pRK7 vector cut with PstI and EcoRI, to produce pRKREK7-IgG.

REK7-IgG was expressed in human embryonic kidney 293 cells (Graham, et al., *J. Gen. Virol*. 36:59 (1977)) and Chinese hamster ovary (CHO) cells by transient transfection using the calcium phosphate precipitation method as described by Capon, et al., supra, and Byrn, et al., supra. The REK7-IgG chimera was purified to greater than 95% homogeneity from serum free cell culture supernatants by affinity chromatography on immobilized Staphylococcus aureus Protein A as described by Capon. et al., supra.

### EXAMPLE 4

### Identification of a Source for REK7 Ligand

In an effort to identify the putative REK7 ligand, REK7-IgG was used to screen cultured cell lines for cell surface expression of REK7-binding activity. Cell lines were assayed by incubation with REK7-IgG and fluorescent anti-IgG antibody followed by fluorescence-activated cell sorting (FACS).

For example, human breast carcinoma cell line BT20 (American type Culture Collection, Rockville, Maryland, USA) was grown in 50/50 (v/v) F12/DMEM low glucose medium, with 10% fetal calf serum (FCS) and 1 mM glutamine (collectively, growth medium), in a 5% CO₂. Cells at 80-90% confluence were harvested with 5 mM EDTA in phosphate buffered saline (PBS), counted and resuspended in binding buffer (50/50 (v/v) F12/DMEM low glucose medium, 5% FCS, and 1mg/ml bovine serum albumin (BSA)) at a cell density of 5 x 10⁶ cells/ml. To 1 ml of cells, 1 µg of REK7-IgG was added and incubated for 2 hours at room temperature. The cells then were collected by centrifugation, incubated with a fluorescein-labelled anti-human IgG1-Fc for 1 hour at room temperature, then analyzed by FACS.

The BT20 cell line and the human cervical carcinoma cell line HeLa (American type Culture Collection, Rockville, Maryland, USA) were found to specifically bind the REK7-IgG. Furthermore, pretreatment of these cells with phosphatidylinositol-specific phospholipase C prior to incubation with REK7-IgG was found to decrease the binding of the REK7-IgG, suggesting the REK7 ligand is linked to the cell membrane by a glycophosphatidyl-inositol (GPI) anchor.

### EXAMPLE 5

### Purification of REK7 Ligand from BT20 Cells

BT20 cells were grown to 80% confluence in 150 mm plates, harvested by 5 mM EDTA in PBS, and seeded into 850 mm² roller bottles with growth medium. After 1 week of growth the cells were nearly confluent. The growth medium was removed, the cells washed with PBS, and serum-free growth medium was added. After 5 days, conditioned medium was harvested (12 liters from 72 roller bottles), centrifuged, sterile filtered, concentrated in a 12 kD molecular weight cutoff Amicon filter, and stored at -70°C.

200 ml of BT20 conditioned media, concentrated as described above from 12 liters and frozen, was thawed, centrifuged at 17,000 rpm in a Sorvall SS34 rotor, and filtered through a 0.45 µm filter to clarify the solution. The 200 ml was pumped through a 2.0 ml CD4-IgG - Protein A (CD4-IgG covalently linked to Protein A through IgG moiety) immunoaffinity precolumn and a 1.0 ml REK7-IgG - Protein A (REK7-IgG covalently linked to Protein A through IgG moiety) immunoaffinity column in tandem with a flow rate of 0.5 ml/min. All chromatography was carried out at 4° C. Following one complete passage of the media through both columns, the flow rate was reduced to 0.2 ml/min, and the media was recycled through both columns. Following the loading of the REK7-IgG column, the two column were separated from each other, washed with 10 column volumes of PBS, and then eluted with four separate washes of 100 mM sodium citrate, pH 3.0 (2 ml each for the CD4-IgG column and 1 ml each for the REK7-IgG column). The first two washes were allowed to immediately flow through the column whereas the last two were incubated for 15 minutes before collection. The eluates were brought to pH 7.4 by the addition of 50 mM potassium phosphate, and an aliquot of each was analyzed by sodium dodecycl sulfate (SDS)-polyacrylamide gel electrophoresis (PAGE). Following silver staining of the gel, stained band of approximately 28,000, 27,000, and 25,000 Daltons was seen from the eluate from the last two washes, that were not seen in eluate obtained by similar treatment of the CD4-IgG precolumn.

### EXAMPLE 6

### Protein Sequencing

The 28,000, 27,000, and 25,000 Dalton protein bands that were observed on SDS-PAGE were transferred to a PVDF membrane (Millipore Corporation) by electroblotting, and then subjected to amino acid sequencing using a Applied Biosystems 473A or 470A sequencer. The N-terminus of each of the 28,000 and 25,000 Dalton proteins was blocked, but the N-terminus of the 27,000 Dalton protein gave the following sequence: DRYAVYW(N)SSNPRFQRGDYHIDVXINDY (SEQ. ID NO. 12).

Separate sequence analysis of the three protein bands after cyanogen bromide cleavage or digestion with Lys-C endopeptidase indicated that the 28,000, 27,000, and 25,000 Dalton proteins were related. In particular, the 27,000 Dalton and 25,000 Dalton proteins appeared to be proteolytic processed forms of the 28,000 Dalton protein. Sequence analysis of the 28,000 Dalton band after digestion with Lys-C endopeptidase resulted in two sequences, one of which was nearly identical to the N-terminal sequence of the 27,000 Dalton protein and also had three additional N-terminal residues, AVA. The following internal amino acid sequences were determined for cyanogen bromide and Lys-C fragments of the proteins:

### EXAMPLE 7

### Isolation of REK7 Ligand cDNA

Based on the above protein sequences obtained for the REK7 ligand, two degenerate PCR primers were synthesized for use in isolating cDNA encoding the REK7 ligand:

These degenerate primers were used to amplify the cDNAs of a BT20 cell cDNA library using standard PCR methods, and two resulting amplified cDNAs were subcloned and sequenced. These cDNAs, approximately 180 bp and 135 bp in size, encoded deduced amino acid sequences matching the above amino acid sequences determined for the REK7 ligand. The 180 bp fragment was used to screen a human fetal brain cDNA library (containing approximately 2 x 10⁶ clones) by hybridization under high stringency conditions. The nucleotide sequence determined from two independent positive cDNA clones is shown in Figure 2, along with the deduced amino acid sequence of the REK7 ligand, termed AL-1.

### EXAMPLE 8

### Biological Activity

Cerebral cortex from postnatal day 2 (P2) Wistar rat pups was dissected in 4°C Hanks balanced salt solution (HBSS, calcium and magnesium free) under sterile conditions. After initial trituration with a 10ml pipette, cells were dissociated by two passages through an 18 gauge injection cannula attached to a 10ml syringe. The cell suspension was strained through a 70 µm cell strainer (Falcon) and centrifuged at 800 g for 5 minutes. Pelleted cells were resuspended in 50/50 (v/v) DMEM/F12 with 10% fetal bovine serum, 15mM HEPES pH7.4. The cell suspension was plated into 75 cm² tissue culture flasks, and placed into a 37° C, 5% CO₂ incubator. Cultures were grown to confluence (10-15 days) and the medium was changed every 3 days. Once cultures were confluent the flasks were shaken in a rotary shaker at 300 rpm at 37° C for 24 hrs to obtain purified astrocytes. Media with the suspended cells and debris was replaced and cultures were incubated for another 24 hours. Purified astrocyte cultures were trypsinized (0.05% trypsin, 0.025mM EDTA in Hanks) from the flasks and then replated into 60mm tissue culture treated dishes (2 dishes per 75cm² flask).

Cerebral cortex of embryonic day 16 (E16) rats was dissected in 4° C HBSS under sterile conditions. Media was changed to 1 ml of Defined Neuronal Medium ("DNM"; Peterson et al., *Dev. Brain Res.* 48:187-195 (1989) and cortices were triturated by pipeting 15 times through a plastic disposable pipet blue. 9 ml of DNM then was added and the cell suspension was strained through a 45µm cell strainer and centrifuged at 800 g for 5 minutes. Pelleted cells were resuspended in 5 ml of DNM and 4 x 10⁵ cells were plated onto 60mm dishes containing astrocytes which had been pretreated for 6 hours with either REK7-IgG (30µg/ml), CD4-IgG (30µg/ml), or no added IgG (control). Cultures were grown for 4 days and then fixed for 15 minutes with 4% paraformaldehyde, in 100 mM Sodium Phosphate buffer, pH 7.4, followed by 3 washes with PBS.

The addition of REK7-IgG to the co-cultures completely prevented the formation of axon bundles. In contrast, the addition of CD4-IgG, and other receptor-IgGs (e.g., TrkA-IgG (Shelton et al. *J. Neurosci*. 15:477-491 (1995), Eph-related HTK-IgG (Bennett et al. *J. Biol. Chem*. 269:14211-14218 (1994)) had no effect on axon bundling. These results indicate a role for REK7 and its ligand, AL-1, in axon fasciculation, which is a crucial step in the development of the nervous system during regeneration following injury.

Consistent with that role, upon incubation of the cultured neuronal cells with labelled anti-REK7 antibodies, there was specific binding of the antibodies to the surface of axonal fibers within axon bundles. A related EPH-family receptor, SEK1 (Gilardi-Hebenstreit et al, *Oncogene* 7:2499-2506 (1992)) failed to bind to the surface of the axonal fibres within the bundles. Furthermore, AL-1 is expressed predominantly on astrocytes in coculture, while REK-7 is expressed on both axon fibers and cell bodies of the cortical neurons.

### EXAMPLE 9

### Biological Activity; Activation of REK7 by AL-1

When cells expressing REK7 were incubated with AL-1, specific phosphorylation of REK7 was observed, indicating that AL-1 not only binds to REK7 but also activates REK7. Cultured primary cortical neurons were analyzed by immunoprecipitation and immunoblotting with anti-REK7 and anti-phosphotyrosine antibodies. Membrane-bound AL-1 transiently expressed on the surface of transfected 293 cells, strongly activated endogenous REK7 expressed in cortical neurons, as indicated by autophosphorylation of the 135K REK7 polypeptide. In contrast, purified soluble AL-1 only weakly stimulated tyrosine autophosphorylation of REK7 ∼2-fold (Winslow et al., *Neuron*, 14:973-981 (1995), which is incorporated by reference). These data (i) confirm the presence of REK7 in cortical neurons, (ii) indicate that AL-1 is an activating ligand, and (iii) demonstrate that membrane-attachment is required for maximal receptor activation as has been seen with other members of this ligand family (Davis et al., *Science* 266:816-819(1994)).

HEK 293 cells were transfected with an AL-1 cDNA expression plasmid using the calcium phosphate coprecipitation method (Simonsen and Levinson, *Proc. Natl. Acad. Sci.* 80:2495-2499 (1983)). Primary cortical neurons from E16 rats were plated at a density of 5 x 10⁶ cells /15 cm dish and cultured for 4 days. These cells were then treated with purified soluble AL-1 (0.1-1 µg/ml) or 293 cells expressing membrane-bound AL-1 for 10 min at 37 °C. Immunoprecipitation of lysates with rabbit anti-REK7 and immunoblotting with mouse anti-phosphotyrosine was essentially as described (Kaplan et al, *Science* 252:554-559 (1991); Kaplan et al., *Nature* 350:158-160 (1991)). Immunoblotted bands were visualised using a horseradish peroxidase-conjugated sheep anti-mouse antibody and the ECL fluorescence detection system (Amersham) as described by the manufacturer.

### EXAMPLE 10

### Activity of AL-1-IgG Fusion

An AL-1-IgG fusion protein was demonstrated to be a soluble, active form of AL-1. DNA encoding a soluble AL-1-IgG chimera was constructed by joining the DNAs encoding the extracellular domain of AL-1 and the Fc domain of the IgG₁, similar to the construction of REK7-IgG. AL-1-IgG fusion protein was generated by transfection of 293 cells with the plasmid pRKAL-1-IgG. Conditioned media was collected after 3 days and AL-1-IgG was purified by Protein A chromatography. The ability of AL-1-IgG to activate the REK7 receptor was determined by tyrosine autophosphorylation of rat embryonic (E16) cortical neurons REK7 receptor as described above. Using the same density and number of cortical cells (5x 10⁶ cells/15 cm plate), 1 ug/ml of AL-1-IgG was compared with 1 ug/ml soluble AL-1, or an equivalent number of 293 cells expressing membrane bound AL-1. No addition, and mock transfected 293 cells were included as controls. AL-1-IgG greatly activated the autophosphorylation of REK7, to a similar extent as membrane-bound AL-1. These results indicate that the soluble AL-1-IgG fusion protein can be used as an agonist for the REK7 receptor in *in vitro* and *in vivo* studies. The inability of soluble AL-1 to activate REK7 autophosphorylation, although the affinity chromatography purification demonstrates binding to the receptor, indicates that the soluble form of AL-1 (and EPH receptor ligands in general toward their cognate receptor) can act as antagonists of REK7.

### EXAMPLE 11

### Soluble AL-1 is an antagonist of axon bundling

Soluble AL-1 bound to but did not efficiently activate REK7, and soluble AL-1 acted as an antagonist of REK7 receptor activity and blocked axon fasciculation in the cell culture system. The addition of soluble AL-1 to the culture medium of cortical neuron-astrocyte co-cultures specifically prevented the formation of axon bundles, as did REK7-IgG. This result, taken together with the observations that (i) AL-1 is expressed in the co-cultures (predominantly on astrocytes); (ii) REK7 is expressed on both the axons fibres and cell bodies of the cortical neurons and (iii) AL-1 can activate REK7, further implicates AL-1 and REK7 as the molecules involved in axon bundling whose action is inhibited by REK7-IgG.

The effect on axon bundle formation of the anti-REK7 and anti-SEK antibodies, added to the culture medium at 200 µg/ml was tested. Neither reagent had any apparent effect on axon bundling in these cultures. Since the anti-REK7 antibodies bind to the axon fibres but fail to block bundle formation, the mere binding to axons is not sufficient to interfere with the bundling process, which further supports that the inhibitory effects of REK7-IgG and soluble AL-1 are due to a specific inhibition of REK7 function.

The following mechanism is not meant to be limiting to the invention. The role of REK7 in axon fasciculation is probably indirect, i.e. REK7 and AL-1 do not themselves function as adhesion molecules but rather are involved in regulating the fasciculation process. Activation of REK7 expressed on the neurons, by AL-1 expressed on the astrocytes, might activate a signaling pathway that promotes fasciculation, for example by up-regulating or activating adhesion molecules. Activation of REK7 may cause growth cone repulsion and collapse, forcing axons together for fasciculation. AL-1 expressed on astrocytes would serve as a repulsive cue to axons driving axons together. This model is compatible with the current view that astrocytes play an important role during development of the CNS, where they are thought to provide a substratum and trophic support for growing axons (Hatten et al., *Semin. Neurosci.* 2:455-465 (1990)).

As taught herein a tyrosine kinase is required for axon fasciculation. Neurons in the developing or regenerating nervous system presumably require two types of factors, those that promote growth and survival. and. those that provide spatial or directional guidance in the establishment of neuronal pathways (Tessier-Lavigne, *Curr. Opin.Genet. Devel*. 4:596-601 (1994)). Tyrosine kinases are known to play a well-established role in the former but have not hitherto been shown to participate in the latter. The teachings herein, combined with the observation that both REK7 and AL-1 are expressed in the brain, indicate a role in the formation of neuronal pathways, a crucial feature of both development and regeneration in the nervous system.

## Claims

1. An isolated nucleic acid encoding a functional AL-1 protein comprising an amino acid sequence that is at least 75% identical to the amino acid sequence shown in Figure 2 for human AL-1.

2. The isolated nucleic acid according to claim 2 encoding an amino acid sequence that is at least 85% identical to the amino acid sequence shown in Figure 2 for human AL-1.

3. An isolated nucleic acid molecule according to claim 1, comprising a nucleotide sequence encoding the amino acid sequence shown in Figure 2 for mature AL-1.

4. An isolated nucleic acid molecule comprising a nucleotide sequence encoding the amino acid sequence of the extracellular domain shown in Figure 2 for AL-1.

5. An isolated nucleic acid encoding a functional AL-1 protein comprising an amino acid sequence that is at least 75% identical to the amino acid sequence of the extracellular domain shown in Figure 2 for human AL-1.

6. An isolated nucleic acid according to claim 5, comprising the amino acid sequence of the extracellular domain shown in Figure 2 for AL-1.

7. An isolated nucleic acid comprising a nucleotide sequence encoding an amino acid sequence that is at least 75 % identical to the amino acid sequence of the human AL-1 extracellular domain shown in Figure 2, fused to an IgG heavy chain constant region sequence.

8. An isolated nucleic acid that hybridizes to DNA encoding mature human AL-1 of Figure 2 under stringent conditions, and that encodes a polypeptide comprising an immune epitope of the mature human AL-1 which binds antibodies specific for AL-1; wherein stringent conditions refers to those that (1) employ low ionic strength and high temperature for washing, for example, 0.015 M NaCl/0.0015 M sodium citrate/0.1% SDS at 50°C, or (2) employ during hybridization a denaturing agent such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM NaCl, 75 mM sodium citrate at 42°C, or for example 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/mL), 0.1% SDS, and 10% dextran sulfate at 42°C, with washes at 42°C in 0.2 x SSC and 0.1% SDS.

9. An expression vector comprising the nucleotide sequence of any one of claims I to 8 operably linked to a promoter.

10. A host cell transformed with the expression vector of claim 9. /

11. A method of using the host cell of claim 10, which comprises culturing the host cell under conditions such that the expression vector is replicated.

12. A process, comprising culturing the transformed cell of claim 10 in a cell culture medium under conditions such that the AL-1 protein is synthesized.

13. The process of claim 12, wherein said nucleotide sequence encodes a secretable form of AL-1, and the cell host cell is suitable for permitting secretion into the culture medium, the process further comprising recovering the polypeptide from the cell culture medium.

14. A method for producing an AL-1 protein comprising an amino acid sequence that is at least 75 % identical to the amino acid sequence of human AL-1 as shown in Figure 2, said method comprising:
(a) transforming a cell containing an endogenous gene encoding said protein with a homologous DNA comprising an amplifiable gene and a flanking sequence of at least about 150 base pairs that is homologous with a DNA sequence within or in proximity to said endogenous gene, whereby the homologous DNA integrates into the cell genome by recombination;
(b) culturing the cells under conditions that select for amplification of the amplifiable gene, whereby said endogenous gene is also amplified; and thereafter
(c) recovering said protein from the cells.

15. An isolated AL-1 protein comprising an amino acid sequence that is at least 75% identical to the amino acid sequence shown in Figure 2 for human AL-1.

16. The isolated protein according to claim 15, comprising an amino acid sequence that is at least 85% identical to the amino acid sequence shown in Figure 2 for human AL-1.

17. An isolated protein according to claim 15, comprising the amino acid sequence shown in Figure 2 for mature AL-1.

18. An isolated AL-1 protein comprising an amino acid sequence that is at least 75% identical to the amino acid sequence of the extracellular domain shown in Figure 2 for human AL-1.

19. An isolated AL-1 protein according to claim 18, comprising the amino acid sequence of the extracellular domain shown in Figure 2 for AL-1.

20. An immunoadhesin comprising an amino acid sequence that is at least 75 % identical to the amino acid sequence of the extracellular domain of AL-1 shown in Figure 2, fused to an IgG heavy chain constant region sequence.

21. An antibody that is capable of specifically binding to the amino acid sequence shown in Figure 2 for mature AL-1.

22. An antibody of claim 21 that is a monoclonal antibody.

23. An antibody of claim 21 or claim 22 that is a neutralizing antibody.

24. An AL-1 protein according to any one of claims 15 to 20, for pharmaceutical use.

25. A pharmaceutical composition comprising the protein of any one of claims 15 to 20 and a physiologically acceptable excipient.

26. A sterile surgical article comprising an AL-1 protein of any one of claims 15 to 20.

27. A composition for the debridement of bums comprising an AL-1 protein of any one of claims 15 to 20, and a proteolytic enzyme which does not inactivate the neovascularizing activity of said protein.

28. A pharmaceutical comprising an AL-1 protein of any one of claims 15 to 20, together with NGF, BDNF, NT-3, NT-4/5 or a second eph receptor family ligand.

29. A pharmaceutical preparation for topical application to a wound, comprising an AL-1 protein of any one of claims 15 to 20, together with one or more components selected from collagen, a skin graft, growth factors, antibiotics, debridement agents and angiogenin.

30. A pharmaceutical preparation according to claim 29, wherein said protein comprises an immunoadhesin in which said amino acid sequence is fused to an IgG heavy chain constant region sequence.

31. The use of an AL-1 protein according to any one of claims 15 to 20 in the preparation of a medicament for treating a neurologic disease or disorder in a mammal.

32. The use according to claim 31 wherein the neurologic disease or disorder is Alzheimer's disease, amyotrophic lateral sclerosis, trauma-induced, surgery-induced, stroke-induced, ischemia-induced, infection-induced, metabolic disease-related, nutritional deficiency-induced, malignancy-induced, neurotoxicity, Bell's palsy, spinal muscular atrophy or paralysis, Parkinson's disease, epilepsy, multiple sclerosis, Huntington's chorea, Down's Syndrome, nerve deafness, Meniere's disease, post-polio syndrome, Charcot-Marie-Tooth disease, Refsum's disease, Abetalipoproteinemia, Tangier disease, Krabbe's disease, Metachromatic leukodystrophy, Fabry's disease, and Dejerine-Sottas syndrome.

33. The use according to claim 31 or claim 32 wherein the medicament further comprises NGF, BDNF, NT-3, NT-4/5, or a second eph receptor family ligand.

34. The use of an AL-1 protein of any one of claims 15 to 20 in the preparation of a medicament for accelerating the neovascularization of a wound.

35. The use according to claim 34 wherein the medicament is suitable for application topically by direct contact with the wound and the wound is a fresh surgical incision.

36. The use according to claim 34 or claim 35 wherein the medicament further comprises collagen.

37. The use according to any one of claims 34 to 36, wherein said protein is an immunoadhesin in which said amino acid sequence is fused to an IgG heavy chain constant region sequence.

38. The use according to any one of claims 34 to 37, wherein the wound is a bum and the composition further comprises a skin graft.

39. The use according to any one of claims 34 to 38, which further comprises the use of a substance selected from growth factors, antibiotics, debridement agents and angiogenin.

40. A method comprising:
(a) contacting nucleic acid from a cell or a tissue with a labelled DNA which comprises a sequence of at least 15 nucleotides selected from the nucleotide sequence shown in Figure 2 and which is unique to AL-1, under conditions which will allow hybridization of complementary nucleotide sequences specific to AL-1 or its alleles; and
(b) detecting any hybridization which occurs.

41. A method comprising:
(a) contacting nucleic acid from a cell or a tissue with a DNA which comprises a sequence of at least 15 nucleotides selected from the nucleotide sequence shown in Figure 2 and which is unique to AL-1, under conditions which will allow hybridization of complementary nucleotide sequences specific to AL-1 or its alleles;
(b) amplifying in a polymerase chain reaction the nucleic acid to which the DNA of step (a) hybridizes; and
(c) detecting any nucleic acid produced by the polymerase chain reaction of step (b).

## Patentansprüche

1. Isolierte Nukleinsäure, die für ein funktionelles AL-1-Protein kodiert, das eine Aminosäuresequenz umfasst, die zu zumindest 75 % mit der in Fig. 2 angeführten Aminosäuresequenz von Human-AL-1 identisch ist.

2. Isolierte Nukleinsäure nach Anspruch 1, die für eine Aminosäuresequenz kodiert, die zu zumindest 85 % mit der in Fig. 2 angeführten Aminosäuresequenz von Human-AL-1 identisch ist.

3. Isoliertes Nukleinsäuremolekül nach Anspruch 1, das eine Nukleotidsequenz umfasst, die für die in Fig. 2 angeführte Aminosäuresequenz von Human-AL-1 kodiert.

4. Isoliertes Nukleinsäuremolekül, das eine Nukleotidsequenz umfasst, die für die in Fig. 2 angeführte Aminosäuresequenz der extrazellulären Domäne von AL-1 kodiert.

5. Isolierte Nukleinsäure, die für ein funktionelles AL-1-Protein kodiert, das eine Aminosäuresequenz umfasst, die zu zumindest 75 % mit der in Fig. 2 angeführten Aminosäuresequenz der extrazellulären Domäne von Human-AL-1 identisch ist.

6. Isolierte Nukleinsäure nach Anspruch 5, welche die in Fig. 2 angeführte Aminosäuresequenz der extrazellulären Domäne von AL-1 umfasst.

7. Isolierte Nukleinsäure, die eine Nukleotidsequenz umfasst, die für eine Aminosäuresequenz kodiert, die zu zumindest 75 % mit der in Fig. 2 angeführten Aminosäuresequenz der extrazellulären Human-AL-1-Domäne identisch ist, und die mit einer Sequenz einer konstanten Region einer IgG-Schwerkette fusioniert ist.

8. Isolierte Nukleinsäure, die an DNA hybridisiert, welche unter stringenten Bedingungen für reifes Human-AL-1 aus Fig. 2 kodiert, und die für ein Polypeptid kodiert, das ein Immunepitop des reifen Human-AL-1 umfasst, welches für AL-1 spezifische Antikörper bindet; worin sich stringente Bedingungen auf solche Bedingungen beziehen, bei denen (1) geringe Ionenstärke und hohe Temperaturen zum Waschen eingesetzt werden, z.B. 0,015 M NaCl/0,0015 M Natriumcitrat/0,1 % SDS bei 50 °C, oder (2) während der Hybridisierung ein Denaturierungsmittel, wie z.B. Formamid, eingesetzt wird, z.B. 50 Vol.-% Formamid mit 0,1 % Rinderserumalbumin/0,1 % Ficoll/0,1 % Polyvinylpyrrolidon/50 mM Natriumphosphatpuffer mit pH 6,5 mit 750 mM NaCl, 75 mM Natriumcitrat bei 42 °C, oder z.B. 50 % Formamid, 5 x SSC (0,75 M NaCl, 0,075 M Natriumcitrat), 50 mM Natriumphosphat (pH 6,8), 0,1 % Natriumpyrophosphat, 5 x Denthardt-Lösung, beschallte Lachsspermien-DNA (50 µg/ml), 0,1 % SDS und 10 % Dextransulfat bei 42 °C, mit Waschungen bei 42 °C in 0,2 x SSC und 0,1 % SDS.

9. Expressionsvektor, umfassend eine Nukleotidsequenz nach einem der Ansprüche 1 bis 8, die operabel an einen Promotor gebunden ist.

10. Wirtszelle, die mit einem Expressionsvektor nach Anspruch 9 transformiert ist.

11. Verfahren zur Verwendung einer Wirtszelle nach Anspruch 10, das die Kultivierung der Wirtszelle unter solchen Bedingungen umfasst, dass der Expressionsvektor repliziert wird.

12. Verfahren, umfassend die Kultivierung einer transformierten Zelle nach Anspruch 10 in einem Zellkulturmedium unter solchen Bedingungen, dass das AL-1-Protein synthetisiert wird.

13. Verfahren nach Anspruch 12, worin die Nukleotidsequenz für eine sekretierbare Form von AL-1 kodiert und die Zellwirtszelle geeignet ist, Sekretion in das Kulturmedium zu ermöglichen, wobei das Verfahren außerdem die Gewinnung des Polypeptids aus dem Zellkulturmedium umfasst.

14. Verfahren zur Herstellung eines AL-1-Proteins, das eine Aminosäuresequenz umfasst, die zu zumindest 75 % mit der in Fig. 2 angeführten Aminosäuresequenz von Human-AL-1 identisch ist, wobei das Verfahren Folgendes umfasst:
(a) Transformation einer Zelle, die ein endogenes Gen umfasst, welches für das Protein mit einer homologen DNA kodiert, die ein amplifizierbares Gen und eine flankierende Sequenz aus zumindest etwa 150 Basenpaaren umfasst und homolog mit einer DNA-Sequenz im oder in der Nähe des endogenen Gen(s) ist, wobei die homologe DNA durch Rekombination in das Zellgenom integriert wird;
(b) Kultivierung der Zellen unter Bedingungen, die bezüglich Amplifikation des amplifizierbaren Gens selektieren, wodurch das endogene Gen ebenfalls amplifiziert wird; und danach
(c) Gewinnung des Proteins aus den Zellen.

15. Isoliertes AL-1-Protein, das eine Aminosäuresequenz umfasst, die zu zumindest 75 % mit der in Fig. 2 angeführten Aminosäuresequenz von Human-AL-1 identisch ist.

16. Isoliertes Protein nach Anspruch 15, das eine Aminosäuresequenz umfasst, die zu zumindest 85 % mit der in Fig. 2 angeführten Aminosäuresequenz von Human-AL-1 identisch ist.

17. Isoliertes Protein nach Anspruch 15, das die in Fig. 2 angeführte Aminosäuresequenz für reifes AL-1 umfasst.

18. Isoliertes AL-1-Protein, das eine Aminosäuresequenz umfasst, die zu zumindest 75 % mit der in Fig. 2 angeführten Aminosäuresequenz der extrazellulären Domäne von Human-AL-1 identisch ist.

19. Isoliertes AL-1-Protein nach Anspruch 18, das die in Fig. 2 angeführte Aminosäuresequenz der extrazellulären Domäne von AL-1 umfasst.

20. Immunoadhäsin, das eine Aminosäuresequenz umfasst, die zu zumindest 75 % mit der in Fig. 2 angeführten Aminosäuresequenz der extrazellulären Domäne von AL-1 identisch ist, und die mit einer Sequenz einer konstanten Region einer IgG-Schwerkette fusioniert ist.

21. Antikörper, der zur spezifischen Bindung an die in Fig. 2 angeführte Aminosäuresequenz von reifem AL-1 fähig ist.

22. Antikörper nach Anspruch 21, der ein monoklonaler Antikörper ist.

23. Antikörper nach Anspruch 21 oder 22, der ein neutralisierender Antikörper ist.

24. AL-1-Protein nach einem der Ansprüche 15 bis 20 zur pharmazeutischen Verwendung.

25. Pharmazeutische Zusammensetzung, die ein Protein nach einem der Ansprüche 15 bis 20 und einen physiologisch annehmbaren Exzipienten umfasst.

26. Steriler chirurgischer Gegenstand, der ein AL-1-Protein nach einem der Ansprüche 15 bis 20 umfasst.

27. Zusammensetzung für das Débridement von Verbrennungen, die ein AL-1-Protein nach einem der Ansprüche 15 bis 20 und ein proteolytisches Enzym umfasst, das die Neovaskularisierungsaktivität des Proteins nicht deaktiviert.

28. Arzneimittel, das ein AL-1-Protein nach einem der Ansprüche 15 bis 20 zusammen mit NGF, BDNF, NT-3, NT-4/5 oder einem zweiten eph-Rezeptorfamilien-Liganden umfasst.

29. Pharmazeutisches Präparat zur topischen Anwendung an einer Wunde, das ein AL-1-Protein nach einem der Ansprüche 15 bis 20 zusammen mit einer oder mehreren Komponenten umfasst, die aus Kollagen, Hauttransplantaten, Wachstumsfaktoren, Antibiotika, Débridement-Wirkstoffen und Angiogenin ausgewählt sind.

30. Pharmazeutisches Präparat nach Anspruch 29, worin das Protein ein Immunoadhäsin umfasst, worin die Aminosäuresequenz an eine Sequenz einer konstanten Region einer IgG-Schwerkette fusioniert ist.

31. Verwendung eines AL-1-Proteins nach einem der Ansprüche 15 bis 20 bei der Herstellung eines Medikaments zur Behandlung einer neurologischen Erkrankung oder Störung bei einem Säugetier.

32. Verwendung nach Anspruch 31, worin die neurologische Erkrankung oder Störung Alzheimer-Krankheit, amyotrophe Lateralsklerose, durch ein Trauma ausgelöst, durch eine Operation ausgelöst, durch einen Schlaganfall ausgelöst, durch Ischämie ausgelöst, durch eine Infektion ausgelöst, mit einer Stoffwechselerkrankung in Zusammenhang stehend, durch mangelnde Ernährung ausgelöst, durch Malignität ausgelöst, Neurotoxizität, Bell-Lähmung, spinale Muskelatrophie oder Lähmung, Parkinson-Krankheit, Epilepsie, Multiple Sklerose, Chorea Huntington, Down-Syndrom, Nerventaubheit, Meniere-Krankheit, Postpoliosyndrom, Charcot-Marie-Tooth-Krankheit, Refsum-Krankheit, Abetalipoproteinämie, Tangier-Krankheit, Krabbe-Krankheit, metachromatische Leukodystrophie, Fabry-Krankheit oder Dejerine-Sottas-Syndrom ist.

33. Verwendung nach Anspruch 31 oder 32, worin das Medikament außerdem NGF, BDNF, NT-3, NT-4/5 oder einen zweiten eph-Rezepotorfamilien-Liganden umfasst.

34. Verwendung eines AL-1-Proteins nach einem der Ansprüche 15 bis 20 bei der Herstellung eines Medikaments zur Beschleunigung der Neovaskularisierung einer Wunde.

35. Verwendung nach Anspruch 34, worin das Medikament zur topischen Anwendung durch direkten Kontakt mit der Wunde geeignet ist und die Wunde ein frischer Operationsschnitt ist.

36. Verwendung nach Anspruch 34 oder 35, worin das Medikament außerdem Kollagen umfasst.

37. Verwendung nach einem der Ansprüche 34 bis 36, worin das Protein ein Immunoadhäsin ist, worin die Aminosäuresequenz an eine Sequenz einer konstanten Region einer IgG-Schwerkette fusioniert ist.

38. Verwendung nach einem der Ansprüche 34 bis 37, worin die Wunde eine Verbrennung ist und die Zusammensetzung außerdem ein Hauttransplantat umfasst.

39. Verwendung nach einem der Ansprüche 34 bis 38, die außerdem die Verwendung einer aus Wachstumsfaktoren, Antibiotika, Débridement-Wirkstoffen und Angiogenin ausgewählten Substanz umfasst.

40. Verfahren, umfassend:
(a) das Kontaktieren einer Nukleinsäure aus einer Zelle oder einem Gewebe mit einer markierten DNA, die eine Sequenz aus zumindest 15 Nukleotiden, ausgewählt aus der in Fig. 2 angeführten Nukleotidsequenz, umfasst und die nur in AL-1 vorkommt, unter Bedingungen, die Hybridisierung von für AL-1 oder seine Allele spezifischen komplementären Nukleotidsequenzen ermöglichen; und
(b) das Detektieren jeglicher stattfindender Hybridisierung.

41. Verfahren, umfassend:
(a) das Kontaktieren einer Nukleinsäure aus einer Zelle oder einem Gewebe mit einer DNA, die eine Sequenz von zumindest 15 Nukleotiden, ausgewählt aus der in Fig. 2 angeführten Nukleotidsequenz, umfasst und die nur in AL-1 vorkommt, unter Bedingungen, die Hybridisierung von für AL-1 oder seine Allele spezifischen komplementären Nukleotidsequenzen ermöglichen;
(b) das Amplifizieren der Nukleinsäure, an welche die DNA aus Schritt (a) hybridisiert, in einer Polymerasekettenreaktion; und
(c) das Detektieren jeglicher Nukleinsäure, die durch die Polymerasekettenreaktion aus Schritt (b) gebildet wird.

## Revendications

1. Acide nucléique isolé codant pour une protéine AL-1 fonctionnelle comprenant une séquence d'aminoacides qui est identique à au moins 75% à la séquence d'aminoacides représentée sur la figure 2 pour la AL-1 humaine.

2. Acide nucléique isolé suivant la revendication 2, codant pour une séquence d'aminoacides qui est identique à au moins 85% à la séquence d'aminoacides représentée sur la figure 2 pour la AL-1 humaine.

3. Molécule d'acide nucléique isolée suivant la revendication 1, comprenant une séquence de nucléotides codant pour la séquence d'aminoacides représentée sur la figure 2 pour la AL-1 mature.

4. Molécule d'acide nucléique isolée comprenant une séquence de nucléotides codant pour la séquence d'aminoacides du domaine extracellulaire représentée sur la figure 2 pour la AL-1.

5. Acide nucléique isolé codant pour une protéine AL-1 fonctionnelle comprenant une séquence d'aminoacides qui est identique à au moins 75% à la séquence d'aminoacides du domaine extracellulaire représentée sur la figure 2 pour la AL-1 humaine.

6. Acide nucléique isolé suivant la revendication 5, comprenant la séquence d'aminoacides du domaine extracellulaire représentée sur la figure 2 pour la AL-1.

7. Acide nucléique isolé comprenant une séquence de nucléotides codant pour une séquence d'aminoacides qui est identique à au moins 75% à la séquence d'aminoacides du domaine extracellulaire de la AL-1 humaine représentée sur la figure 2, fusionnée à une séquence de région constante de chaîne lourde de IgG.

8. Acide nucléique isolé qui s'hybride avec un ADN codant pour la AL-1 humaine mature de la figure 2 dans des conditions drastiques, et qui code pour un polypeptide comprenant un épitope humain de la AL-1 humaine mature qui se lie à des anticorps spécifiques de AL-1 ; les conditions drastiques consistant en des conditions qui (1) utilisent une faible force ionique et une température élevée pour le lavage, par exemple NaCl 0,015 M/citrate de sodium 0,0015 M/0,1% de SDS à 50°C, ou (2) utilisent au cours de l'hybridation un agent dénaturant tel que la formamide, par exemple un tampon à 50% (en volume/volume) de formamide avec 0,1% de sérum-albumine bovine/0,1% de Ficoll/0,1% de polyvinylpyrrolidone/50 mM de phosphate de sodium à pH 6,5 avec 750 mM de NaCl et 75 mM de citrate de sodium à 42°C ou, par exemple, 50% de formamide, et 5 x SSC (NaCl 0,75 M, citrate de sodium 0,075 M), phosphate de sodium 50 mM (pH 6,8), 0,1% de pyrophosphate de sodium, solution de Denhardt 5 x, ADN de sperme de saumon traité par ultrasons (50 µg/ml), 0,1% de SDS et 10% de sulfate de dextrane à 42°C, avec des lavages à 42°C dans des solutions de SSC 0,2 x et 0,1% de SDS.

9. Vecteur d'expression comprenant la séquence de nucléotides suivant l'une quelconque des revendications 1 à 8 liée de manière fonctionnelle à un promoteur.

10. Cellule hôte transformée avec le vecteur d'expression suivant la revendication 9.

11. Méthode d'utilisation de la cellule hôte suivant la revendication 10, qui comprend la culture de la cellule hôte dans des conditions telles que le vecteur d'expression soit répliqué.

12. Procédé comprenant la culture de la cellule transformée suivant la revendication 10, dans un milieu de culture cellulaire dans des conditions telles que la protéine AL-1 soit synthétisée.

13. Procédé suivant la revendication 12, dans lequel ladite séquence de nucléotides code pour une forme sécrétable de AL-1, et la cellule hôte est apte à permettre la sécrétion dans le milieu de culture, le procédé comprenant en outre la séparation du polypeptide à partir du milieu de culture cellulaire.

14. Méthode pour la production d'une protéine AL-1 comprenant une séquence d'aminoacides qui est identique à au moins 75% à la séquence d'aminoacides de la AL-1 humaine représentée sur la figure 2, ladite méthode comprenant les étapes consistant :
(a) à transformer une cellule contenant un gène endogène codant pour ladite protéine avec un ADN homologue comprenant un gène amplifiable et une séquence adjacente d'au moins environ 150 paires de bases qui est homologue à une séquence d'ADN dans ledit ou à proximité dudit gène endogène, l'ADN homologue subissant ainsi une intégration dans le génome de la cellule par recombinaison ;
(b) à cultiver les cellules dans des conditions qui effectuent une sélection pour l'amplification du gène amplifiable, ledit gène endogène étant ainsi amplifié ; et ensuite
(c) à recueillir ladite protéine à partir des cellules.

15. Protéine AL-1 isolée comprenant une séquence d'aminoacides qui est identique à au moins 75% à la séquence d'aminoacides représentée sur la figure 2 pour la AL-1 humaine.

16. Protéine isolée suivant la revendication 15, comprenant une séquence d'aminoacides qui est identique à au moins 85% à la séquence d'aminoacides représentée sur la figure 2 pour la AL-1 humaine.

17. Protéine isolée suivant la revendication 15, comprenant la séquence d'aminoacides représentée sur la figure 2 pour la AL-1 mature.

18. Protéine AL-1 isolée comprenant une séquence d'aminoacides qui est identique à au moins 75% à la séquence d'aminoacides du domaine extracellulaire représentée sur la figure 2 pour la AL-1 humaine.

19. Protéine AL-1 isolée suivant la revendication 18, comprenant la séquence d'aminoacides du domaine extracellulaire représentée sur la figure 2 pour la AL-1.

20. Immunoadhésine comprenant une séquence d'aminoacides qui est identique à au moins 75% à la séquence d'aminoacides du domaine extracellulaire de l'AL-1 représentée sur la figure 2, fusionnée à une séquence de région constante de chaîne lourde de IgG.

21. Anticorps qui est capable de se lier spécifiquement à la séquence d'aminoacides représentée sur la figure 2 pour la AL-1 mature.

22. Anticorps suivant la revendication 21, qui est un anticorps monoclonal.

23. Anticorps suivant la revendication 21 ou la revendication 22, qui est un anticorps neutralisant.

24. Protéine AL-1 suivant l'une quelconque des revendications 15 à 20, à usage pharmaceutique.

25. Composition pharmaceutique comprenant la protéine suivant l'une quelconque des revendications 15 à 20 et un excipient physiologiquement acceptable.

26. Article chirurgical stérile comprenant une protéine AL-1 suivant l'une quelconque des revendications 15 à 20.

27. Composition pour débrider des brûlures, comprenant une protéine AL-1 suivant l'une quelconque des revendications 15 à 20 et une enzyme protéolytique qui n'active pas l'activité de néovascularisation de ladite protéine.

28. Agent pharmaceutique comprenant une protéine AL-1 suivant l'une quelconque des revendications 15 à 20, conjointement avec du NGF, du BDNF, du NT-3, du NT-4/5 ou un second ligand de la famille des récepteurs eph.

29. Préparation pharmaceutique pour l'application topique à une plaie, comprenant une protéine AL-1 suivant l'une quelconque des revendications 15 à 20, conjointement avec un ou plusieurs constituants choisis entre le collagène, un greffon cutané, des facteurs de croissance, des antibiotiques, des agents de débridement et l'angiogénine.

30. Préparation pharmaceutique suivant la revendication 29, dans laquelle ladite protéine comprend une immunoadhésine dans laquelle ladite séquence d'aminoacides est fusionnée à une séquence de région constante de chaîne lourde de IgG.

31. Utilisation d'une protéine AL-1 suivant l'une quelconque des revendications 15 à 20 dans la préparation d'un médicament destiné au traitement d'une maladie ou affection neurologique chez un mammifère.

32. Utilisation suivant la revendication 31, dans laquelle la maladie ou affection neurologique est la maladie d'Alzheimer, la sclérose latérale amyotrophique, une maladie ou affection induite par un traumatisme, induite par une intervention chirurgicale, induite par un ictus, induite par une ischémie, induite par une infection, en rapport avec une maladie métabolique, induite par une déficience nutritionnelle ou induite par un état malin, une neurotoxicité, la paralysie de Bell, l'atrophie ou la paralysie musculaire rachidienne, la maladie de Parkinson, l'épilepsie, la sclérose en plaques, la chorée de Huntington, le syndrome de Down, la surdité nerveuse, la maladie de Ménière, le syndrome post-poliomyélitique, la maladie de Charcot-Marie-Tooth, la maladie de Refsum, l'abétalipoprotéinémie, la maladie de Tangier, la maladie de Krabbe, la leucodystrophie métachromatique, la maladie de Fabry et le syndrome de Déjerine-Sottas.

33. Utilisation suivant la revendication 31 ou la revendication 32, dans laquelle le médicament comprend en outre du NGF, du BDNF, du NT-3, du NT-4/5 ou un second ligand de la famille des récepteurs eph.

34. Utilisation d'une protéine AL-1 suivant l'une quelconque des revendications 15 à 20 dans la préparation d'un médicament destiné à accélérer la néovascularisation d'une plaie.

35. Utilisation suivant la revendication 34, dans laquelle le médicament est apte à l'application topique par contact direct avec la plaie et la plaie est une incision chirurgicale fraîche.

36. Utilisation suivant la revendication 34 ou la revendication 35, dans laquelle le médicament comprend en outre du collagène.

37. Utilisation suivant l'une quelconque des revendications 34 à 36, dans laquelle ladite protéine est une immunoadhésine dans laquelle ladite séquence d'aminoacides est fusionnée à une séquence de région constante de chaîne lourde de IgG.

38. Utilisation suivant l'une quelconque des revendications 34 à 37, dans laquelle la plaie est une brûlure et la composition comprend en outre un greffon cutané.

39. utilisation suivant l'une quelconque des revendications 34 à 38, qui comprend en outre l'utilisation d'une substance choisie entre des facteurs de croissance, des antibiotiques, des agents de débridements et l'angiogénine.

40. Méthode comprenant :
(a) la mise en contact d'un acide nucléique d'une cellule ou d'un tissu avec un ADN marqué qui comprend une séquence d'au moins 15 nucléotides choisis dans la séquence de nucléotides représentée sur la figure 2 et qui est unique à AL-1, dans des conditions qui permettent l'hybridation de séquences de nucléotides complémentaires spécifiques de AL-1 ou de ses allèles ; et
(b) la détection de toute hybridation se produisant.

41. Méthode comprenant :
(a) la mise en contact d'un acide nucléique d'une cellule ou d'un tissu avec un ADN qui comprend une séquence d'au moins 15 nucléotides choisis dans la séquence de nucléotides représentée sur la figure 2 et qui est unique à AL-1, dans des conditions qui permettent l'hybridation de séquences de nucléotides complémentaires spécifiques de AL-1 ou de ses allèles ;
(b) l'amplification, dans une réaction en chaîne avec une polymérase, de l'acide nucléique auquel s'hybride l'ADN de l'étape (a) ; et
(c) la détection de tout acide nucléique produit par la réaction en chaîne avec une polymérase de l'étape (b).
